(19) Europäisches Patentamt
European Patent Office
Office européen des brevets

(11) **EP 2 147 919 A1**

(12) **EUROPÄISCHE PATENTANMELDUNG**


(43) Veröffentlichungstag:
**27.01.2010 Patentblatt 2010/04**

(21) Anmeldenummer: **08013316.8**

(22) Anmeldetag: **24.07.2008**

(51) Int Cl.:
***C07D 413/12*** *(2006.01)* ***C07D 413/14*** *(2006.01)*
***C07D 413/06*** *(2006.01)* ***C07D 261/08*** *(2006.01)*
***A01N 43/80*** *(2006.01)*

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**



(71) Anmelder: **Bayer CropScience Aktiengesellschaft**
**40789 Monheim (DE)**

(72) Erfinder:
- **Zeiss, Hans-Joachim, Dr.**
  **65843 Sulzbach (DE)**
- **Dittgen, Jan Dr.**
  **60316 Frankfurt (DE)**
- **Feucht, Dieter Dr.**
  **65760 Eschborn (DE)**
- **Häuser-Hahn, Isolde Dr.**
  **51375 Leverkusen (DE)**
- **Kehne, Heinz Dr.**
  **65719 Hofheim (DE)**
- **Rosinger, Christopher Dr,**
  **65719 Hofheim (DE)**


(54) **Heterocyclisch substituierte Amide, Verfahren zu deren Herstellung und deren Verwendung als Herbizide**


(57) Es werden Verbindungen der allgmeinen Formel (I) und deren N-Oxide oder Salze, Verfahren zu deren Herstellung und deren Verwendung als Herbizide beschrieben.

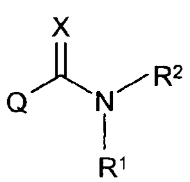

(I)

In der allgmeinen Formel (I) steht Q für einen aromatischen Heterocyclus, mit der Ausnahme von 4-Pyridyl, X für ein Sauerstoff- oder Schwefelatom und $R^1$ für verschiedene Reste. $R^2$ bedeutet ein Fragment der allgemeinen Formel (II)

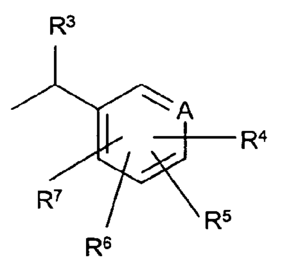

(II)

in dem A = CH oder N ist, und
$R^3$ ein fünfgliedriger aromatischer Ring, der ausgewählt ist aus der Gruppe bestehend aus 1,2-Oxazol, 1,3-Oxazol, 1,2-Thiazol, 1,3-Thiazol, Pyrazol, Imidazol, 1,2,4-Triazol und 1,2,4-Oxadiazol, sowie
$R^2$, $R^4$, $R^5$, $R^6$ und $R^7$ verschiedene Reste

bedeuten.


EP 2 147 919 A1

## Beschreibung

Die Erfindung betrifft das technische Gebiet der Herbizide, insbesondere der heterocyclisch substituierten Amide und Verfahren zu deren Herstellung sowie Formulierungen, die heterocyclisch substituierte Amide enthalten, zur selektiven Bekämpfung von Unkräutern und Ungräsem in Nutzpflanzenkulturen.

Es ist bekannt, dass Amide, die heterocyclisch substituiert sind, herbizide Eigenschaften besitzen. Aus dem Dokument WO2005/070889 sind als herbizide wirksame Amide Verbindungen der allgemeinen Formel (A) bekannt,

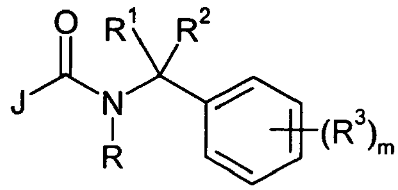

(A)

worin,

- J für Pyridin, Pyridinium, Pyrazol oder Isothiazol, das jeweils substituiert ist, stehen, und
- $R^1$ H oder $(C_1\text{-}C_4)$-Alkyl, und
- $R^2$ H, $(C_1\text{-}C_4)$-Alkyl, $(C_1\text{-}C_4)$-Haloalkyl, $(C_3\text{-}C_6)$-Cycloalkyl, $(C_2\text{-}C_3)$-alkenyl, $(C_2\text{-}C_3)$-alkynyl, $(C_2\text{-}C_4)$-alkoxymethyl, cyano, $(C_1\text{-}C_4)$-Alkoxy oder $(C_2\text{-}C_4)$- alkoxycarbonyl.

bedeuten.

Jedoch weisen die bekannten Verbindungen im Hinblick auf die Bekämpfung von Schadpflanzen verschiedene Nachteile auf. Zu den Nachteilen gehören neben einer zu geringen Aktivität ein zu geringes Spektrum der bekämpften Schadpflanzen sowie eine zu geringe Selektivität in Nutzpflanzenkulturen.

Die Aufgabe der vorliegenden Erfindung besteht in der Bereitstellung heterocyclisch substituierter Amide mit verbesserter herbizider Wirksamkeit und verbesserter Selektivität.

Gelöst wird die Aufgabe durch Verbindungen der allgemeinen Formel (I):

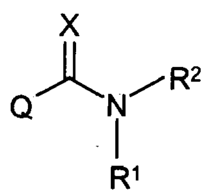

(I)

in der Q ein aromatischer Heterocyclus, mit der Ausnahme von 4-Pyridyl, ist, wobei

X ein Sauerstoff- oder Schwefelatom ist,

$R^1$ Wasserstoff, $(C_1\text{-}C_4)$-Alkyl, $(C_3\text{-}C_4)$-Cycloalkyl oder

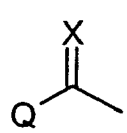

bedeutet,

$R^2$ ein Fragment der allgemeinen Formel (II):

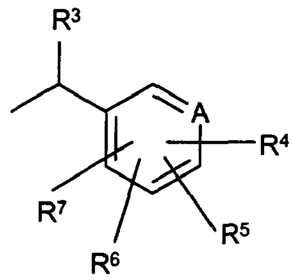

(II)

bedeutet, in dem A = CH oder N ist,

$R^3$ ein fünfgliedriger aromatischer Ring bedeutet, der ausgewählt ist aus der Gruppe bestehend aus 1,2-Oxazol, 1,3-Oxazol, 1,2-Thiazol, 1,3-Thiazol, Pyrazol, Imidazol, 1,2,4-Triazol oder 1,2,4-Oxadiazol,

$R^4$, $R^5$, $R^6$ und $R^7$ unabhängig voneinander Wasserstoff, Halogen, Cyano, Nitro, Alkyl, Cycloalkyl, Alkenyl, Cycloalkenyl, Alkinyl, Alkoxy, Cycloalkoxy, Alkenyloxy, Cycloalkenyloxy, Alkinyloxy, Alkylthio, Cycloalkylthio, Alkenylthio, Cycloalkenylthio, Alkinylthio, Alkylsulfinyl, Alkenylsulfinyl, Alkinylsulfinyl, Alkylsulfonyl, Alkenylsulfonyl, Alkinylsulfonyl, $NR^8R^9$, $R^8CONR^9$, $R^8R^9NSO_2$ oder Aryl bedeuten, sowie

$R^8$ und $R^9$ unabhängig voneinander Wasserstoff sowie jeweils gegebenenfalls substituiertes Alkyl, Cycloalkyl, Alkenyl oder Alkinyl bedeuten.

Die vorliegende Erfindung umfasst alle Isomere, Atropisomere, geometrische sowie optische Isomere und im Fall der Stickstoff enthaltenden Heterocyclen auch die *N*-Oxide.

Von besonderem Interesse sind heterocyclische, aromatische Amide, d. h. durch aromatische Heterocyclen substituierte Amide, der allgemeinen Formel (I), in der

$R^3$ ein fünfgliedriger aromatischer Ring bedeutet, der ausgewählt ist aus der Gruppe bestehend aus 1,2-Oxazol, 1,3-Oxazol, 1,2-Thiazol, 1,3-Thiazol und Pyrazol.

Bevorzugt sind heterocyclische, aromatische Amide der allgemeinen Formel (I), in der

$R^4$, $R^5$, $R^6$ und $R^7$ unabhängig voneinander Wasserstoff, Halogen, Cyano, Nitro, $(C_1$- $C_6)$-Alkyl, wobei der Alkylrest unsubstituiert ist oder durch einen oder mehrere Substituenten, ausgewählt aus der aus Halogen, Cyano, $(C_1$-$C_4)$-Alkoxy oder $(C_1$-$C_4)$-Alkylthio bestehenden Gruppe, substituiert ist, $(C_3$-$C_7)$-Cycloalkyl, wobei der Cycloalkylrest unsubstituiert ist oder durch einen oder mehrere Substituenten, ausgewählt aus der aus Halogen, Cyano, $(C_1$-$C_4)$-Alkyl, $(C_1$- $C_4)$-Alkoxy oder $(C_1$-$C_4)$-Alkylthio bestehenden Gruppe, substituiert ist, $(C_2$- $C_6)$-Alkenyl, wobei der Alkenylrest unsubstituiert ist oder durch einen oder mehrere Substituenten, ausgewählt aus der aus Halogen, Cyano, $(C_1$-$C_4)$-Alkoxy oder $(C_1$-$C_4)$-Alkylthio bestehenden Gruppe, substituiert ist, $(C_3$-$C_7)$- Cycloalkenyl, wobei der Cycloalkenylrest unsubstituiert ist oder durch einen oder mehrere Substituenten, ausgewählt aus der aus Halogen, Cyano, $(C_1$- $C_4)$-Alkyl, $(C_1$-$C_4)$-Alkoxy oder $(C_1$-$C_4)$-Alkylthio bestehenden Gruppe, substituiert ist, $(C_2$-$C_6)$-Alkinyl, wobei der Alkinylrest unsubstituiert ist oder durch einen oder mehrere Substituenten, ausgewählt aus der aus Halogen, Cyano, $(C_1$-$C_4)$-Alkoxy oder $(C_1$-$C_4)$-Alkylthio bestehenden Gruppe, substituiert ist, $(C_1$-$C_6)$-Alkoxy, wobei der Alkoxyrest unsubstituiert ist oder durch einen oder mehrere Substituenten, ausgewählt aus der aus Halogen, Cyano, $(C_1$-$C_4)$-Alkoxy oder $(C_1$-$C_4)$-Alkylthio bestehenden Gruppe, substituiert ist, $(C_3$-$C_7)$-Cycloalkoxy, wobei der Cycloalkoxyrest unsubstituiert ist oder durch einen oder mehrere Substituenten, ausgewählt aus der aus Halogen, Cyano, $(C_1$-$C_4)$-Alkyl, $(C_1$-$C_4)$-Alkoxy oder $(C_1$-$C_4)$-Alkylthio bestehenden Gruppe, substituiert ist, $(C_2$-$C_6)$-Alkenyloxy, wobei der Alkenyloxyrest unsubstituiert ist oder durch einen oder mehrere Substituenten, ausgewählt aus der aus Halogen, Cyano, $(C_1$-$C_4)$-Alkoxy oder $(C_1$-$C_4)$- Alkylthio bestehenden Gruppe, substituiert ist, $(C_3$-$C_7)$-Cycloalkenyloxy, wobei der Cycloalkenyloxyrest unsubstituiert ist oder durch einen oder mehrere Substituenten, ausgewählt aus der aus Halogen, Cyano, $(C_1$-$C_4)$-Alkyl, $(C_1$- $C_4)$-Alkoxy oder $(C_1$-$C_4)$-Alkylthio

bestehenden Gruppe, substituiert ist, ($C_2$- $C_6$)-Alkinyloxy, wobei der Alkinyloxyrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_1$- $C_6$)-Alkylthio, wobei der Alkylthiorest unsubstituiert ist oder durch einen oder mehrere Substituenten, ausgewählt aus der aus Halogen, Cyano, ($C_1$-$C_4$)- Alkoxy oder ($C_1$-$C_4$)-Alkylthio bestehenden Gruppe, substituiert ist, ($C_3$-$C_7$)- Cycloalkylthio, wobei der Cycloalkylthiorest unsubstituiert ist oder durch einen oder mehrere Substituenten, ausgewählt aus der aus Halogen, Cyano, ($C_1$- $C_4$)-Alkyl, ($C_1$-$C_4$)-Alkoxy oder ($C_1$-$C_4$)-Alkylthio bestehenden Gruppe, substituiert ist, ($C_2$-$C_6$)-Alkenylthio, wobei der Alkenylthiorest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_3$-$C_7$)-Cycloalkenylthio, wobei der Cycloalkenylthiorest unsubstituiert ist oder durch einen oder mehrere Substituenten, ausgewählt aus der aus Halogen, Cyano, ($C_1$-$C_4$)-Alkyl, ($C_1$-$C_4$)-Alkoxy oder ($C_1$-$C_4$)- Alkylthio bestehenden Gruppe, substituiert ist, ($C_2$-$C_6$)-Alkinylthio, wobei der Alkinylthiorest unsubstituiert ist oder durch einen oder mehrere Substituenten, ausgewählt aus der aus Halogen, Cyano, ($C_1$-$C_4$)-Alkoxy oder ($C_1$-$C_4$)- Alkylthio bestehenden Gruppe, substituiert ist, ($C_1$-$C_6$)-Alkylsulfinyl, wobei der Alkylsulfinylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_2$-$C_6$)-Alkenylsulfinyl, wobei der Alkenylsulfinylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_2$-$C_6$)-Alkinylsulfinyl, wobei der Alkinylsulfinylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_1$-$C_6$)-Alkylsulfonyl wobei der Alkylsulfonylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_2$-$C_6$)-Alkenylsulfonyl wobei der Alkenylsulfonylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_2$-$C_6$)-Alkinylsulfonyl wobei der Alkinylsulfonylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, $NR^8R^9$, $R^8CONR^9$, $R^8R^9NSO_2$ oder gegebenenfalls substituiertes Aryl bedeuten.

Ganz besonders bevorzugt sind Verbindungen der allgemeinen Formel (I), worin

$R^4$, $R^5$,$R^6$ und $R^7$ unabhängig voneinander Wasserstoff, Halogen, Cyano, Nitro, ($C_1$- $C_5$)-Alkyl, Dihalogenalkyl und Trihalogenalkyl, ($C_1$-$C_3$)-Alkoxy-($C_1$-$C_5$)-alkyl, ($C_1$-$C_5$)-Alkoxy, Dihalogenalkoxy und Trihalogenalkoxy, ($C_1$-$C_3$)-Alkoxy-($C_1$- $C_5$)-alkoxy, ($C_1$-$C_3$)-Alkenyloxy, ($C_1$-$C_3$)-Alkinyloxy, ($C_1$-$C_5$)-Alkylthio, Dihalogenalkylthio und Trihalogenalkylthio, ($C_1$-$C_5$)-Alkylsulfinyl, ($C_1$-$C_5$)- Alkylsulfonyl und ($C_1$-$C_5$)-Dialkylamino bedeuten.

Am meisten bevorzugt sind Verbindungen der allgemeinen Formel (I), in der

$R^3$ 1,2-Oxazol, und

$R^4$, $R^5$,$R^6$ und $R^7$ unabhängig voneinander Wasserstoff, Fluor, Chlor, Brom, Cyano, Nitro, ($C_1$-$C_4$)-Alkyl, Difluormethyl und Trifluormethyl, ($C_1$-$C_4$)-Alkoxy, Difluormethoxy und Trifluormethoxy, ($C_1$-$C_4$)-Alkylthio, Difluormethylthio, Trifluormethylthio, ($C_1$-$C_2$)-Sulfinyl, ($C_1$-$C_2$)-Sulfonyl und ($C_1$-$C_2$)-Dialkylamino

bedeuten.

Ebenfalls bevorzugt sind heterocyclische, aromatische Amide der allgemeinen Formel (I), in der

$R^8$ und $R^9$ unabhängig voneinander Wasserstoff, ($C_1$-$C_6$)-Alkyl bedeuten, wobei der Alkylrest unsubstituiert ist oder durch einen oder mehrere Substituenten, ausgewählt aus der aus Halogen, Cyano, ($C_1$-$C_4$)-Alkoxy oder ($C_1$-$C_4$)- Alkylthio bestehenden Gruppe, substituiert ist, ($C_3$-$C_7$)-Cycloalkyl, wobei der Cycloalkylrest unsubstituiert ist oder durch einen oder mehrere Substituenten, ausgewählt aus der aus Halogen, Cyano, ($C_1$-$C_4$)-Alkyl, ($C_1$-$C_4$)-Alkoxy oder ($C_1$-$C_4$)-Alkylthio bestehenden Gruppe, substituiert ist, ($C_2$-$C_6$)-Alkenyl, wobei der Alkenylrest unsubstituiert ist oder durch einen oder mehrere Substituenten, ausgewählt aus der aus Halogen, Cyano, ($C_1$-$C_4$)-Alkoxy oder ($C_1$-$C_4$)- Alkylthio bestehenden Gruppe, substituiert ist, ($C_2$-$C_6$)-Alkinyl, wobei der Alkinylrest unsubstituiert ist oder durch einen oder mehrere Substituenten,

ausgewählt aus der aus Halogen, Cyano, $(C_1-C_4)$-Alkoxy oder $(C_1-C_4)$- Alkylthio bestehenden Gruppe, substituiert ist, bedeuten.

Besonders bevorzugt sind heterocyclische, aromatische Amide der allgemeinen Formel (I), worin

Q ein heterocyclischer, aromatischer Rest ist, der aus der Q1-Q12 bestehenden Gruppe

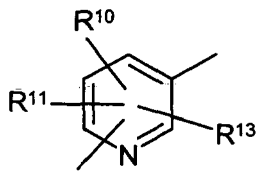
(Q1)

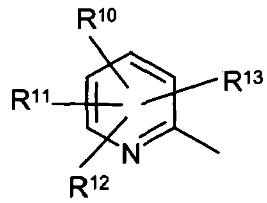
(Q2)

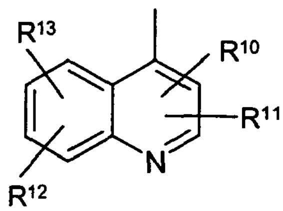
(Q3)

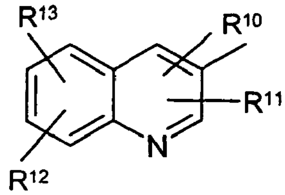
(Q4)

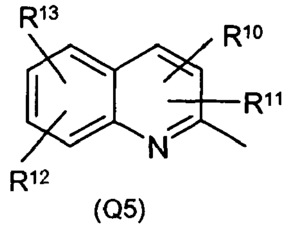
(Q5)

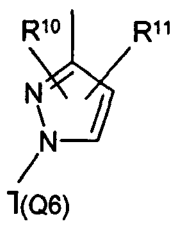
(Q6)

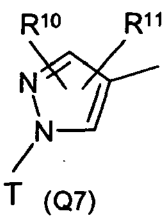
(Q7)

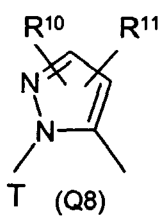
(Q8)

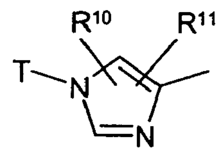
(Q9)

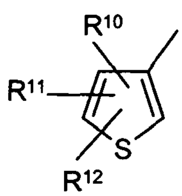
(Q10)

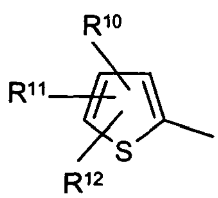
(Q11)

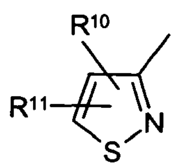
(Q12)

ausgewählt ist, und unsubstituiert ist oder durch einen oder mehrere Substituenten, ausgewählt aus der aus $R^{10}$, $R^{11}$, $R^{12}$ oder $R^{13}$ bestehenden Gruppe, substituiert ist, wobei

$R^{10}$, $R^{11}$,$R^{12}$ oder $R^{13}$ unabhängig voneinander die Bedeutung von $R^4$, $R^5$, $R^6$ und $R^7$ haben, und T ein unsubstituiertes $(C_1-C_6)$-Alkyl, Benzyl oder Phenyl bedeutet.

Die Erfindung betrifft auch Amine der allgemeinen Formel (IVa) sowie deren N-Oxide oder Salze

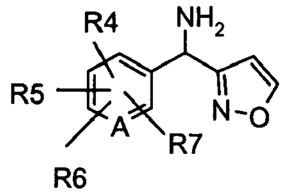

(IVa)

worin

A = CH oder N ist, und
$R^4$, $R^5$, $R^6$ und $R^7$ die in einem der Ansprüche 1, 3, 4 oder 5 angegebene Bedeutung haben.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Alkyl bedeutet einen geradkettigen oder verzweigten offenkettigen Kohlenwasserstoffrest.

Der Ausdruck "($C_1$-$C_4$)Alkyl" bedeutet eine Kurzschreibweise für Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "($C_1$-$C_6$)Alkyl", umfassen entsprechend auch gradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.
Alkenyl schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl.

Alkinyl schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.

Alkenyl bedeutet z.B. Vinyl, welches ggf. durch weitere Alkylreste substituiert sein kann, z.B. Prop-1-en-1-yl, But-1-en-1-yl, Allyl, 1-Methyl-prop-2-en-1-yl, 2-Methylprop-2-en-1-yl, But-2-en-1-yl, 1-Methyl-but-3-en-1-yl und 1-Methyl-but-2-en-1-yl, 2-Methylprop-1-en-1-yl, 1-Methylprop-1-en-1-yl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl, Pentenyl, 2-Methylpentenyl oder Hexenyl.

($C_2$-$C_6$)-Alkynyl bedeutet beispielsweise Ethinyl, Propargyl, 1-Methyl-prop-2-in-1-yl, 2-Butinyl, 2-Pentinyl oder 2-Hexinyl, vorzugsweise Propargyl, But-2-in-1-yl, But-3-in-1-yl oder 1-Methyl-but-3-in-1-yl.

Cycloalkyl bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind.
Im Falle von gegebenenfalls substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Bicyclo[2.2.1]hept-2-yl (Norbornyl), Adamantan-1-yl und Adamantan-2-yl.

Im Falle von substituiertem Cycloalkyl werden auch spirocyclische aliphatische Systeme umfaßt, wie beispielsweise Spiro[2.2]pent-1-yl, Spiro[2.3]hex-1-yl, Spiro[2.3]hex-4-yl, 3-Spiro[2.3]hex-5-yl.

Cycloalkenyl bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Aryl bedeutet ein mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl. Vom Begriff ,,gegebenenfalls substituierte Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist.

Von der Systematik her ist Aryl in der Regel auch von dem Begriff "gegebenenfalls substituiertes Phenyl"umfasst.

Alkoxy bedeutet ein über ein Sauerstoffatom gebundenen Alkylrest, Alkenyloxy bedeutet ein über ein Sauerstoffatom gebundenen Alkenylrest, Alkinyloxy bedeutet ein über ein Sauerstoffatom gebundenen Alkinylrest, Cycloalkyloxy bedeutet ein über ein Sauerstoffatom gebundenen Cycloalkylrest und Cycloalkenyloxy bedeutet ein über ein Sauerstoffatom gebundenen Cycloalkenylrest.

Alkylthio bedeutet ein über ein Schwefelatom gebundenen Alkylrest, Alkenylthio bedeutet ein über ein Schwefelatom gebundenen Alkenylrest, Alkinylthio bedeutet ein über ein Schwefelatom gebundenen Alkinylrest, Cycloalkylthio bedeutet ein über ein Schwefelatom gebundenen Cycloalkylrest und Cycloalkenylthio bedeutet ein über ein Schwefelatom gebundenen Cycloalkenylrest.

Haloalkyl, -alkenyl und -alkinyl bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie $CH_2CH_2Cl$, $CH_2CH_2F$, $CHClCH_3$, $CHFCH_3$, $CH_2Cl$, $CH_2F$; Perhaloalkyl wie $CCl_3$ oder $CF_3$ oder $CF_2CF_3$; Polyhaloalkyl wie $CHF_2$, $CH_2F$, $CH_2CHFCl$, $CHCl_2$, $CF_2CF_2H$, $CH_2CF_3$,; Haloalkoxy ist z.B. $OCF_3$, $OCHF_2$, $OCH_2F$, $OCF_2CF_3$, $OCH_2CF_3$ und $OCH_2CH_2Cl$; Entsprechendes gilt für Haloalkenyl und andere durch Halogen substituierten Reste.

Mit der Definition "mit einem oder mehreren Resten substituiert ist" sind, wenn nicht anders definiert, unabhängig voneinander ein oder mehrere gleiche oder verschiedene Reste gemeint, wobei zwei oder mehrere Reste an einem Cyclus als Grundkörper einen oder mehrere Ringe bilden können.

Substituierte Reste, wie ein substituierter Alkyl-, Alkenyl-, Alkinyl-, Cycloalkyl-, Cycloalkenyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfinylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylthioalkyl, Alkoxyalkyl, gegebenfalls substituiertes Mono- und Dialkylaminoalkyl und Hydroxyalkyl bedeuten.

Im Begriff "substituierte Reste" wie substituiertes Alkyl etc. sind als Substituenten neben den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und Dialkenylaminocarbonyl, Mono- und Dialkinylaminocarbonyl, Mono- und Dialkenylamino, Mono- und Dialkinylamino, Trialkenylsilyl, Trialkinylsilyl, gegebenenfalls substituiertes Cycloalkenyl, gegebenenfalls substituiertes Cycloalkinyl, Phenyl, Phenoxy etc. eingeschlossen. Im Falle von substituierten cyclischen Resten mit aliphatischen Anteilen im Ring sind auch cyclische Systeme mit jenen Substituenten umfaßt, die mit einer Doppelbindung am Ring gebunden sind, z. B. mit einer Alkylidengruppe wie Methyliden oder Ethyliden oder einer Oxogruppe, Iminogruppe oder substituierten Iminogruppe substituiert sind.

Wenn zwei oder mehrere Reste einen oder mehrere Ringe bilden, so können diese carbocyclisch, heterocyclisch, gesättigt, teilgesättigt, ungesättigt, beispielsweise auch aromatisch und gegebenenfalls weiter substituiert sein. Die annellierten Ringe sind vorzugsweise 5- oder 6-Ringe, besonders bevorzugt sind benzokondensierte Cyclen.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind beispielsweise Amino, Hydroxy, Halogen, Nitro, Cyano, Isocyano, Mercapto, Isothiocyanato, Carboxy, Carbonamid, $SF_5$, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfenyl, Alkylsulfinyl, wobei beide Enantiomere der Alkylsulfinylgruppe umfasst sind, Alkylsulfonyl, Monoalkyl-aminosulfonyl, Dialkyl-aminosulfonyl, Alkylphosphinyl, Alkylphosphonyl, wobei für Alkylphosphinyl bzw. Alkylphosphonyl beide Enantiomere umfasst sind, N-Alkyl-aminocarbonyl, N,N-Dialkyl-aminocarbonyl, N-Alkanoyl-aminocarbonyl, N-Alkanoyl-N-alkylaminocarbonyl, Aryl, Aryloxy, Benzyl, Benzyloxy, Benzylthio, Arylthio, Arylamino, Benzylamino, Heterocyclyl und Trialkylsilyl.

Substituenten, die aus mehreren Substituentenebenen zusammengesetzt sind, sind bevorzugt Alkoxyalkyl, Alkylthioalkyl, Alkylthioalkoxy, Alkoxyalkoxy, Phenethyl, Benzyloxy, Haloalkyl, Haloalkoxy, Haloalkylthio, Haloalkanoyl, Haloalkylcarbonyl, Haloalkoxycarbonyl, Haloalkoxyalkoxy, Haloalkoxyalkylthio, Haloalkoxyalkanoyl, Haloalkoxyalkyl.

Kollektionen aus Verbindungen der Formel I und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Bamstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harzen unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und A. Stadler), Verlag Wiley, 2005.

Die Herstellung gemäß der hier beschriebenen Verfahren liefert Verbindungen der Formel I und deren Salze in Form von Substanzkollektionen, die "Bibliotheken" genannt werden. Gegenstand der voliegenden Erfindung sind auch Bibliotheken, die mindestens zwei Verbindungen der Formel I und deren Salzen enthalten.

Heterocyclisch substituierte Amide der allgemeinen Formel I können ein oder mehrere Chiraltätszentren aufweisen, weshalb sie als Racemat, als Enantiomerengemisch, als eines der beiden Enantiomere, als Diastereomerengemisch oder als eines von mehreren Diastereomeren vorliegen können.

Die Trennung der Enantiomeren erfolgt durch dem Fachmann bekannte Methoden, wie zum Beispiel durch Chromatograhie an chiralen Sorbentien. Auch Diasteromere lassen sich durch dem Fachmannn bekannte Methoden wie Kristallisation oder Chromatographie trennen.

Die chromatographische Trennung kann sowohl im analytischen Maßstab zur Feststellung des Enantiomerenüberschusses bzw. des

Diastereomerenüberschusses, wie auch im präparativen Maßstab zur Herstellung von Prüfmustern für die biologische Ausprüfung erfolgen.

Handelt es sich um ein Enantiomerengemisch und ist ein Enantiomer im Überschuß vorhanden, so wird dieser

Überschuß als Enantiomerenüberschuß oder ,,enantiomeric excess", abgekürzt ,,ee" bezeichnet, der wie folgt definiert ist:

$$ee = (2X-1) \times 100\%$$

X steht für die Molfraktion (=Molanteile) des im Überschuß vorhandenen Enantiomers in der Mischung. So bedeuten z. B. 40% ee ein Enantiomerenverhältnis von 70:30.

Der ,,ee" kann durch verschiedene physikalische Methoden bestimmt werden, wie zum Beispiel vorher erwähnt, durch Chromatographie an chiralen Sorbentien in der flüssigen wie auch in der Gasphase, NMR-Messungen mit chiralen Derivaten oder durch Bestimmung des spezifischen Drehwerts, was allerdings voraussetzt, dass der spezifische Drehwert des reinen Enantiomeren bekannt ist. Die so bestimmbare optische Reinheit p,

$$p = [\alpha]/[\alpha]_{max}$$

wird in der Praxis oft zur Charakterisierung von Enantiomerengemischen verwendet, wobei $[\alpha]$ der gemessene, spezifische Drehwert von polarisiertem Licht einer bestimmten Frequenz und $[\alpha]_{max}$ der spezifische Drehwert des reinen Enantiomeren ist.

Die mit 100 multiplizierte optische Reinheit wird als optische Ausbeute P bezeichnet und ist dem Enantiomerenüberschuß oder ,,enantiomeric excess", kurz ,,ee" äquivalent:

$$P = p \times 100\%$$

Entsprechendes gilt für die Charakterisierung von Diastereomerengemischen, mit dem Unterschied, dass die Messung des spezifischen Drehwerts nicht zur Charakterisierung des Diastereomerengemischs ausreicht. In diesem Fall können physikalische Methoden, wie zum Beispiel Chromatographie an chiralen Sorbentien in der flüssigen wie auch in der Gasphase, angewandt werden.

Gegenstand der vorliegenden Erfindung sind ferner Verfahren zur Herstellung von Verbindungen der allgemeinen Formel (I), deren *N*-Oxide oder deren Salze, nach einem der Ansprüche 1 bis 7, worin Q, X, $R^1$, $R^2$, $R^3$, $R^4$, $R^5$, $R^6$ und $R^7$ die in einem der Ansprüche 1, 2, 3, 4 oder 5 angegebene Bedeutung haben, und worin $R^8$, $R^9$, $R^{10}$, $R^{11}$, $R^{12}$, $R^{13}$ und T die in einem der Ansprüche 6, 7 oder 8 angegebene Bedeutung haben.

Die Synthese der erfindungsgemäßen Verbindungen erfolgt am zweckmäßigsten aus der betreffenden Carbonsäure (III) und dem jeweiligen Amin (IV):

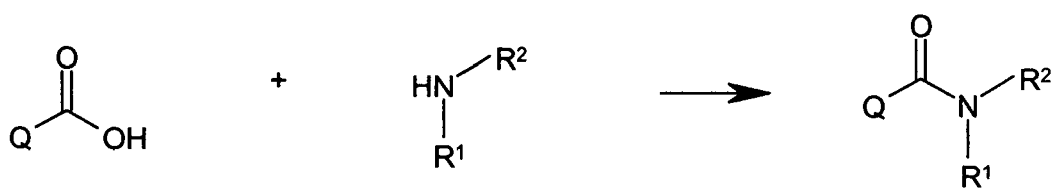

(III) (IV) (I)

Die entsprechenden Thioverbindungen, d.h. X = S, sind auf analoge Weise herstellbar.

Die Aktivierung der Carbonsäure erfolgt mit gängigen Kondensationsragenzien, wie Carbonyldiimidazol (CDI), N-Hydroxy-1,2,3-benzotriazol (HOBt), 1,3-Dicyclohexylcarbidiimid (DCC) oder 1-[3-(Dimethylamino)propyl]-3-ethylcarbodiimid (EDC).

Vorteilhaft können auch polymergebundene Reagenzien wie an Polystyrol gebundenes Cyclohexylcarbodiimid (DCC-Polystyrol) eingesetzt werden.

Die Kondensationsreagenzien werden äquimolar oder bis zum 3-fachen Überschuß eingesetzt, vorzugsweise äquimolar oder bis zum 1.5-fachen Überschuß.

Die Carbonsäuren können auch in die entsprechenden Carbonsäurehalogenide umgewandelt werden, die dann in Gegenwart einer Base mit dem Amin (IV) umgesetzt werden können. Bevorzugt werden die Carbonsäuren (III) in die betreffenden Carbonylchloride oder Carbonylbromide (V) überführt und dann in Gegenwart einer Base mit dem Amin (IV) umgesetzt.

Die Herstellung der Carbonylhalogenide (V) aus den Carbonsäuren (III) erfolgt mit gängigen Halogenierungsreagenzien wie Oxalylchlorid, Thionylchlorid, Phosphoroxychlorid, Oxalylbromid, Thionylbromid oder Phosphoroxybromid.

Zum Binden der entstehenden Säure dienen organische Basen, wieTriethylamin, Pyridin, N-Methylmorpholin aber auch anorganische Basen wie Natriumcarbonat, Kaliumcarbonat oder Kaliumhydroxid, bevorzugt jedoch Triethylamin und Pyridin.

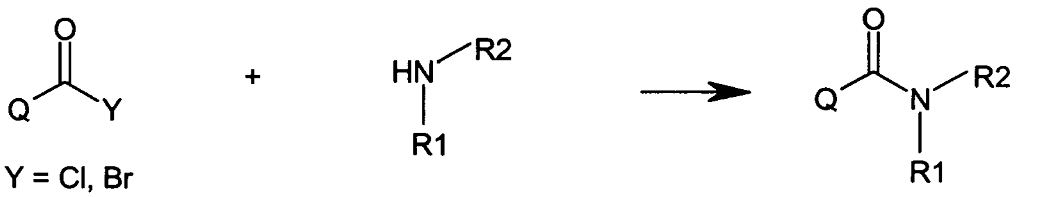

(V) (IV) (I)

Auch polymergebundene Basen wie DMAP-Polystyrol können zum Einsatz kommen.

Die Basen werden äquimolar oder bis zum 4-fachen Überschuß eingesetzt.

Als Lösungsmittel für alle Reaktionen können Ether, wie Diethylether, Diisopropylether, tert-Butyl-methylether, Tetrahydrofuran, 1,4-Dioxan oder Dimethoxyethan, chlorierte Kohlenwasserstoffe wie Dichlormethan, 1,2-Dichlorethan oder Chloroform, aromatische Kohlenwasserstoffe wie Toluol, Xylol oder Chlorbenzol, tertiäre Amide, wie N,N-Dimethylformamid, N,N-Dimethylacetamid oder N-Methylpyrrolidon, oder Nitrile wie Acetonitril verwendet werden.

Die Carbonsäuren der Formeln (III) sind in großer Auswahl kommerziell erhältlich. Speziell substituierte Nicotinsäuren (IIIa) können ebenso wie speziell substiuierte Pyrazolcarbonsäuren (IIIb) und 1,2-Thiazolcarbonsäuren (IIIc) nach den in dem Dokument WO2005/070889 publizierten Vorschriften hergestellt werden.

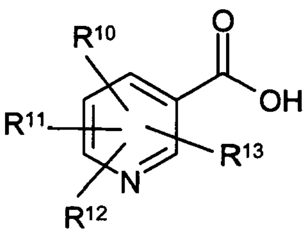

(IIIa)

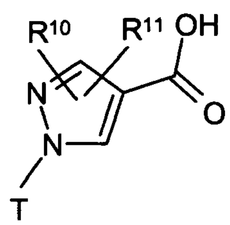

(IIIb)

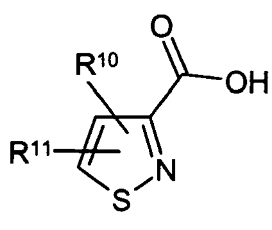

(IIIc)

Thioamide der allgemeinen Formel (I), also Verbindungen, in denen X = S ist, können aus Amiden der allgemeinen Formel (I) (X = O)

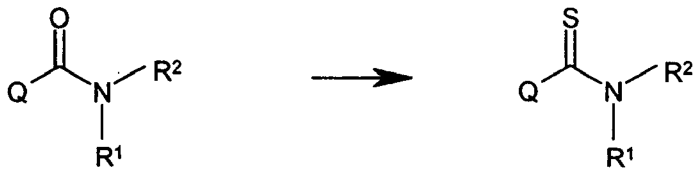

mit Thiolierungsreagenzien wie Diphosphorpentasulfid oder Lawessons Reagenz

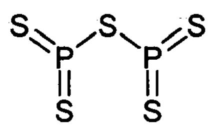

Diphosphorpentasulfid

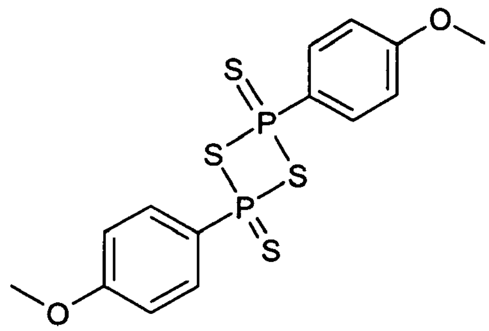

Lawessons Reagenz

erhalten werden.

Verfahren zur Herstellung von Aminen der allgemeinen Formel (IVa) sind ebenfalls Gegenstand der Erfindung. Schema 1 fasst das Gesamtverfahren zur Herstellung von Aminen der allgemeinen Formel (IVa) ausgehend von Acetophenon-Verbindungen, bzw. ihren heterocyclischen Analoga gem. der Formel (VI) zusammen. Die Erfindung betrifft jedoch auch die einzelnen Teilschritte des in Schema 1 zusammengefassten Gesamtverfahrens, d.h. insbesondere die Herstellung von Aminen der allgemeinen Formel (IVa) ausgehend von Verbindungen der Formel (VIII), bzw. ausgehend von Verbindungen der Formel (IX), bzw. ausgehend von Verbindungen der Formel (X).

Gem. Schema 1 können Amine der Formel (IVa), in denen A=CH oder N ist und $R^4$, $R^5$, $R^6$ und $R^7$ die in einem der Ansprüche 1, 2, 3, 4 oder 5 angegebene Bedeutung haben, in mehreren Stufen aus den entsprechenden Acetophenonen oder ihren heterocyclischen Analoga (VI) hergestellt werden. Acetophenone und ihre heterocyclischen Analoga (VI) sind in großer Anzahl und mit den verschiedensten Substitutionsmustern kommerziell erhältlich.

## Schema 1

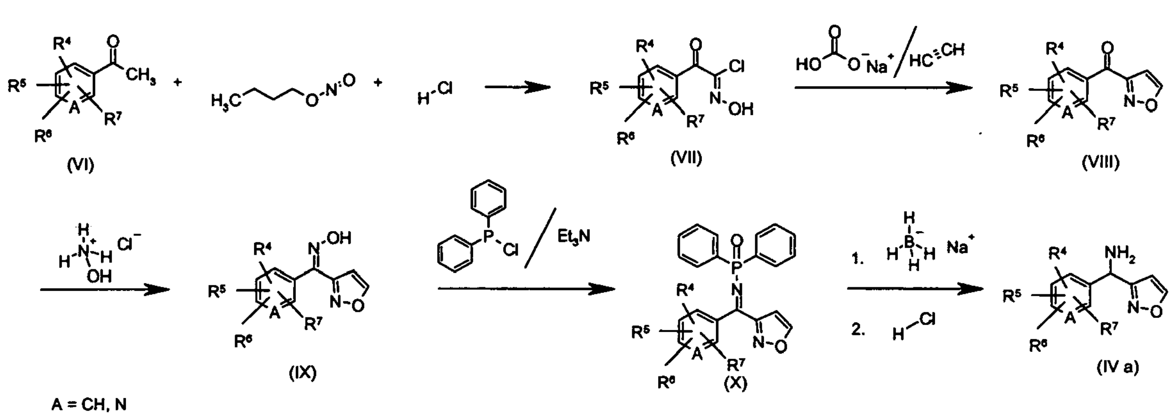

(VI) (VII) (VIII) (IX) (X) (IV a)

A = CH, N

Die Herstellung der Ketone (VIII) ist prinzipiell bekannt (Heterocycles 2002, 57(12), 2299-2308).

So können in einem ersten Schritt Acetophenone oder ihre heterocyclischen Analoga (VI) in saurer Lösung mit Alkylnitriten in die entsprechenden Hydroxamsäurechloride (VII) umgewandelt werden. Als Alkylnitrite kommen zum Beispiel Ethylnitrit, n-Propylnitrit, Isopropylnitrit, n-Butylnitrit, Isobutylnitrit, sec-Butylnitrit, tert-Butylnitrit, n-Amylnitrit, 3-Methylbutylnitrit (Isoamylnitrit), n-Hexylnitrit oder 2-Ethylhexylnitrit zur Verwendung, wobei n-Butyl-nitril bevorzugt ist. Als Lösungsmittel können wasserfreie, etherische Lösungsmittel wie Diethylether, Diisopropylether, Methyl-tert-butylether, Tetrahydrofuran, 1,4-Dioxan oder 1,2-Dimethoxyethan verwendet werden. Als Säure wird zweckmäßigerweise gasförmiger Chlorwasserstoff verwendet.

Die auf diese Weise erhaltenen Hydroxamsäurechloride (VII) werden am besten ohne weitere Reinigung in der nächsten Stufe in Gegenwart einer organischen oder anorganischen Base, vorzugsweise Natriumcarbonat, mit Acetylen umgesetzt, wobei intermediär aus dem Hydroxamsäurechlorid ein Nitriloxid generiert wird, das in einer [1,3]-dipolaren Cycloaddition mit dem Acetylen zu einem 1.2-Oxazol reagiert.

Als organische Base steht eine breite Palette von Reagenzien zur Verfügung, von denen beispielhaft Triethylamin, Diisopropylethylamin, Dicyclohexylamin, N,N-Dimethylanilin, N-Methylmorpholin oder Pyridin genannt seien. Als anorganische Basen können unter anderem Natriumcarbonat, Natriumhydrogencarbonat, Natriumhydroxid, Kaliumcarbonat, Kaliumhydrogencarbonat oder Kaliumhydroxid verwendet werden. Als Lösungsmittel kommen Alkohole, wie zum Beispiel Methanol, Ethanol, n-Propanol, Isopropanol, n-Butanol, Isobutanol oder Kohlenwasserstoffe sowie chlorierte Kohlenwasserstoffe, wie zum Beispiel Heptan, Toluol, Dichlormethan, 1,2-Dichlorethan oder Ether wie zum Beispiel Diethylether, Diisopropylether, Methyl-tert-butylether, Tetrahydrofuran, 1,4-Dioxan,1,2-Dimethoxyethan in Frage.

Die Umsetzung der Ketone (VIII) zu den Oximen (IX) kann mit Hydroxylamin oder seinen Salzen, also zum Beispiel Hydroxylaminsulfat, Hydroxylaminhydrogensulfat, Hydroxylaminnitrat, Hydroxylaminphosphat, Hydroxylaminperchlorat oder Hydroxylaminhydrochlorid vorgenommen werden. Als Lösungsmittel werden Alkohole, wie zum Beispiel Methanol, Ethanol, n-Propanol, Isopropanol, n-Butanol, Isobutanol oder Mischungen dieser Alkohole mit Wasser verwendet. Die Mischungsverhältnisse Alkohol/Wasser betragen dabei 10:1 bis 1:1. Die Oxime (IX) fallen bei dieser Reaktion als E/Z-Gemische an.

Die chemoselektive Reduktion der Oximgruppe in (IX) in Gegenwart des 1,2-Oxazolrings, in dem die N-O-Bindung potentiell ebenfalls reduziert werden kann, gelingt überraschenderweise in einer zweistufigen Reaktion, ohne dass der Isoxazolring geöffnet wird. Eine ähnliche Methodik ist für strukturell völlig andersartige Kohlehydratderivate in Carbohydrate Research 1995, 288, 345-50 beschrieben.

Dazu wird zunächst das Oxim (IX) mit Diarylchlorphosphanen, vorzugsweise Chlordiphenylphosphan, in Gegenwart einer organischen Base, wie zum Beispiel Triethylamin, Düsopropylethylamin, Dicyclohexylamin, N,N-Dimethylanilin, N-Methylmorpholin oder Pyridin, zu einem Diaryl(methylenamino)phosphinsäureamid (X), wobei Triethylamin bevorzugt ist, umgesetzt. Als Lösungsmittel für diese Reaktion kommen Kohlenwasserstoffe sowie chlorierte Kohlenwasserstoffe, wie zum Beispiel Heptan, Toluol, Dichlormethan, 1,2-Dichlorethan oder Gemische aus den beiden vorgenannten Lösungsmitteln sowie Ether, wie zum Beispiel Diethylether, Diisopropylether, Methyl-tert-butylether, Tetrahydrofuran, 1,4-Dioxan,1,2-Dimethoxyethan in Frage, wobei die Umwandlung der Oximverbindungen (IX) in die N-phosphinyliminverbindungen (X), vorzugsweise mit Chlordiphenylphosphan und Triethylamin in Dichlormethan erfolgt.

Die Iminbindung in dem so erhaltenen Diaryl(methylenamino)phosphinsäureamid (X) wird anschließend mit Natriumborhydrid in einem etherischen Lösungsmittel, wie zum Beispiel Diethylether, Diisopropylether, Methyl-tert-butylether, Tetrahydrofuran, 1,4-Dioxan oder 1,2-Dimethoxyethan zu einem Diaryl(methylamino)phosphin-säureamid reduziert, aus welchem durch Kochen mit verdünnter Salzsäure und anschließender Neutralisation das Amin der allgemeinen Formel (IVa) freigesetzt wird.

Gemäß Dokument W02005/070889 kann die Einführung bestimmter Substituenten R zur Herstellung von Verbindungen, in denen $R^{10}$ und/oder $R^{11}$ ($C_1$-$C_6$)-Alkoxy, ($C_3$-$C_7$)-Cycloalkoxy, ($C_1$-$C_6$)-Alkenyloxy, ($C_3$-$C_7$)-Cycloalkenyloxy, ($C_1$-$C_6$)-Alkinyloxy, ($C_1$-$C_6$)-Alkylthio, ($C_3$-$C_7$)-Cycloalkylthio ($C_1$-$C_6$)-Alkenylthio, ($C_3$-$C_7$)-Cycloalkenylthio oder ($C_1$-$C_6$)-Alkinylthio bedeutet, auch auf der Stufe des Carbonsäureamids erfolgen. In Schema 2 ist dies exemplarisch am Beispiel von Nikotinsäureamiden dargestellt.

Schema 2

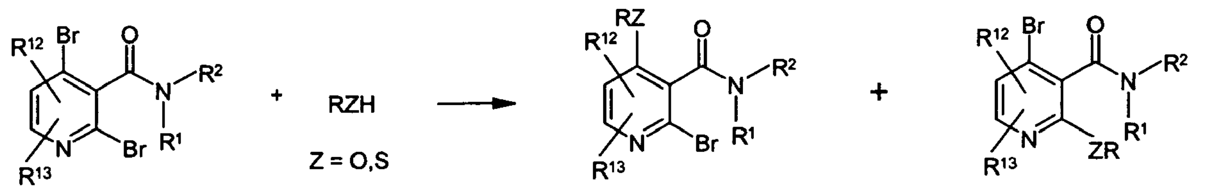


(XI)

Dazu wird ein Alkohol oder ein Thioalkohol (XI) in Gegenwart einer starken Base, wie Natriumhydrid oder Kalium-tert-butylat, in einem polaren Lösungsmittel bei 0-100 ˚C mit den halogensubstituierten Carbonsäureamiden umgesetzt. Die Reaktionsdauer beträgt 1 -24 Stunden.

Im Fall von zweifach mit Halogen substituierten Carbonsäureamiden können, wie in Schema 2 gezeigt, Regioisomere entstehen, die sich durch die dem Fachmann bekannten Methoden, wie Kristallisation oder Chromatographie voneinander trennen lassen.

Als polare Lösungsmittel für diese Reaktion kommen tertiäre Amide, wie N,N-Dimethylformamid, N,N-Dimethylacetamid oder N-Methylpyrrolidon, Ether, wie Diethylether, Diisopropylether, tert-Butyl-methylether, Tetrahydrofuran, 1,4-Dioxan oder Dimethoxyethan, Nitrile wie Acetonitril oder Sulfoxide wie Dimethylsulfoxid in Frage.

Abhängig vom Substituentenmuster können Verbindungen der Formel (I) Salze bilden. Salzbildung kann durch Einwirkung einer Base auf solche Verbindungen der Formel (I) erfolgen, die ein acides Wasserstoffatom tragen. Geeignete Basen sind beispielsweise organische Amine, wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel $[NRR'R''R''']^+$, worin R bis R''' jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie $(C_1$-$C_4)$-Trialkylsulfonium- und $(C_1$-$C_4)$-Trialkylsulfoxoniumsalze.

Besitzen die Verbindungen der Formel (I) ein basisches Zentrum, so können sie durch Anlagerung einer geeigneten anorganischen oder organischen Säure, wie Mineralsäuren, wie zum Beispiel HCl, HBr, $H_2SO_4$, $H_3PO_4$ oder $HNO_3$, oder organischen Säuren, wie zum Beispiel Ameisensäure, Essigsäure, Propionsäure, Oxalsäure, Milchsäure oder Salicylsäure oder Sulfonsäuren, wie zum Beispiel p-Toluolsulfonsäure, an die basische Gruppe Salze bilden. Diese Salze enthalten dann die konjugierte Base der Säure als Anion.

Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden.

Die erfindungsgemäßen Verbindungen der Formel (I) und/oder deren Salze, im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Eine wirksame Menge einer oder mehrerer der Verbindungen der Formel (I) oder eines ihrer *N*-Oxide oder eines ihrer Salze können in Form von Spritzpulvern, emulgierbaren Konzentraten, versprühbaren Lösungen, Stäubemitteln oder Granulaten in den üblichen Zubereitungen angewendet werden. Es ist vorgesehen, dass die Zusammensetzung mindestens eines der im Pflanzenschutz üblichen Formulierungshilfsmittels enthalten kann.

Gegenstand der Erfindung sind auch pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenem, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration

etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll. Monokotyle Schadpflanzen umfassen die Gattungen Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Emteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel I oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel I als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen

- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259),
- transgene Kulturpflanzen mit modifizierter Fettsäurezusammensetzung (WO 91/13972),
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461),
- gentechnisch veränderte Pflanzen mit reduzierter Photorespiration, die höhere Erträge und höhere Stresstoleranz aufweisen (EPA 0305398),
- Transgene Kulturpflanzen, die pharmazeutisch oder diagnostisch wichtige Proteine produzieren ("molecular pharming"),
- transgene Kulturpflanzen, die sich durch höhere Erträge oder bessere Qualitat auszeichnen,
- transgene Kulturpflanzen die sich durch eine Kombinationen z. B. der o. g. neuen Eigenschaften auszeichnen ("gene stacking").

Zahlreiche molekularbiologische Techniken, mit denen neue transgene Pflanzen mit veränderten Eigenschaften hergestellt werden können, sind dem Prinzip nach bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die

Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel I als Herbizide zur Bekämpfung von Schadpflanzen in transgenen Kulturpflanzen.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 13th edition, The British Crop Protection Council and the Royal Soc. of Chemistry, 2003 und dort zitierter Literatur beschrieben sind.

Als bekannte Herbizide, die mit den erfindungsgemäßen Verbindungen kombiniert werden können, sind z.B. folgende Wirkstoffe zu nennen (Anmerkung: Die Verbindungen sind entweder mit dem "common name" nach der International Organization for Standardization (ISO) oder mit einer üblichen Codenummer zusammen mit dem chemischen Namen bezeichnet) und umfassen stets sämtliche Anwendungsformen wie Säuren, Salze, Ester und Isomere wie Stereoisomere und optische Isomere. Dabei sind eine und zum Teil auch mehrere Anwendungsformen genannt:

2,4-D, Acetochlor, Acifluorfen, Acifluorfen-sodium, Aclonifen, Alachlor, Alloxydim, Alloxydim-sodium, Ametryn, Amicarbazone, Amidosulfuron, Aminopyralid, Amitrole, Anilofos, Asulam, Atrazine, Azafenidin, Azimsulfuron, Beflubutamid, Benazolin, Benazolin-ethyl, Bencarbazone, Benfuresate, Bensulfuron-methyl, Bentazone, Benzfendizone, Benzobicyclon, Benzofenap, Bifenox, Bilanafos, Bispyribac-natrium, Bromacil, Bromobutide, Bromofenoxim, Bromoxynil, Butachlor, Butafenacil, Butenachlor, Butralin, Butroxydim, Butylate, Cafenstrole, Carbetamide, Carfentrazone-ethyl, Chlomethoxyfen, Chloridazon, Chlorimuron-ethyl, Chlornitrofen, Chlorotoluron, Chlorsulfuron, Cinidon-ethyl, Cinmethylin, Cinosulfuron, Clefoxydim, Clethodim, Clodinafop-propargyl, Clomazone, Clomeprop, Clopyralid, Cloransulammethyl, Cumyluron, Cyanazine, Cyclosulfamuron, Cycloxydim, Cyhalofop-butyl, Desmedipham, Dicamba, Dichlobenil, Dichlorprop, Dichlorprop-P, Diclofop-methyl, Diclosulam, Difenzoquat, Diflufenican, Diflufenzopyr, Dikegulac-sodium, Dimefuron, Dimepiperate, Dimethachlor, Dimethametryn, Dimethenamid, Triaziflam, Diquatdibromide, Dithiopyr, Diuron, Dymron, EPTC, Esprocarb, Ethalfluralin, Ethametsulfuron-methyl, Ethofumesate, Ethoxyfen, Ethoxysulfuron, Etobenzanid, Fenoxaprop-ethyl, Fenoxaprop-P-ethyl, Fentrazamide, Flamprop-M-isopropyl, Flamprop-M-methyl, Flazasulfuron, Florasulam, Fluazifop, Fluazifop-butyl, Fluazifop--butyl, Fluazolate, Flucarbazone-sodium, Flucetosulfuron, Fluchloralin, Flufenacet, Flufenpyr, Flumetsulam, Flumiclorac-pentyl, Flumioxazin, Fluometuron, Fluorochloridone, Fluoroglycofen-ethyl, Flupoxam, Flupyrsulfuron-methyl-sodium, Fluridone, Fluroxypyr, Fluroxypyr-butoxypropyl, Fluroxypyr-meptyl, Flurprimidol, Flurtamone, Fluthiacet-methyl, Fomesafen, Foramsulfuron, Glufosinate, Glufosinateammonium, Glyphosate, Halosulfuron-methyl, Haloxyfop, Haloxyfop-ethoxyethyl, Haloxyfop-methyl, Haloxyfop-P-methyl, Hexazinone, HOK-201 (d.h. N-(2,4-Difluorphenyl)-N-(1-methylethyl)-5-oxo-1-(tetrahydro-2H-pyran-2-ylmethyl)-1,5-dihydro-4H-1,2,4-triazol-4-carboxamid), IDH-100 (d.h. (5-Hydroxy-1-methyl-1H-pyrazol-4-yl)(3,3,4-trimethyl-1,1-dioxido-2,3-dihydro-1-benzothiophen-5-yl)methanon), Imazamethabenz-methyl, Imazamox, Imazapic, Imazapyr, Imazaquin, Imazethapyr, Imazosulfuron, Indanofan, Iodosulfuron-methyl-natrium, loxynil, Isoproturon, Isouron, Isoxaben, Isoxachlortole, Isoxaflutole, Ketospiradox, Lactofen, Lenacil, Linuron, MCPA, Mecoprop, Mecoprop-P, Mefenacet, Mesosulfuron-methyl, Mesotrione, Metamifop, Metamitron, Metazachlor, Methabenzthiazuron, Methyldymron, Metobromuron, Metolachlor, Metosulam, Metoxuron, Metribuzin, Metsulfuron-methyl, Molinate, Monolinuron, MonosulfuronNaproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonic acid, Pendimethalin, Pendralin, Penoxsulam, Pentoxazone, Pethoxamid, Phenmedipham, Picloram, Picolinafen, Pinoxaden, Piperophos, Pretilachlor, Primisulfuron-methyl, Profluazol, Profoxydim, Prohexadione-calcium, Prometryn, Propachlor, Propanil, Propaquizafop, Propisochlor, Propoxycarbazone-sodium, Propyzamide, Prosulfocarb, Prosulfuron, Pyraclonil, Pyraflufen-ethyl, Pyrasulfotole, Pyrazolate, Pyrazosulfuron-ethyl, Pyrazoxyfen, Pyribambenz-isopropyl, Pyribenzoxim, Pyributicarb, Pyridafol, Pyridate, Pyriftalid, Pyriminobac-methyl, Pyrimisulfan, Pyrithiobac-sodium, Pyroxasulfone, Pyroxsulam, Quinclorac, Quinmerac, Quinoclamine, Quizalofop-ethyl, Quizalofop-P-ethyl, Quizalofop-P-tefuryl, Rimsulfuron, Sethoxydim, Simazine, Simetryn, S-Metolachlor, Sulcotrione, Sulfentrazone, Sulfometuron-methyl, Sulfosate, Sulfosulfuron, SYN-523 (d.h. Ethyl-[(3-{2-chlor-4-fluor-5-[3-methyl-2,6-dioxo-4-(trifluormethyl)-3,6-dihydropyrimidin-1(2H)-yl]phenoxy}pyridin-2-yl)oxy]acetate), SYP-249 (d.h. 1-(Ethoxycarbonyl)2-methylprop-2-en-1-yl-5-[2-chlor-4-(trifluormethyl)phenoxy]-2-nitrobenzolcarboxylat), Tebuthiuron, Tembotrione, Tepraloxydim, Terbuthylazine, Terbutryn, Terfuryltrione, Thenylchlor, Thiazopyr, Thiencarbazone-methyl, Thifensulfuron-methyl, Thiobencarb, Tiocarbazil, Tralkoxydim, Triallate, Triasulfuron, Tribenuron-methyl, Triclopyr, Tridiphane, Trifloxysulfuron, Trifluralin, Triflusulfuron-methyl und Tritosulfuron.

Mischungsformulierungen erfindungsgemäßer Verbindungen der allgemeinen Formel (I) können auch Fungizide und/oder Insektizide enthalten.

Von besonderem Interesse ist die selektive Bekämpfung von Schadpflanzen in Kulturen von Nutz- und Zierpflanzen. Obwohl die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten.

Diesbezüglich sind Kombinationen erfindungsgemäßer Verbindungen der allgemeinen Formel (I) von besonderem Interesse, welche Verbindungen der allgemeinen Formel (I) bzw. deren Kombinationen mit anderen Herbiziden oder Pestiziden und Safenem enthalten.

Die Safener, welche mit einem antidotisch wirksamen Gehalt eingesetzt werden, reduzieren die phytotoxischen Nebenwirkungen der eingesetzten Herbizide bzw. Herbizid-Pestizid-Kombinationen, z.B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide.

Folgende Gruppen von Verbindungen kommen beispielsweise als Safener in Frage:

S1) Verbindungen aus der Gruppe heterocyclischer Carbonsäurederivate:

S1[a]) Verbindungen vom Typ der Dichlorphenylpyrazolin-3-carbonsäure (S1[a]), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl-5-(ethoxycarbonyl)-5-methyl-2-pyrazolin-3-carbonsäure-ethylester (S1-1) ("Mefenpyr-diethyl"), und verwandte Verbindungen, wie sie in der WO-A-91/07874 beschrieben sind;

S1[b]) Derivate der Dichlorphenylpyrazolcarbonsäure (S1[b]), vorzugsweise Verbindungen wie 1-(2,4-Dichlorphenyl)-5-methylpyrazol-3-carbonsäureethylester (S1-2), 1-(2,4-Dichlorphenyl)-5-isopropylpyrazol-3-carbonsäureethylester (S1-3), 1-(2,4-Dichlorphenyl)-5-(1,1-dimethyl-ethyl)pyrazol-3-carbonsäure-ethylester (S1-4) und verwandte Verbindungen, wie sie in EP-A-333 131 und EP-A-269 806 beschrieben sind;

S1[c]) Derivate der 1,5-Diphenylpyrazol-3-carbonsäure (S1[c]), vorzugsweise Verbindungen wie 1-(2,4-Dichlorphenyl)-5-phenylpyrazol-3-carbonsäure-ethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;

S1[d]) Verbindungen vom Typ der Triazolcarbonsäuren (S1[d]), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen, wie sie in EP-A-174 562 und EP-A-346 620 beschrieben sind;

S1[e]) Verbindungen vom Typ der 5-Benzyl- oder 5-Phenyl-2-isoxazolin-3- carbonsäure, oder der 5,5-Diphenyl-2-isoxazolin-3-carbonsäure(S1[e]), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.

S2) Verbindungen aus der Gruppe der 8-Chinolinyloxyderivate (S2):

S2[a]) Verbindungen vom Typ der 8-Chinolinoxyessigsäure (S2[a]), vorzugsweise (5-Chlor-8-chinolinoxy)essigsäure-(1-methylhexyl)-ester ("Cloquintocetmexyl") (S2-1), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;

S2[b]) Verbindungen vom Typ der (5-Chlor-8-chinolinoxy)malonsäure (S2[b]), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.

S3) Wirkstoffe vom Typ der Dichloracetamide (S3), die häufig als Vorauflaufsafener (bodenwirksame Safener) angewendet werden, wie z. B.
"Dichlormid" (N,N-Diallyl-2,2-dichloracetamid) (S3-1),
"R-29148" (3-Dichloracetyl-2,2,5-trimethyl-1,3-oxazolidin) der Firma Stauffer (S3-2),
"R-28725" (3-Dichloracetyl-2,2,-dimethyl-1 ,3-oxazolidin) der Firma Stauffer (S3-3),
"Benoxacor" (4-Dichloracetyl-3,4-dihydro-3-methyl-2H-1,4-benzoxazin) (S3-4),
"PPG-1292" (N-Allyl-N-[(1,3-dioxolan-2-yl)-methyl]-dichloracetamid) der Firma PPG Industries (S3-5),
"DKA-24" (N-Allyl-N-[(allylaminocarbonyl)methyl]-dichloracetamid) der Firma Sagro-Chem (S3-6),
"AD-67" oder "MON 4660" (3-Dichloracetyl-1-oxa-3-aza-spiro[4,5]decan) der Firma Nitrokemia bzw. Monsanto (S3-7),
"TI-35" (1-Dichloracetyl-azepan) der Firma TRI-Chemical RT (S3-8), "Diclonon" (Dicyclonon) oder "BAS145138" oder "LAB145138" (S3-9)
(3-Dichloracetyl-2,5,5-trimethyl-1,3-diazabicyclo[4.3.0]nonan) der Firma BASF, "Furilazol" oder "MON 13900" ((RS)-3-Dichloracetyl-5-(2-furyl)-2,2-dimethyl-oxazolidin) (S3-10), sowie dessen (R)-Isomer (S3-11).

S4) Verbindungen aus der Klasse der Acylsulfonamide (S4):

S4[a]) N-Acylsulfonamide der Formel (S4[a]) und deren Salze wie sie in der WO-A-97/45016 beschrieben sind,

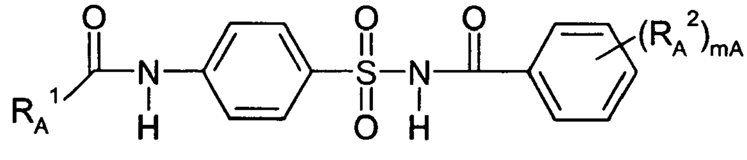

(S4[a])

worin

$R_A{}^1$ $(C_1-C_6)$Alkyl, $(C_3-C_6)$Cycloalkyl, wobei die 2 letztgenannten Reste durch $v_A$ Substituenten aus der Gruppe Halogen, $(C_1-C_4)$Alkoxy, $(C_1-C_6)$Halo- alkoxy und $(C_1-C_4)$Alkylthlo und im Falle cyclischer Reste auch durch $(C_1-C_4)$Alkyl und $(C_1-C_4)$Haloalkyl substituiert sind;

$R_A{}^2$ Halogen, $(C_1-C_4)$Alkyl, $(C_1-C_4)$Alkoxy, $CF_3$;

$m_A$ 1 oder 2;

$v_A$ ist 0, 1, 2 oder 3 bedeuten;

S4[b]) Verbindungen vom Typ der 4-(Benzoylsulfamoyl)benzamide der Formel (S4[b]) und deren Salze, wie sie in der WO-A-99/16744 beschrieben sind,

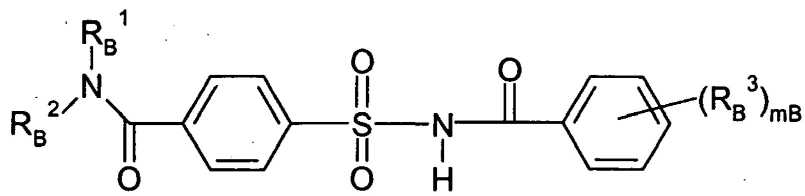

(S4[b])

worin

$R_B{}^1$, $R_B{}^2$ unabhängig voneinander Wasserstoff, $(C_1-C_6)$Alkyl, $(C_3-C_6)$Cycloalkyl, $(C_3-C_6)$Alkenyl, $(C_3-C_6)$Alkinyl,
$R_B{}^3$ Halogen, $(C_1-C_4)$Alkyl, $(C_1-C_4)$Haloalkyl oder $(C_1-C_4)$Alkoxy und
$m_B$ 1 oder 2 bedeuten,
z.B. solche worin
$R_B{}^1$ = Cyclopropyl, $R_B{}^2$ = Wasserstoff und $(R_B{}^3)$ = 2-OMe ist (S4-1, "Cyprosulfamide",),
$R_B{}^1$ = Cyclopropyl, $R_B{}^2$ = Wasserstoff und $(R_B{}^3)$ = 5-Cl-2-OMe ist (S4-2),
$R_B{}^1$ = Ethyl, $R_B{}^2$ = Wasserstoff und $(R_B{}^3)$ = 2-OMe ist (S4-3),
$R_B{}^1$ = Isopropyl, $R_B{}^2$ = Wasserstoff und $(R_B{}^3)$ = 5-Cl-2-OMe ist (S4-4) und
$R_B{}^1$ = Isopropyl, $R_B{}^2$ = Wasserstoff und $(R_B{}^3)$ = 2-OMe ist (S4-5).

S4[c]) Verbindungen aus der Klasse der Benzoylsulfamoylphenylharnstoffe der Formel (S4[c]), wie sie in der EP-A-365484 beschrieben sind

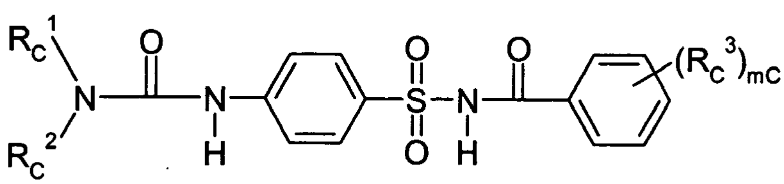

(S4[c])

worin

$R_C{}^1$, $R_C{}^2$ unabhängig voneinander Wasserstoff, $(C_1-C_8)$Alkyl, $(C_3-C_8)$Cycloalkyl, $(C_3-C_6)$Alkenyl, $(C_3-C_6)$Alkinyl,

$R_C{}^3$ Halogen, $(C_1-C_4)$Alkyl, $(C_1-C_4)$Alkoxy, $CF_3$

$m_C$ 1 oder 2 bedeuten;

beispielsweise
1-[4-(N-2-Methoxybenzoylsulfamoyl)phenyl]-3-methylharnstoff,
1-[4-(N-2-Methoxybenzoylsulfamoyl)phenyl]-3,3-dimethylharnstoff,
1-[4-(N-4,5-Dimethylbenzoylsulfamoyl)phenyl]-3-methylharnstoff.

S5) Wirkstoffe aus der Klasse der Hydroxyaromaten und der aromatischaliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.

S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydrochinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.

S7) Verbindungen aus der Klasse der Diphenylmethoxyessigsäurederivate (S7), z.B. Diphenylmethoxyessigsäuremethylester (CAS-Reg.Nr. 41858-19-9) (S7-1), Diphenylmethoxyessigsäureethylester oder Diphenylmethoxyessigsäure wie sie in der WO-A-98/38856 beschrieben sind.

S8) Verbindungen der Formel (S8),wie sie in der WO-A-98/27049 beschrieben sind

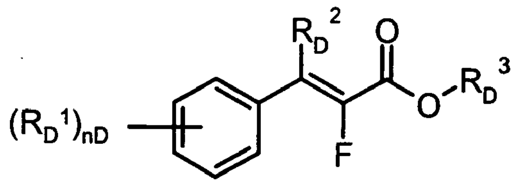

(S8)

worin die Symbole und Indizes folgende Bedeutungen haben:

$R_D^1$ ist Halogen, $(C_1-C_4)$Alkyl, $(C_1-C_4)$Haloalkyl, $(C_1-C_4)$Alkoxy, $(C_1-C_4)$Haloalkoxy,
$R_D^2$ ist Wasserstoff oder $(C_1-C_4)$Alkyl
$R_D^3$ ist Wasserstoff, $(C_1-C_8)$Alkyl, $(C_2-C_4)$Alkenyl, $(C_2-C_4)$Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze
$n_D$ ist eine ganze Zahl von 0 bis 2.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS- Regno: 219479-18-2), 1,2-Dihydro-4-hydroxy-1-methyl-3-(5-tetrazolyl- carbonyl)-2-chinolon (CAS-Reg.Nr. 95855-00-8), wie sie in der WO-A-1999/000020 beschrieben sind.

S10) Verbindungen der Formeln ($S10^a$) oder ($S10^b$)
wie sie in der WO-A-2007/023719 und WO-A-2007/023764 beschrieben sind

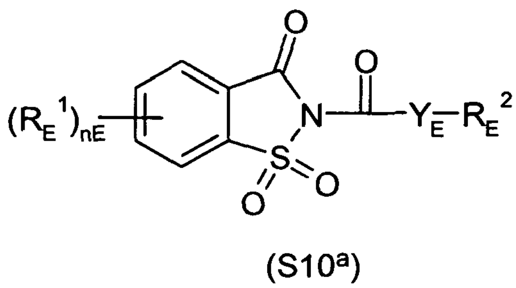

($S10^a$)

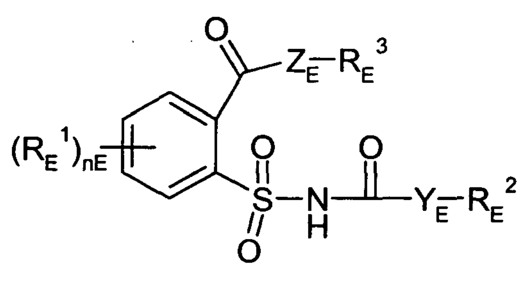

($S10^b$)

worin

$R_E^1$ Halogen, $(C_1-C_4)$Alkyl, Methoxy, Nitro, Cyano, $CF_3$, $OCF_3$
$Y_E$, $Z_E$ unabhängig voneinander O oder S,
$n_E$ eine ganze Zahl von 0 bis 4,
$R_E^2$ $(C_1-C_{16})$Alkyl, $(C_2-C_6)$Alkenyl, $(C_3-C_6)$Cycloalkyl, Aryl; Benzyl, Halogenbenzyl,

$R_E^3$ Wasserstoff oder ($C_1$-$C_6$)Alkyl bedeuten.

S11) Wirkstoffe vom Typ der Oxyimino-Verbindungen (S11), die als Saatbeizmittel bekannt sind, wie z. B. "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist, "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.

S12) Wirkstoffe aus der Klasse der Isothiochromanone (S12), wie z.B. Methyl-[(3-oxo-1 H-2-benzothiopyran-4 (3H)-yliden)methoxy]acetate (CAS-Reg.Nr. 205121-04-6) (S12-1) und verwandte Verbindungen aus WO-A-1998/13361.

S13) Eine oder mehrere Verbindungen aus Gruppe (S13):

"Naphthalic anhydrid" (1,8-Naphthalindicarbonsäureanhydrid) (S13-1), das als Saatbeiz-Safener für Mais gegen Schäden von Thiocarbamatherbiziden bekannt ist,
"Fenclorim" (4,6-Dichlor-2-phenylpyrimidin) (S13-2), das als Safener für Pretilachlor in gesätem Reis bekannt ist,
"Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
"CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
"MG 191" (CAS-Reg.Nr. 96420-72-3) (2-Dichlormethyl-2-methyl-1,3-dioxolan) (S13-5) der Firma Nitrokemia, das als Safener für Mais bekannt ist, "MG-838" (CAS-Reg.Nr. 133993-74-5) (2-propenyl 1-oxa-4-azaspiro[4.5]decane-4-carbodithioate) (S13-6) der Firma Nitrokemia
"Disulfoton" (O,O-Diethyl S-2-ethylthioethyl phosphordithioat) (S13-7),
"Dietholate" (O,O-Diethyl-O-phenylphosphorotioat) (S13-8),
"Mephenate" (4-Chlorphenyl-methylcarbamat) (S13-9).

S14) Wirkstoffe, die neben einer herbiziden Wirkung gegen Schadpflanzen auch Safenerwirkung an Kulturpflanzen wie Reis aufweisen, wie z. B.
"Dimepiperate" oder "MY-93" (S-1-Methyl-1-phenylethyl-piperidin-1-carbothioat), das als Safener für Reis gegen Schäden des Herbizids Molinate bekannt ist,
"Daimuron" oder "SK 23" (1-(1-Methyl-1-phenylethyl)-3-p-tolyl-harnstoff), das als Safener für Reis gegen Schäden des Herbizids Imazosulfuron bekannt ist,
"Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist,
"Methoxyphenon" oder "NK 049" (3,3'-Dimethyl-4-methoxy-benzophenon), das als Safener für Reis gegen Schäden einiger Herbizide bekannt ist,
"CSB" (1-Brom-4-(chlormethylsulfonyl)benzol) von Kumiai, (CAS-Reg.Nr. 54091-06-4), das als Safener gegen Schäden einiger Herbizide in Reis bekannt ist.

S15) Wirkstoffe, die vorrangig als Herbizide eingesetzt werden, jedoch auch Safenerwirkung auf Kulturpflanzen aufweisen, z.B.
(2,4-Dichlorphenoxy)essigsäure (2,4-D),
(4-Chlorphenoxy)essigsäure,
(R,S)-2-(4-Chlor-o-tolyloxy)propionsäure (Mecoprop),
4-(2,4-Dichlorphenoxy)buttersäure (2,4-DB),
(4-Chlor-o-tolyloxy)essigsäure (MCPA),
4-(4-Chlor-o-tolyloxy)buttersäure,
4-(4-Chlorphenoxy)buttersäure,
3,6-Dichlor-2-methoxybenzoesäure (Dicamba),
1-(Ethoxycarbonyl)ethyl-3,6-dichlor-2-methoxybenzoat (Lactidichlor-ethyl).

Das Mischungsverhältnis von Herbizid(mischung) zu Safener hängt im allgemeinen von der Aufwandmenge an Herbizid und der Wirksamkeit des jeweiligen Safeners ab und kann innerhalb weiter Grenzen variieren.

So liegt das Verhältnis bespielsweise im Bereich von 200:1 bis 1:200, vorzugsweise im Bereich von 100:1 bis 1:100, insbesondere im Bereich von 20:1 bis 1:20. Die Safener können analog zu den Verbindungen der allgemeinen Formel I oder deren Mischungen mit weiteren Herbiziden bzw. Pestiziden formuliert werden und als Fertigformulierung oder Tankmischung mit den Herbiziden bereitgestellt und angewendet werden.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- und Streugranulate sowie versprühbare Lösungen werden vor der Anwendung üblicherweise nicht mehr mit weiteren, inerten Stoffen verdünnt.

Mit den äußeren Bedingungen wie Temperatur, Feutigkeit, der Art des verwendeten Herbizids u. a. variiert die erforderliche Aufwandmenge der Verbindungen der Formel I. Sie kann deshalb innerhalb weiter Grenzen schwanken, z. B. zwischen 0,001 und 10,000 kg/ha oder mehr Aktivsubstanz, vorzugsweise liegt sie jedoch zwischen 0,005 und 5,000 kg/ha.

A. Synthesebeispiele

In den folgenden Synthesebeispielen beziehen sich die Mengenangaben (auch Prozentangaben) auf das Gewicht, sofern nichts anderes speziell angegeben ist.

Beispiel 1: 6-Chlor-N-[(2-fluor-4-methoxyphenyl)(isoxazol-3-yl)methyl]nicotinamid

0,190 g (1,08 mmol) 6-Chlornicotinsäurechlorid werden in 2 ml Dichlormethan gelöst und bei Raumtemperatur zu einer Lösung von 0,200 g (0,90 mmol) 1-(2-Fluor-4-methoxyphenyl)-1-(isoxazol-3-yl)methanamin und 0,219 g (2,16 mmol) Triethylamin in 3 ml Dichlormethan getropft. Die Mischung wird über Nacht bei Raumtemperatur gerührt und nacheinander mit gesättigter Natriumhydrogencarbonatlösung, 1 N Salzsäure und Wasser gewaschen. Die organische Phase wird über $MgSO_4$ getrocknet und eingengt. Das Rohprodukt wird durch präparative HPLC an Purospher RP18-e (Merck) gereinigt. Es werden 0,110 g (33,8 % der Theorie) eines weißen Feststoffs vom Schmelzpunkt 126 ˚C erhalten.

1 H-NMR ($CDCl_3$, 300 MHz) δ 8,83 (d, 1 H) 8,39 (d, 1 H) 8,10 (dd, 1 H) 7,42 (d, 2H) 7,25 (t, 1 H) 6,70 (dd, 1 H) 6,65 (d, 1 H) 6,59 (d, 1 H) 6,22 (d, 1 H) 3,80 (s, 3H)

MS (Chemische lonisation = CI) 362 $(M+H)^+$

Beispiel 2: 2-Chlor-N-[(2-fluor-4-methoxyphenyl)(isoxazol-3-yl)methyl]-6-methyl-4-n-propoxynicotinamid

0,184 g (0,80 mmol) 2-Chlor-6-methyl-4-propoxynicotinsäure (hergestellt gemäß WO2005/070889) werden in 5 ml Dichlormethan gelöst, mit 0,203 g (1,60 mmol) Oxalylchlorid versetzt und nach Zugabe von 3 Tropfen N,N-Dimethylformamid 2 Stunden am Rückfluß gekocht. Das überschüssige Oxalylchlorid wird im Vakuum abgezogen, der Rückstand mit 5 ml Dichlormethan versetzt und erneut im Vakuum eingeengt.

Das so erhaltene Öl wird in 2 ml Dichlormethan gelöst und bei Raumtemperatur zu einer Lösung von 0,179 g (0,81 mmol) 1-(2-Fluor-4-methoxyphenyl)-1-(isoxazol-3-yl)methanamin, 0,245 g (2,42 mmol) Triethylamin und einer Spatelspitze Polystyrol-DMAP in 3 ml Dichlormethan getropft. Die Mischung wird über Nacht bei Raumtemperatur gerührt, filtriert und nacheinander mit gesättigter Natriumhydrogencarbonatlösung, 1 N Salzsäure und Wasser gewaschen. Die organische Phase wird über $MgSO_4$ getrocknet und eingengt. Das Rohprodukt wird durch präparative HPLC an Purospher RP18-e (Merck) gereinigt. Es werden 0,06 g (16,5 % der Theorie) eines wachsartigen Feststoffs erhalten.

1 H-NMR ($CDCl_3$, 600 MHz) δ 8,35 (d, 1 H) 7,37 (t, 1 H) 7,01 (d, 1 H) 6,69 (dd, 1 H) 6,63 (dd, 1 H) 6,61 (s, 1 H) 6,60 (d, 1 H) 6.38 (d, 1 H) 3,97 (m, 2H) 3,80 (s, 3H) 2,50 (s, 3H) 1,72 (m, 2H) 0,88 (t, 3H)

MS (CI) 434 $(M+H)^+$

Beispiel 3: 2-Brom-N-[(2-fluor-4-methoxyphenyl)(isoxazol-3-yl)methyl]-4-isopropoxy-6-methylnicotinamid

Stufe A:

Zu einer Lösung von 0,621 g (2,795 mmol) 1-(2-Fluor-4-methoxyphenyl)-1-(isoxazol-3-yl)methanamin und 0,311 g (3,074 mmol) Triethylamin in 5 ml Dichlormethan wird bei 0˚C eine Lösung von 1,000 g (2,795 mmol) 2,4-Dibrom-6-methylnicotinoylbromid (hergestellt gemäß WO2005/070889) in 5 ml Dichlormethan getropft. Die Mischung wird über Nacht bei Raumtemperatur gerührt, filtriert und nacheinander mit gesättigter Natriumhydrogencarbonatlösung, 1 N Salzsäure und Wasser gewaschen. Die organische Phase wird über $MgSO_4$ getrocknet und eingeengt. Das Rohprodukt wird durch präparative HPLC an Purospher RP18-e (Merck) gereinigt. Es werden 1,38 g (98,9 % der Theorie) eines wachsartigen Feststoffs erhalten.

1 H-NMR ($CDCl_3$, 400 MHz) δ 8,38 (d, 1 H) 7,38 (m, 2H) 7,01 (d, 1 H) 6,70 (dd, 1 H) 6,65 (dd, 1 H) 6,60 (d, 1

H) 6,35 (d, 1 H) 3,80 (s, 3H) 2,53 (s, 3H)

MS (CI) 498 $(M+H)^+$

Stufe B:

Eine Lösung von 0,600 g (1,202 mmol) 2,4-Dibrom-N-[(2-fluor-4-methoxyphenyl)(isoxazol-3-yl)methyl]-6-methylnicotinamid (Stufe A, Beispiel 29) in 2 ml trockenem N,N-Dimethylformamid wird nach einander mit 0.043 g (1,443 mmol) 80 %-igem Natriumhydrid und 0,108 g (1,803 mmol) 2-Propanol versetzt und über Nacht bei 80˚ C gerührt. Nach Abkühlen auf Raumtemperatur wird die Mischung auf Wasser gegossen und dreimal mit Ethylacetat extrahiert. Die organische Phase wird über $MgSO_4$ getrocknet und eingengt. Das Rohprodukt wird durch präparative HPLC an Purospher RP18-e (Merck) gereinigt. Es werden 0,043 g (7,5 % der Theorie) eines Feststoffs erhalten.

1 H-NMR ($CDCl_3$, 400 MHz) δ 8,36 (d, 1 H) 7,38 (t, 1 H) 6,89 (d, 1 H) 6,70 (dd, 1 H) 6,65 (dd, 1 H) 6,64 (s, 1 H) 6,60 (d, 1 H) 6,40 (d, 1 H) 4,60 (m, 1 H) 3,80 (s, 3H) 2,50 (s, 3H) 1,28 (d, 3H)

MS (CI) 478 $(M+H)^+$

Beispiel 4: 4-Brom-N-[(2-fluor-4-methoxyphenyl)(isoxazol-3-yl)methyl]-2-isopropoxy-6-methylnicotinamid

Bei der präparativen Chromatographie von Stufe B aus Beispiel 8 werden als zweite Fraktion 13 mg (2,3% der Theorie) eines Isomeren von Beispiel 8 erhalten.

1 H-NMR ($CDCl_3$, 400 MHz) δ 8,36 (d, 1H) 7,40 (t, 1H) 7,11 (d, 1H) 6,95 (s, 1H) 6,70 (dd, 1H) 6,65 (dd, 1H) 6,60 (d, 1H) 6,40 (d, 1H) 5,35 (m, 1H) 3,80 (s, 3H) 2,40 (s, 3H) 1,25 (t, 3H)

MS (CI) 478 $(M+H)^+$

Beispiel 5: 2-Chlor-N-[(4-chlorphenyl)(isoxazol-3-yl)methyl]-6-methyl-4-(propylthio)nicotinamid

Stufe A:

Eine Lösung von 0,200 g (0,971 mmol) 2,4-Dichlor-6-methylnicotinsäure in 1 ml trockenem N,N-Dimethylformamid wird nacheinander mit 0.087 g (2,912 mmol) 80 %-igem Natriumhydrid und 0,084 g (1,107 mmol) 2-Propanthiol versetzt und 30 Minuten bei Raumtemperatur gerührt. Die Mischung wird auf Wasser gegossen, angesäuert und dreimal mit Ethylacetat extrahiert. Die organische Phase wird über $MgSO_4$ getrocknet und eingengt. Es werden 0,200 g (81,33 % der Theorie) eines Feststoffs erhalten.

Stufe B:

0,200g (0,814 mmol) 2-Chlor-6-methyl-4-(n-propylthio)nicotinsäure (Stufe A) werden in 5 ml Dichlormethan gelöst, mit 0,207 g (1,628 mmol) Oxalylchlorid versetzt und nach Zugabe von 3 Tropfen N,N-Dimethylformamid 2 Stunden am Rückfluß gekocht. Das überschüssige Oxalylchlorid wird im Vakuum abgezogen, der Rückstand mit 5 ml Dichlormethan versetzt und erneut im Vakuum eingeengt.

Das so erhaltene Öl wird in 2 ml Dichlormethan gelöst und bei Raumtemperatur zu einer Lösung von 0,158 g (0,757 mmol) (4-Chlorphenyl)(isoxazol-3-yl)methanamin und 0,230 g (2,271 mmol) Triethylamin in 3 ml Dichlormethan getropft. Die Mischung wird über Nacht bei Raumtemperatur gerührt, filtriert und nacheinander mit gesättigter Natriumhydrogencarbonatlösung, 1 N Salzsäure und Wasser gewaschen. Die organische Phase wird über $MgSO_4$ getrocknet und eingengt. Das Rohprodukt wird durch präparative HPLC an Purospher RP18-e (Merck) gereinigt. Es werden 0,06 g (16,5 % der Theorie) eines wachsartigen Feststoffs erhalten.

1 H-NMR ($CDCl_3$, 400 MHz) δ 8,40 (d, 1 H) 7,36 (m, 4H) 6,99 (d, 1 H) 6,90 (s, 1 H) 6,46 (d, 1 H) 6,30 (d, 1 H) 2,90 (t, 2H) 2,50 (s, 3H) 1,70 (m, 2H) 1,02 (t, 3H)

MS (CI) 436 $(M+H)^+$

Beispiel 6: N-[(2-Fluor-4-methoxyphenyl)(isoxazol-3-yl)methyl]-4-methylpyridin-2-carboxamid

0,200 g (1,458 mmol) 4-Methylpyridin-2-carbonsäure werden in 5 ml 1,2-Dichlorethan gelöst und nacheinander mit 0,284 g (1,75 mmol) Carbonyldiimidazol und 0,324 g (1,458 mmol) 1-(2-Fluor-4-methoxyphenyl)-1-(isoxazol-3-yl) methanamin versetzt. Die Mischung wird 4 Stunden bei 40 ˚C gerührt und nach dem Abkühlen auf Raumtemperatur zweimal mit Wasser gewaschen. Die organische Phase wird über $MgSO_4$ getrocknet und eingengt. Das Rohprodukt wird durch präparative HPLC an Purospher RP18-e (Merck) gereinigt. Es werden 0,100 g (19,9 % der Theorie) eines wachsartigen Feststoffs erhalten.

1 H-NMR ($CDCl_3$, 400 MHz) δ 9,10 (d, 1H) 8,44 (d, 1H) 8,35 (d, 1H) 8,01 (s, 1H) 7,34 (t, 1H) 7,25 ( d, 1H) 6,66

(m, 2H) 6,65 (d, 1H) 6,31 (d, 1H) 3,78 (s, 3H) 2,41 (s, 3H)
MS(CI) 342 $(M+H)^+$

Beispiel 7: 5-Chlor-N-[(4-chlorphenyl)(isoxazol-3-yl)methyl]-1,3-dimethyl-1 H-pyrazol-4-carboxamid

0,225 g (1,166 mmol) 5-Chlor-1,3-dimethylpyrazol-4-carbonsäurechlorid werden in 2 ml Dichlormethan gelöst und bei Raumtemperatur zu einer Lösung von 0,221 g (1,060 mmol) (4-Chlorphenyl)(isoxazol-3-yl)methanamin und 0,236 g (2,331 mmol) Triethylamin in 3 ml Dichlormethan getropft. Die Mischung wird über Nacht bei Raumtemperatur gerührt und nacheinander mit gesättigter Natriumhydrogencarbonatlösung, 1 N Salzsäure und Wasser gewaschen. Die organische Phase wird über $MgSO_4$ getrocknet und eingengt. Das Rohprodukt wird durch präparative HPLC an Purospher RP18-e (Merck) gereinigt. Es werden 0,110 g (27,3 % der Theorie) eines weißen Feststoffs vom Schmelzpunkt 135 ˚C erhalten.
1 H-NMR ($CDCl_3$, 400 MHz) δ 8,38 (d, 1H) 7,50 (d, 1H) 7,35 (m, 4H) 6,39 (d, 1H) 6,19 (d, 1H) 3,80 (s, 3H) 2,45 (s, 3H)
MS (CI) 365 $(M+H)^+$

Beispiel 8: 1-Benzyl-5-chlor-N-[(2-fluor-4-methoxyphenyl)(isoxazol-3-yl)methyl]-1 H-imidazol-4-carboxamid

0,450 g (1,772 mmol) 1-Benzyl-5-chlorimidazol-4-carbonsäure werden in 5 ml Dichlormethan gelöst, mit 0,462 g (3,641 mmol) Oxalylchlorid versetzt und nach Zugabe von 3 Tropfen N,N-Dimethylformamid 2 Stunden am Rückfluß gekocht. Das überschüssige Oxalylchlorid wird im Vakuum abgezogen, der Rückstand mit 5 ml Dichlormethan versetzt und erneut im Vakuum eingeengt.
0,225 g (0,886 mmol) des so erhaltenen 1-Benzyl-5-chlorimidazol-4-carbonsäurechlorids werden in 2 ml Dichlormethan gelöst und bei Raumtemperatur zu einer Lösung von 0,171 g (0,770 mmol) 1-(2-Fluor-4-methoxyphenyl)-1-(isoxazol-3-yl)methanamin und 0,171 g (1,694 mmol) Triethylamin in 3 ml Dichlormethan getropft. Die Mischung wird über Nacht bei Raumtemperatur gerührt und nacheinander mit gesättigter Natriumhydrogencarbonatlösung, 1 N Salzsäure und Wasser gewaschen. Die organische Phase wird über $MgSO_4$ getrocknet und eingengt. Das Rohprodukt wird durch präparative HPLC an Purospher RP18-e (Merck) gereinigt. Es werden 0,180 g (51,3 % der Theorie) 1-Benzyl-5-chlor-N-[(2-fluor-4-methoxyphenyl)(isoxazol-3-yl)methyl]-1H-imidazol-4-carboxamid erhalten.
1 H-NMR ($CDCl_3$, 400 MHz) δ 8,33 (d, 1 H) 8,13 (d, 1 H) 7,45 (s, 1 H) 7,35 (m, 4H) 7,25 (dd, 2H) 6,65 (m, 3H) 6,30 (d, 1 H) 5,14 (s, 2H) 3,78 (s, 3H)
MS (CI) 341 $(M+H)^+$

Beispiel 9: 2-Brom-N-[(2-fluor-4-methoxyphenyl)(isoxazol-3-yl)methyl]-4,5-dimethylthiophen-3-carboxamid

0,450 g (1,914 mmol) 2-Brom-4,5-dimethylthiophen-3-carbonsäure werden in 5 ml Dichlormethan gelöst, mit 0,486 g (3,828 mmol) Oxalylchlorid versetzt und nach Zugabe von 3 Tropfen N,N-Dimethylformamid 2 Stunden am Rückfluß gekocht. Das überschüssige Oxalylchlorid wird im Vakuum abgezogen, der Rückstand mit 5 ml Dichlormethan versetzt und erneut im Vakuum eingeengt. 0,225 g (0,887 mmol) des so erhaltenen 2-Brom-4,5-dimethylthiophen-3-carbonsäurechlorids werden in 2 ml Dichlormethan gelöst und bei Raumtemperatur zu einer Lösung von 0,179 g (0,807 mmol) 1-(2-Fluor-4-methoxyphenyl)-1-(isoxazol-3-yl)methanamin und 0,180 g (1,775 mmol) Triethylamin in 3 ml Dichlormethan getropft. Die Mischung wird über Nacht bei Raumtemperatur gerührt und nacheinander mit gesättigter Natriumhydrogencarbonatlösung, 1 N Salzsäure und Wasser gewaschen. Die organische Phase wird über $MgSO_4$ getrocknet und eingengt. Das Rohprodukt wird durch präparative HPLC an Purospher RP18-e (Merck) gereinigt. Es werden 0,100 g (26,8 % der Theorie) eines weißen Feststoffs vom Schmelzpunkt 143 ˚C erhalten.
1 H-NMR ($CDCl_3$, 400 MHz) δ 8,36 (d, 1H) 7,35 (t, 1H) 6,70 (dd, 1H) 6,67 (dd, 1H) 6,40 (d, 1H) 6,32 (d, 1H) 3,80 (s, 3H) 2,27 (s, 3H) 2,15 (s, 3H)
MS (CI) 339 $(M+H)^+$

Beispiel 10: 4,5-Dichlor-N-[(2-fluor-4-methoxyphenyl)(isoxazol-3-yl)methyl]isothiazol-3-carboxamid

0,200 g (1,010 mmol) 4.5-Dichlorisothiazol-3-carbonsäure werden in 5 ml 1,2-Dichlorethan gelöst, mit 0,197 g (1,212 mmol) Carbonyldiimidazol versetzt und zwei Stunden bei 40˚ C gerührt. 0,224 g (1,010mmol) 1-(2-Fluor-4-methoxyphenyl)-1-(isoxazol-3-yl)methanamin werden zugegeben. Die Mischung wird 4 Stunden bei 40 ˚C gerührt und nach dem Abkühlen auf Raumtemperatur zweimal mit Wasser gewaschen. Die organische Phase wird über $MgSO_4$ getrocknet und eingengt. Das Rohprodukt wird durch präparative HPLC an Purospher RP18-e (Merck) gereinigt. Es werden 0,080 g (19,3 % der Theorie) eines wachsartigen Feststoffs erhalten.
1 H-NMR ($CDCl_3$, 400 MHz) δ 8,36 (d, 1 H) 8,28 (d, 1 H) 7,31 (t, 1 H) 6,70 (dd, 1 H) 6,65 (dd, 1 H) 6,56 (d, 1

H) 6,25 (d, 1 H) 3,80 (s, 3H)

MS(CI) 402 $(M+H)^+$

Beispiel 11: N-[(2-Fluor-4-methoxyphenyl)(isoxazol-3-yl)methyl]chinolin-3-carboxamid

0,500 g (2,887 mmol) Chinolin-3-carbonsäure werden in 5 ml Dichlormethan gelöst, mit 0,733 g (5,775 mmol) Oxalylchlorid versetzt und nach Zugabe von 3 Tropfen N,N-Dimethylformamid 2 Stunden am Rückfluß gekocht. Das überschüssige Oxalylchlorid wird im Vakuum abgezogen, der Rückstand mit 5 ml Dichlormethan versetzt und erneut im Vakuum eingeengt. 0,250 g (1,305 mmol) des so erhaltenen Chinolin-3-carbonsäurechlorids werden in 2 ml Dichlormethan gelöst und bei Raumtemperatur mit einer Lösung von 0,264 g (1,186 mmol) 1-(2-Fluor-4-methoxyphenyl)-1-(isoxazol-3-yl)methanamin und 0,360 g (3,558 mmol) Triethylamin in 3 ml Dichlormethan versetzt. Die Mischung wird über Nacht bei Raumtemperatur gerührt und nacheinander mit gesättigter Natriumhydrogencarbonatlösung, 1 N Salzsäure und Wasser gewaschen. Die organische Phase wird über $MgSO_4$ getrocknet und eingengt. Das Rohprodukt wird durch präparative HPLC an Purospher RP18-e (Merck) gereinigt. Es werden 0,120 g (24,9 % der Theorie) eines wachsartigen Feststoffs erhalten.

1 H-NMR ($CDCl_3$, 400 MHz) δ 9,34 (d, 1H) 8,62 (d, 1H) 8,40 (d, 1H) 8,15 (d, 1H) 7,91 (d, 1 H) 7,81 (dd, 1 H) 7,61 (t, br, 2H) 7,35 (t, 1 H) 6,69 (m, 3H) 6,28 (d, 1 H) 3,80 (s, 3H)

Beispiel 12: N-[(2-Fluor-4-methoxyphenyl)(isoxazol-3-yl)methyl]-2-ethyl-3-methylchinolin-4-carboxamid

0,200 g (0,929 mmol) 2-Ethyl-3-methyl-chinolin-4-carbonsäure werden in 5 ml (8,155 g, 68,547 mmol) Thionylchlorid suspendiert und 2 Stunden bei Raumtemperatur gerührt. Das überschüssige Thionylchlorid wird im Vakuum abgezogen, der Rückstand mit 5 ml Dichlormethan versetzt und erneut im Vakuum eingeengt. 0,200 g (0,856 mmol) des so erhaltenen 2-Ethyl-3-methyl-chinolin-4-carbonsäurechlorids werden in 2 ml Dichlormethan gelöst und bei Raumtemperatur mit einer Lösung von 0,190 g (0,856 mmol) 1-(2-Fluor-4-methoxyphenyl)-1-(isoxazol-3-yl)methanamin und 0,260 g (2,567 mmol) Triethylamin in 3 ml Dichlormethan versetzt. Die Mischung wird über Nacht bei Raumtemperatur gerührt und nacheinander mit gesättigter Natriumhydrogencarbonatlösung, 1 N Salzsäure und Wasser gewaschen. Die organische Phase wird über $MgSO_4$ getrocknet und eingengt. Das Rohprodukt wird durch präparative HPLC an Purospher RP18-e (Merck) gereinigt. Es werden 0,070 g (19,5 % der Theorie) eines wachsartigen Feststoffs erhalten.

1 H-NMR ($CDCl_3$, 400 MHz) δ 8,41 (d, 1 H) 8,00 (d, 1 H) 7,70 (s, br, 1 H) 7,62 (t, 1 H) 7,45 (t, 1 H) 7,30 (t, 1 H) 7,08 (d, 1 H) 6,85 (d, 1 H) 6,71 (m, 2H) 6,29 (d, 1 H) 3,81 (s, 3H) 2,96 (q, 2H) 2,41 (s, 3H) 1,35 (t, 3H)

MS(CI) 420 $(M+H)^+$

Beispiel 13: 5-Brom-N-[(5-brom-2-chlorpyridin-3-yl)carbonyl]-2-chlor-N-[(4-chlorphenyl)(isoxazol-3-yl)methyl]nicotinamid

0,500 g (2,115 mmol) 2-Chlor-5-bromnicotinsäure werden in 5 ml Dichlormethan gelöst, mit 0,537 g (4,229 mmol) Oxalylchlorid versetzt und nach Zugabe von 3 Tropfen N,N-Dimethylformamid 2 Stunden am Rückfluß gekocht. Das überschüssige Oxalylchlorid wird im Vakuum abgezogen, der Rückstand mit 5 ml Dichlormethan versetzt und erneut im Vakuum eingeengt. 0,250 g (0,981 mmol) des so erhaltenen 2-Chlor-5-bromnicotinsäurechlorids werden in 2 ml Dichlormethan gelöst und bei Raumtemperatur mit einer Lösung von 0,186 g (0,892 mmol) (4-Chlorphenyl)(isoxazol-3-yl)methanamin und 0,271 g (2,675 mmol) Triethylamin in 3 ml Dichlormethan versetzt. Die Mischung wird über Nacht bei Raumtemperatur gerührt und nacheinander mit gesättigter Natriumhydrogencarbonatlösung, 1 N Salzsäure und Wasser gewaschen. Die organische Phase wird über $MgSO_4$ getrocknet und eingengt. Das Rohprodukt wird durch präparative HPLC an Purospher RP18-e (Merck) gereinigt. Es werden 0,050 g (8,6 % der Theorie) eines wachsartigen Feststoffs erhalten.

1 H-NMR ($CDCl_3$, 400 MHz) δ 8,48 (d, 1 H) 8,32 (d, 2H) 7,90 (d, 2H) 7,69 (d, 2H) 7,42 (d, 2H) 7,20 (s, 1 H) 6,33 (d, 1 H)

MS(CI) 644 $(M+H)^+$

In den nachfolgenden Tabellen werden folgende Abkürzungen verwendet:

| | |
|---|---|
| Et | bedeutet Ethyl, |
| nPr | bedeutet 1-Propyl, |
| iPr | bedeutet 2-Propyl, |
| cycloPr | bedeutet Cyclopropyl, |
| iBu | bedeutet 2-Methyl-1-propyl, |

Positionen, die nicht mit den in den Tabellen genannten Substituenten besetzt sind, sind durch Wasserstoff

substituiert.

Tabelle 1: Verbindungen der Formel (Ia)

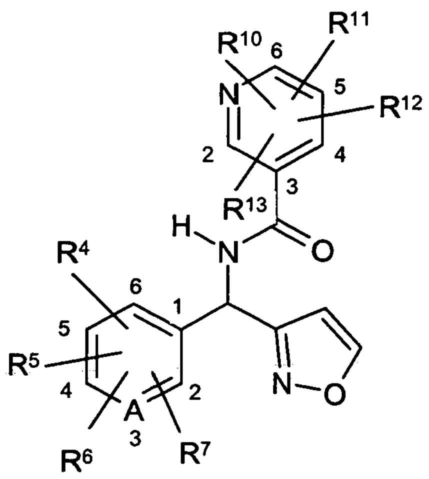

(Ia)

Gemäß den Beispielen 1-5 können die in Tabelle 1 zusammengefassten Verbindungen 14-224 der Formel (Ia) hergestellt werden.

| Beispiel Nr. | $R^{10}$ | $R^{11}$ | $R^{12}$ | A | $R^4$ | $R^5$ | $R^6$ | Fp(°C) | log P |
|---|---|---|---|---|---|---|---|---|---|
| 14 | 6-Cl | H | H | CH | 4-Cl | H | H | 151 | |
| 15 | 2-Cl | 6-Cl | H | CH | 4-Cl | H | H | 160 | |
| 16 | 2-Cl | 6-Cl | H | CH | 2-F | 4-$OCH_3$ | H | 136 | |
| 17 | 5-Cl | 6-Cl | H | CH | 4-Cl | H | H | 193 | |
| 18 | 5-Cl | 6-Cl | H | CH | 2-F | 4-$OCH_3$ | H | 149 | |
| 19 | 5-$CH_3$ | 6-Cl | H | CH | 4-Cl | H | H | 180 | |
| 20 | 5-$CH_3$ | 6-Cl | H | CH | 2-F | 4-$OCH_3$ | H | | |
| 21 | 4-$CH_3$ | 6-$CH_3$ | H | CH | 4-Cl | H | H | | |
| 22 | 4-$CH_3$ | 6-$CH_3$ | H | CH | 2-F | 4-$OCH_3$ | H | | |
| 23 | 6-F | H | H | CH | 4-Cl | H | H | 152 | |
| 24 | 6-F | H | H | CH | 2-F | 4-$OCH_3$ | H | | |
| 25 | 4-Cl | 6-Cl | H | CH | 4-Cl | H | H | 160 | |
| 26 | 4-Cl | 6-Cl | H | CH | 2-F | 4-$OCH_3$ | H | 149 | |
| 27 | 6-Br | H | H | CH | 2-F | 4-$OCH_3$ | H | | |
| 28 | 6-Br | H | H | CH | 4-Cl | H | H | | |
| 29 | 2-Br | 4-Br | 6-$CH_3$ | CH | 2-F | 4-$OCH_3$ | H | | |
| 30 | 6-$OCH_3$ | H | H | CH | 4-Cl | H | H | | |
| 31 | 6-$OCH_3$ | H | H | CH | 2-F | 4-$OCH_3$ | H | | |
| 32 | 2-Br | 4-Br | 6-$CH_3$ | CH | 4-Cl | H | H | | |
| 33 | 2-OnPr | 4-Br | 6-$CH_3$ | CH | 2-F | 4-$OCH_3$ | H | | |
| 34 | 2-Br | 4-OnPr | 6-$CH_3$ | CH | 2-F | 4-$OCH_3$ | H | | |

(fortgesetzt)

| Gemäß den Beispielen 1-5 können die in Tabelle 1 zusammengefassten Verbindungen 14-224 der Formel (Ia) hergestellt werden. | | | | | | | | | |
|---|---|---|---|---|---|---|---|---|---|
| Beispiel Nr. | $R^{10}$ | $R^{11}$ | $R^{12}$ | A | $R^4$ | $R^5$ | $R^6$ | Fp(°C) | log P |
| 35 | 2-Br | 4-OnPr | 6-$CH_3$ | CH | 4-Cl | H | H | | 2.63 |
| 36 | 2-OnPr | 4-Br | 6-$CH_3$ | CH | 4-Cl | H | H | | |
| 37 | 2-Br | 5-nPr | H | CH | 4-Cl | H | H | 179 | |
| 38 | 2-Br | 5-nPr | H | CH | 2-F | 4-$OCH_3$ | H | | |
| 39 | 4-$CF_3$ | H | H | CH | 4-Cl | H | H | | |
| 40 | 4-$CF_3$ | H | H | CH | 2-F | 4-$OCH_3$ | H | | |
| 41 | 2-$OCH_3$ | H | H | CH | 4-Cl | H | H | | |
| 42 | 2-$OCH_3$ | H | H | CH | 2-F | 4-$OCH_3$ | H | | |
| 43 | 4-Cl | H | H | CH | 2-F | 4-$OCH_3$ | H | | |
| 44 | 5-Br | 6-Cl | H | CH | 4-Cl | H | H | | |
| 45 | 5-Br | 6-Cl | H | CH | 2-F | 4-$OCH_3$ | H | | |
| 46 | 5-Br | H | H | CH | 4-Cl | H | H | 172 | |
| 47 | 2-$SCH_3$ | H | H | CH | 4-Cl | H | H | | |
| 48 | 2-$SCH_3$ | H | H | CH | 2-F | 4-$OCH_3$ | H | 128 | |
| 49 | 2-Cl | 6-$CF_3$ | H | CH | 2-F | 4-$OCH_3$ | H | | |
| 50 | 2-Cl | 4-$CH_3$ | 6-Cl | CH | 4-Cl | H | H | | |
| 51 | 2-Cl | 4-$CH_3$ | 6-Cl | CH | 2-F | 4-$OCH_3$ | H | | |
| 52 | 2-Cl | 6-$CH_3$ | H | CH | 4-Cl | H | H | | |
| 53 | 2-Cl | 6-$CH_3$ | H | CH | 2-F | 4-$OCH_3$ | H | | |
| 54 | 2-Cl | H | H | CH | 4-Cl | H | H | 171 | |
| 55 | 2-Cl | H | H | CH | 2-F | 4-$OCH_3$ | H | | |
| 56 | 2-Cl | 4-$CH_3$ | 6-$CH_3$ | CH | 4-Cl | H | H | | |
| 57 | 2-$CH_3$ | H | H | CH | 4-Cl | H | H | | |
| 58 | 6-$CF_3$ | H | H | CH | 2-F | 4-$OCH_3$ | H | | |
| 59 | 5-$CH_3$ | 6-F | H | CH | 4-Cl | H | H | | |
| 60 | 5-$CH_3$ | 6-F | H | CH | 2-F | 4-$OCH_3$ | H | | |
| 61 | 2-Cl | 5-Cl | H | CH | 2-F | 4-$OCH_3$ | H | | |
| 62 | 4-Cl | H | H | CH | 4-Cl | H | H | | |
| 63 | 2-Cl | 6-$CF_3$ | H | CH | 4-Cl | H | H | | |
| 64 | 2-Cl | 4-$CH_3$ | 6-$CH_3$ | CH | 2-F | 4-$OCH_3$ | H | 116 | |
| 65 | 6-$CF_3$ | H | H | CH | 4-Cl | H | H | | |
| 66 | 2-Cl | 5-Cl | H | CH | 4-Cl | H | H | 217 | |
| 67 | 2-F | H | H | CH | 4-Cl | H | H | | |
| 68 | 2-F | H | H | CH | 2-F | 4-$OCH_3$ | H | | |

(fortgesetzt)

| Gemäß den Beispielen 1-5 können die in Tabelle 1 zusammengefassten Verbindungen 14-224 der Formel (Ia) hergestellt werden. | | | | | | | | | |
|---|---|---|---|---|---|---|---|---|---|
| Beispiel Nr. | $R^{10}$ | $R^{11}$ | $R^{12}$ | A | $R^4$ | $R^5$ | $R^6$ | Fp(°C) | log P |
| 69 | 2-Cl | 5-Br | H | CH | 4-Cl | H | H | | |
| 70 | 2-Cl | 5-Br | H | CH | 2-F | 4-$OCH_3$ | H | | |
| 71 | 2-Br | H | H | CH | 4-Cl | H | H | | |
| 72 | 2-Br | H | H | CH | 2-F | 4-$OCH_3$ | H | | |
| 73 | 2-Cl | 4-Et | H | CH | 2-F | 4-$OCH_3$ | H | | |
| 74 | 2-N $(CH_3)_2$ | H | H | CH | 4-Cl | H | H | | |
| 75 | 2-N $(CH_3)_2$ | H | H | CH | 2-F | 4-$OCH_3$ | H | | |
| 76 | 2-$OCH_3$ | 6-$OCH_3$ | H | CH | 2-F | 4-$OCH_3$ | H | | |
| 77 | 2-$CH_3$ | H | H | CH | 2-F | 4-$OCH_3$ | H | | |
| 78 | 4-$CF_3$ | 6-Cl | H | CH | 4-Cl | H | H | | |
| 79 | 4-$CF_3$ | 6-Cl | H | CH | 2-F | 4-$OCH_3$ | H | | |
| 80 | 2-Cl | 5-F | 6-Cl | CH | 4-Cl | H | H | | |
| 81 | 2-Cl | 5-F | 6-Cl | CH | 2-F | 4-$OCH_3$ | H | | |
| 82 | 2-$OCH_3$ | 6-$OCH_3$ | H | CH | 4-Cl | H | H | | |
| 83 | 2-Br | 5-Br | H | CH | 4-Cl | H | H | | |
| 84 | 2-Br | 5-Br | H | CH | 2-F | 4-$OCH_3$ | H | | |
| 85 | 4-$CH_3$ | H | H | CH | 4-Cl | H | H | | |
| 86 | 4-$CH_3$ | H | H | CH | 2-F | 4-$OCH_3$ | H | | |
| 87 | 5-$CH_3$ | H | H | CH | 4-Cl | H | H | | |
| 88 | 5-$CH_3$ | H | H | CH | 2-F | 4-$OCH_3$ | H | | |
| 89 | 5-Br | H | H | CH | 2-F | 4-$OCH_3$ | H | | |
| 90 | 5-Br | H | H | CH | 4-Cl | H | H | | |
| 91 | 2-$OCH_3$ | 5-Br | H | CH | 2-F | 4-$OCH_3$ | H | | |
| 92 | 6-CN | H | H | CH | 4-Cl | H | H | | |
| 93 | 6-CN | H | H | CH | 2-F | 4-$OCH_3$ | H | | |
| 94 | 4-OH | H | H | CH | 4-Cl | H | H | | |
| 95 | 2-$OCH_3$ | 5-F | H | CH | 4-Cl | H | H | | |
| 96 | 2-$OCH_3$ | 5-F | H | CH | 2-F | 4-$OCH_3$ | H | | |
| 97 | 2-$CH_3$ | 6-$CF_3$ | H | CH | 4-Cl | H | H | | |
| 98 | 2-$CH_3$ | 6-$CF_3$ | H | CH | 2-F | 4-$OCH_3$ | H | 141 | |

(fortgesetzt)

| Gemäß den Beispielen 1-5 können die in Tabelle 1 zusammengefassten Verbindungen 14-224 der Formel (Ia) hergestellt werden. | | | | | | | | | |
|---|---|---|---|---|---|---|---|---|---|
| Beispiel Nr. | $R^{10}$ | $R^{11}$ | $R^{12}$ | A | $R^4$ | $R^5$ | $R^6$ | Fp(°C) | log P |
| 99 | 2-SEt | H | H | CH | 4-Cl | H | H | | |
| 100 | 2-SEt | H | H | CH | 2-F | 4-$OCH_3$ | H | | |
| 101 | 2-SEt | H | H | CH | 3-$OCH_3$ | H | H | | |
| 102 | 2-$CH_3$ | 4-Cl | 6-$CH_3$ | CH | 4-Cl | H | H | | |
| 103 | 2-$CH_3$ | 4-Cl | 6-$CH_3$ | CH | 2-F | 4-$OCH_3$ | H | | |
| 104 | 2-$CH_3$ | 4-Cl | 6-$CH_3$ | CH | 3-$OCH_3$ | H | H | | |
| 105 | 6-$SCHF_2$ | H | H | CH | 4-Cl | H | H | | |
| 106 | 6-$SCHF_2$ | H | H | CH | 2-F | 4-$OCH_3$ | H | 144 | |
| 107 | 6-$SCHF_2$ | H | H | CH | 3-$OCH_3$ | H | H | | |
| 108 | 5-Cl | 6-$OCH_3$ | H | CH | 4-Cl | H | H | | |
| 109 | 5-Cl | 6-$OCH_3$ | H | CH | 2-F | 4-$OCH_3$ | H | | |
| 110 | 5-Cl | 6-$OCH_3$ | H | CH | 3-$OCH_3$ | H | H | | |
| 111 | 2-SnPr | H | H | CH | 3-$OCH_3$ | H | H | | |
| 112 | 2-Cl | 4-Et | H | CH | 3-$OCH_3$ | H | H | | |
| 113 | 2-N $(CH_3)_2$ | H | H | CH | 3-$OCH_3$ | H | H | | |
| 114 | 2-F | H | H | CH | 3-$OCH_3$ | H | H | | |
| 115 | 4-$CF_3$ | 6-Cl | H | CH | 3-$OCH_3$ | H | H | | |
| 116 | 6-$CF_3$ | H | H | CH | 3-$OCH_3$ | H | H | | |
| 117 | 2-Cl | 5-F | 6-Cl | CH | 3-$OCH_3$ | H | H | | |
| 118 | 2-Cl | 4-Cl | 6-$CH_3$ | CH | 3-$OCH_3$ | H | H | | |
| 119 | 2-$OCH_3$ | 5-F | H | CH | 3-$OCH_3$ | H | H | | |
| 120 | 2-$OCH_3$ | 5-Br | H | CH | 3-$OCH_3$ | H | H | | |
| 121 | 2-$SCH_3$ | H | H | CH | 3-$OCH_3$ | H | H | | |
| 122 | 2-$OCH_3$ | 6-$OCH_3$ | H | CH | 3-$OCH_3$ | H | H | | |
| 123 | 2-Cl | H | H | CH | 3-$OCH_3$ | H | H | | |
| 124 | 2-Cl | 5-Br | H | CH | 3-$OCH_3$ | H | H | | |
| 125 | 2-Br | 5-Br | H | CH | 3-$OCH_3$ | H | H | | |
| 126 | 6-CN | H | H | CH | 3-$OCH_3$ | H | H | | |
| 127 | 2-$OCH_3$ | H | H | CH | 3-$OCH_3$ | H | H | | |
| 128 | 2-Br | H | H | CH | 3-$OCH_3$ | H | H | | |

(fortgesetzt)

| Gemäß den Beispielen 1-5 können die in Tabelle 1 zusammengefassten Verbindungen 14-224 der Formel (Ia) hergestellt werden. | | | | | | | | | |
|---|---|---|---|---|---|---|---|---|---|
| Beispiel Nr. | $R^{10}$ | $R^{11}$ | $R^{12}$ | A | $R^4$ | $R^5$ | $R^6$ | Fp(°C) | log P |
| 129 | 2-$CH_3$ | 6-$CF_3$ | H | CH | 3-$OCH_3$ | H | H | | |
| 130 | 2-Cl | 4-$CH_3$ | 6-$CH_3$ | CH | 3-$OCH_3$ | H | H | | |
| 131 | 4-$CH_3$ | 5-Br | 6-$CH_3$ | CH | 4-Cl | H | H | | 2.63 |
| 132 | 4-$CH_3$ | 5-Br | 6-$CH_3$ | CH | 2-F | 4-$OCH_3$ | H | | 2.28 |
| 133 | 4-$CH_3$ | 5-Br | 6-$CH_3$ | CH | 3-$OCH_3$ | H | H | | |
| 134 | 5-Br | H | H | CH | 3-$OCH_3$ | H | H | | |
| 135 | 6-OiPr | H | H | CH | 2-F | 4-$OCH_3$ | H | | |
| 136 | 2-Cl | 5-nPr | H | CH | 3-$OCH_3$ | H | H | | |
| 137 | 2-Br | 5-nPr | H | CH | 3-$OCH_3$ | H | H | | |
| 138 | 6-OiPr | H | H | CH | 3-$OCH_3$ | H | H | | |
| 139 | 2-SnPr | H | H | CH | 4-Cl | H | H | 145 | |
| 140 | 2-SnPr | H | H | CH | 2-F | 4-$OCH_3$ | H | | |
| 141 | 5-$CH_3$ | H | H | CH | 3-$OCH_3$ | H | H | | |
| 142 | 2-Cl | 4-Et | H | CH | 4-Cl | H | H | | |
| 143 | 2-Cl | 4-$CH_3$ | H | CH | 4-Cl | H | H | | |
| 144 | 2-Cl | 5-nPr | H | CH | 4-Cl | H | H | 167 | |
| 145 | 2-Cl | 5-nPr | H | CH | 2-F | 4-$OCH_3$ | H | | |
| 146 | 2-OnPr | 4-Cl | 6-$CH_3$ | CH | 2-F | 4-$OCH_3$ | H | | |
| 147 | 5-$CH_3$ | 6-F | H | CH | 3-$OCH_3$ | H | H | | |
| 148 | 2-OnPr | 4-OnPr | 6-$CH_3$ | CH | 4-Cl | H | H | | |
| 149 | 2-Cl | 4-OnPr | 6-$CH_3$ | CH | 4-Cl | H | H | | |
| 150 | 2-Cl | 4-OnPr | 6-$CH_3$ | CH | 3-$OCH_3$ | H | H | | |
| 151 | 4-Cl | H | H | CH | 3-$OCH_3$ | H | H | | |
| 152 | 2-Cl | 4-OiPr | 6-$CH_3$ | CH | 4-Cl | H | H | | |
| 153 | 2-OiPr | 4-Cl | 6-$CH_3$ | CH | 2-F | 4-$OCH_3$ | H | | |
| 154 | 2-$CH_3$ | H | H | CH | 3-$OCH_3$ | H | H | | |
| 155 | 2-OiPr | 4-Cl | 6-$CH_3$ | CH | 4-Cl | H | H | | |
| 156 | 2-Cl | 4-SnPr | 6-$CH_3$ | CH | 2-F | 4-$OCH_3$ | H | | |
| 157 | 2-Br | 4-OiPr | 6-$CH_3$ | CH | 4-Cl | H | H | | |
| 158 | 2-Br | 4-OiPr | 6-$CH_3$ | CH | 3-$OCH_3$ | H | H | | 2.16 |
| 159 | 2-Br | 4-OnPr | 6-$CH_3$ | CH | 3-$OCH_3$ | H | H | | |
| 160 | 2-Br | 4-OnPr | 6-$CH_3$ | CH | 2-Cl | 4-Cl | H | | |
| 161 | 2-Br | 4-OnPr | 6-$CH_3$ | CH | 3-F | 4-F | H | | |
| 162 | 2-Br | 4-OnPr | 6-$CH_3$ | CH | 3-Cl | H | H | | |
| 163 | 2-OiPr | 4-Cl | 6-$CH_3$ | CH | 2-Cl | 4-Cl | H | | |

(fortgesetzt)

| Gemäß den Beispielen 1-5 können die in Tabelle 1 zusammengefassten Verbindungen 14-224 der Formel (Ia) hergestellt werden. | | | | | | | | | |
|---|---|---|---|---|---|---|---|---|---|
| Beispiel Nr. | $R^{10}$ | $R^{11}$ | $R^{12}$ | A | $R^4$ | $R^5$ | $R^6$ | Fp(°C) | log P |
| 164 | 2-OiPr | 4-Cl | 6-$CH_3$ | CH | 3-F | 4-F | H | | |
| 165 | 2-Cl | 4-SnPr | 6-$CH_3$ | CH | 3-$OCH_3$ | H | H | | |
| 166 | 2-Cl | 4-SnPr | 6-$CH_3$ | CH | 3-F | 4-F | H | | |
| 167 | 2-Cl | 4-SnPr | 6-$CH_3$ | CH | 2-Cl | 4-Cl | H | | |
| 168 | 2-Cl | 4-SnPr | 6-$CH_3$ | CH | 3-Cl | H | H | | |
| 169 | 2-Br | 4-OiPr | 6-$CH_3$ | CH | 2-Cl | 4-Cl | H | | |
| 170 | 2-Br | 4-OiPr | 6-$CH_3$ | CH | 3-F | 4-F | H | | |
| 171 | 2-Br | 4-OiPr | 6-$CH_3$ | CH | 3-Cl | H | H | | |
| 172 | 2-Cl | 4-OiPr | 6-$CH_3$ | CH | 3-$OCH_3$ | H | H | | |
| 173 | 2-Cl | 4-OiPr | 6-$CH_3$ | CH | 2-Cl | 4-Cl | H | | |
| 174 | 2-Cl | 4-OiPr | 6-$CH_3$ | CH | 3-F | 4-F | H | | 2.26 |
| 175 | 2-Cl | 4-OiPr | 6-$CH_3$ | CH | 3-Cl | H | H | | |
| 176 | 2-Cl | 4-SiPr | 6-$CH_3$ | CH | 4-Cl | H | H | | |
| 177 | 2-Cl | 4-SiPr | 6-$CH_3$ | CH | 2-F | 4-$OCH_3$ | H | | |
| 178 | 2-Cl | 4-SiPr | 6-$CH_3$ | CH | 3-$OCH_3$ | H | H | | |
| 179 | 2-Cl | 4-SiPr | 6-$CH_3$ | CH | 2-Cl | 4-Cl | H | | |
| 180 | 2-Cl | 4-SiPr | 6-$CH_3$ | CH | 3-F | 4-F | H | | |
| 181 | 2-Cl | 4-SiPr | 6-$CH_3$ | CH | 3-Cl | H | H | | |
| 182 | 2-OnPr | 4-OnPr | 6-$CH_3$ | CH | 2-F | 4-$OCH_3$ | H | | |
| 183 | 2-Cl | 4-OnPr | 6-$CH_3$ | CH | 2-Cl | 4-Cl | H | | |
| 184 | 2-Cl | 4-OnPr | 6-$CH_3$ | CH | 3-F | 4-F | H | | |
| 185 | 2-Cl | 4-OnPr | 6-$CH_3$ | CH | 3-Cl | H | H | | |
| 186 | 2-OnPr | 4-Cl | 6-$CH_3$ | CH | 4-Cl | H | H | | |
| 187 | 2-Cl | 4-OiPr | 6-$CH_3$ | CH | 2-F | 4-$OCH_3$ | H | | 2.18 |
| 188 | 4-$OCH_3$ | H | H | CH | 4-Cl | H | H | | 1.18 |
| 189 | 4-$OCH_3$ | H | H | CH | 2-F | 4-$OCH_3$ | H | | 1.00 |
| 190 | 4-$OCH_3$ | H | H | CH | 3-F | 4-F | H | | 1.07 |
| 191 | 6-Cl | H | H | CH | 3-$OCH_3$ | 4-$OCH_3$ | H | 169 | |
| 192 | 6-Cl | H | H | CH | 2-Cl | H | H | 52 | |
| 193 | 6-Cl | H | H | CH | 3-F | H | H | 141 | |
| 194 | 6-Cl | H | H | CH | 2-$CH_3$ | H | H | | |
| 195 | 6-Cl | H | H | CH | 3-$CH_3$ | H | H | 138 | |
| 196 | 6-Cl | H | H | CH | 3-F | 4-F | H | 190 | |

(fortgesetzt)

| Gemäß den Beispielen 1-5 können die in Tabelle 1 zusammengefassten Verbindungen 14-224 der Formel (Ia) hergestellt werden. | | | | | | | | | |
|---|---|---|---|---|---|---|---|---|---|
| Beispiel Nr. | $R^{10}$ | $R^{11}$ | $R^{12}$ | A | $R^4$ | $R^5$ | $R^6$ | Fp(˚C) | log P |
| 197 | 6-Cl | H | H | CH | 4-$CH_3$ | H | H | 154 | |
| 198 | 6-Cl | H | H | CH | 4-$SO_2CH_3$ | H | H | 174 | |
| 199 | 6-Cl | H | H | CH | 2-$CF_3$ | H | H | 140 | |
| 200 | 6-Cl | H | H | CH | 2-F | 4-$CF_3$ | H | 138 | |
| 201 | 6-Cl | H | H | CH | 2-Cl | 4-Cl | H | 171 | |
| 202 | 6-Cl | H | H | CH | 4-$OCF_3$ | H | H | 131 | |
| 203 | 6-Cl | H | H | CH | 2-Br | 4-F | H | 145 | |
| 204 | 6-Cl | H | H | CH | 3-CN | H | H | 173 | |
| 205 | 6-Cl | H | H | CH | 3-Cl | H | H | 155 | |
| 206 | 6-Cl | H | H | CH | 3-Br | H | H | 167 | |
| 207 | 6-Cl | H | H | CH | 2-F | H | H | 155 | |
| 208 | 6-Cl | H | H | CH | 2-Br | H | H | | |
| 209 | 6-$CH_3$ | H | H | CH | 4-Cl | H | H | | |
| 210 | 6-$CH_3$ | H | H | CH | 2-Br | 4-F | H | 138 | |
| 211 | 6-$CH_3$ | H | H | CH | 2-F | 4-$CF_3$ | H | | |
| 212 | 6-$CH_3$ | H | H | CH | 2-F | 4-$OCH_3$ | H | | |
| 213 | 6-$CH_3$ | H | H | CH | 2-Cl | 4-Cl | H | 168 | |
| 214 | 6-$CH_3$ | H | H | CH | 3-F | 4-F | H | | |
| 215 | 6-$CH_3$ | H | H | CH | 4-$CH_3$ | H | H | | |
| 216 | 2-Cl | 4-OiPr | 6-$CH_3$ | N | 4-Cl | H | H | | |
| 217 | 2-Cl | 4-OnPr | 6-$CH_3$ | N | 4-Cl | H | H | | |
| 218 | 2-Br | 4-OnPr | 6-$CH_3$ | N | 4-Cl | H | H | | |
| 219 | 2-Br | 4-OiPr | 6-$CH_3$ | N | 4-Cl | H | H | | |
| 220 | 2-Cl | 4-SiPr | 6-$CH_3$ | N | 4-Cl | H | H | | |
| 221 | 2-Cl | 4-OnPr | 6-$CH_3$ | N | 4-Cl | 5-Cl | H | | 2.45 |
| 222 | 2-Cl | 4-OiPr | 6-$CH_3$ | N | 4-Cl | 5-Cl | H | | 2.37 |
| 223 | 2-Br | 4-OnPr | 6-$CH_3$ | N | 4-Cl | 5-Cl | H | | 2.60 |
| 224 | 2-Br | 4-OiPr | 6-$CH_3$ | N | 4-Cl | 5-Cl | H | | 2.51 |

Tabelle 2: Verbindungen der Formel (Ib)

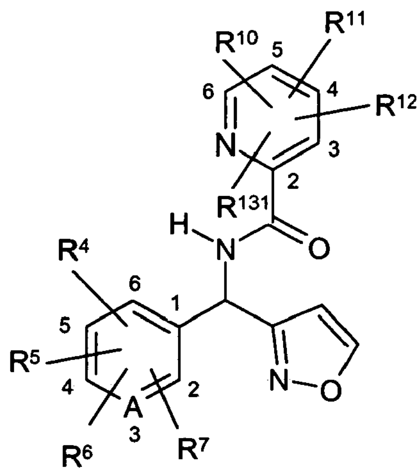

(Ib)

Gemäß Beispiel 6 können die in Tabelle 2 zusammengefassten Verbindungen 225-258 der Formel (Ib) hergestellt werden.

| Beispiel Nr. | $R^{10}$ | $R^{11}$ | $R^{12}$ | A | $R^4$ | $R^5$ | $R^6$ | Fp (˚C) | Log P |
|---|---|---|---|---|---|---|---|---|---|
| 225 | 3-Cl | 5-Cl | H | CH | 4-Cl | H | H | | |
| 226 | 3-Cl | 5-Cl | H | CH | 2-F | 4-$OCH_3$ | H | | |
| 227 | 4-Cl | H | H | CH | 4-Cl | H | H | | |
| 228 | 4-Cl | H | H | CH | 2-F | 4-$OCH_3$ | H | | |
| 229 | 6-Cl | H | H | CH | 4-Cl | H | H | | |
| 230 | 6-Cl | H | H | CH | 2-F | 4-$OCH_3$ | H | | |
| 231 | 6-Cl | H | H | CH | 3-$OCH_3$ | H | H | | |
| 232 | 6-$CH_3$ | H | H | CH | 4-Cl | H | H | | |
| 233 | 6-$CH_3$ | H | H | CH | 2-F | 4-$OCH_3$ | H | | |
| 234 | 6-$CH_3$ | H | H | CH | 3-$OCH_3$ | H | H | | |
| 235 | 4-$CF_3$ | 5-Cl | H | CH | 4-Cl | H | H | | |
| 236 | 4-$CF_3$ | 5-Cl | H | CH | 2-F | 4-$OCH_3$ | H | | |
| 237 | 4-$CF_3$ | 5-Cl | H | CH | 3-$OCH_3$ | H | H | | |
| 238 | 6-Br | H | H | CH | 4-Cl | H | H | | |
| 239 | 6-Br | H | H | CH | 2-F | 4-$OCH_3$ | H | | |
| 240 | 6-Br | H | H | CH | 3-$OCH_3$ | H | H | | |
| 241 | 3-Cl | H | H | CH | 4-Cl | H | H | | |
| 242 | 3-Cl | H | H | CH | 2-F | 4-$OCH_3$ | H | | |
| 243 | 3-Cl | H | H | CH | 3-$OCH_3$ | H | H | | |
| 244 | 4-Cl | H | H | CH | 3-$OCH_3$ | H | H | | |
| 245 | 3-$CF_3$ | H | H | CH | 4-Cl | H | H | 135 | |
| 246 | 3-$CF_3$ | H | H | CH | 2-F | 4-$OCH_3$ | H | | |
| 247 | 3-$CF_3$ | H | H | CH | 3-$OCH_3$ | H | H | | |

(fortgesetzt)

| Gemäß Beispiel 6 können die in Tabelle 2 zusammengefassten Verbindungen 225-258 der Formel (Ib) hergestellt werden. | | | | | | | | | |
|---|---|---|---|---|---|---|---|---|---|
| Beispiel Nr. | $R^{10}$ | $R^{11}$ | $R^{12}$ | A | $R^4$ | $R^5$ | $R^6$ | Fp (˚C) | Log P |
| 248 | 3-Cl | 6-Cl | H | CH | 4-Cl | H | H | | |
| 249 | 3-Cl | 6-Cl | H | CH | 2-F | 4-$OCH_3$ | H | | |
| 250 | 3-Cl | 6-Cl | H | CH | 3-$OCH_3$ | H | H | | |
| 251 | 6-F | H | H | CH | 4-Cl | H | H | | |
| 252 | 6-F | H | H | CH | 2-F | 4-$OCH_3$ | H | | |
| 253 | 6-F | H | H | CH | 3-$OCH_3$ | H | H | | |
| 254 | 4-$CH_3$ | H | H | CH | 2-F | 4-$OCH_3$ | H | | |
| 255 | 4-$CH_3$ | H | H | CH | 3-$OCH_3$ | H | H | | |
| 256 | 3-S(O) $CH_3$ | 5-$CF_3$ | H | CH | 4-Cl | H | H | | |
| 257 | 3-S(O) $CH_3$ | 5-$CF_3$ | H | CH | 2-F | 4-$OCH_3$ | H | | |
| 258 | 3-S(O) $CH_3$ | 5-$CF_3$ | H | CH | 3-$OCH_3$ | H | H | | |

Tabelle 3: Verbindungen der Formel (Ic)

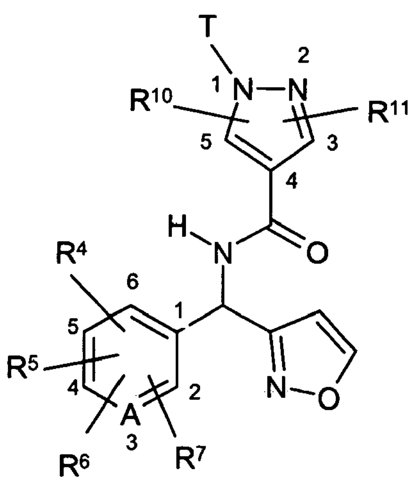

(Ic)

| Gemäß Beispiel 7 können die in Tabelle 3 zusammengefassten Verbindungen 259-279 der Formel (Ic) hergestellt werden. | | | | | | | | | |
|---|---|---|---|---|---|---|---|---|---|
| Beispiel Nr. | T | $R^{10}$ | $R^{11}$ | A | $R^4$ | $R^5$ | $R^6$ | Fp (˚C) | Log P |
| 259 | 1-$CH_3$ | 5-Cl | H | CH | 4-Cl | H | H | 151 | |
| 260 | 1-$CH_3$ | 5-Cl | H | CH | 2-F | 4-$OCH_3$ | H | 152 | 1.80 |
| 261 | 1-$CH_3$ | 3-$CH_3$ | 5-Cl | CH | 2-F | 4-$OCH_3$ | H | 90 | |
| 262 | 1-$CH_3$ | 3-$CF_3$ | 5-Cl | CH | 4-Cl | H | H | 147 | |

(fortgesetzt)

| Gemäß Beispiel 7 können die in Tabelle 3 zusammengefassten Verbindungen 259-279 der Formel (Ic) hergestellt werden. | | | | | | | | | |
|---|---|---|---|---|---|---|---|---|---|
| Beispiel Nr. | T | $R^{10}$ | $R^{11}$ | A | $R^4$ | $R^5$ | $R^6$ | Fp (°C) | Log P |
| 263 | 1-$CH_3$ | 3-$CF_3$ | 5-Cl | CH | 2-F | 4-$OCH_3$ | H | 127 | |
| 264 | 1-$CH_3$ | 3-$CF_3$ | 5-Cl | CH | 3-$OCH_3$ | H | H | 166 | |
| 265 | 1-$CH_3$ | 3-$CH_3$ | H | CH | 4-Cl | H | H | 135 | |
| 266 | 1-$CH_3$ | 3-$CH_3$ | H | CH | 2-F | 4-$OCH_3$ | H | 137 | |
| 267 | 1-Phenyl | 3-$CH_3$ | 5-$CH_3$ | CH | 2-F | 4-$OCH_3$ | H | | |
| 268 | 1-Phenyl | 3-$CH_3$ | 5-$CH_3$ | CH | 3-$OCH_3$ | H | H | | |
| 269 | 1-$CH_3$ | 3-cycloPr | 5-Cl | CH | 4-Cl | H | H | | |
| 270 | 1-$CH_3$ | 3-cycloPr | 5-Cl | CH | 2-F | 4-$OCH_3$ | H | | |
| 271 | 1-iBu | 3-$CH_3$ | 5-Cl | CH | 4-Cl | H | H | 138 | |
| 272 | 1-iBu | 3-$CH_3$ | 5-Cl | CH | 2-F | 4-$OCH_3$ | H | | |
| 273 | 1-iBu | 3-$CH_3$ | 5-Cl | CH | 3-$OCH_3$ | H | H | | |
| 274 | 1-nBu | 3-$CH_3$ | 5-Cl | CH | 4-Cl | H | H | 155 | |
| 275 | 1-nBu | 3-$CH_3$ | 5-Cl | CH | 2-F | 4-$OCH_3$ | H | | |
| 276 | 1-nBu | 3-$CH_3$ | 5-Cl | CH | 3-$OCH_3$ | H | H | | |
| 277 | 1-$CH_3$ | 3-Et | 5-Cl | CH | 4-Cl | H | H | | |
| 278 | 1-$CH_3$ | 3-Et | 5-Cl | CH | 2-F | 4-$OCH_3$ | H | | |
| 279 | 1-$CH_3$ | 3-Et | 5-Cl | CH | 3-$OCH_3$ | H | H | | |

Tabelle 4: Verbindungen der Formel (Id)

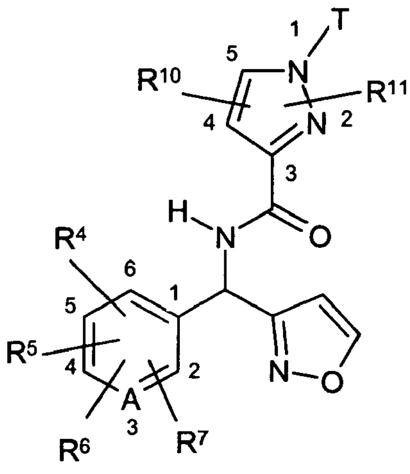

(Id)

(fortgesetzt)

| Gemäß Beispiel 7 können auch die in Tabelle 4 zusammengefassten Verbindungen 280-284 der Formel (Id) hergestellt werden. | | | | | | | | | |
|---|---|---|---|---|---|---|---|---|---|
| Beispiel Nr. | T | $R^{10}$ | $R^{11}$ | A | $R^4$ | $R^5$ | $R^6$ | Fp (˚C) | Log P |
| 280 | 1-$CH_3$ | 5-$CH_3$ | H | CH | 4-Cl | H | H | | |
| 281 | 1-$CH_3$ | 5-$CH_3$ | H | CH | 2-F | 4-$OCH_3$ | H | | |
| 282 | 1-$CH_3$ | 5-Phenyl | H | CH | 4-Cl | H | H | | |
| 283 | 1-$CH_3$ | 5-Phenyl | H | CH | 2-F | 4-$OCH_3$ | H | | |
| 284 | 1-$CH_3$ | 5-Phenyl | H | CH | 3-$OCH_3$ | H | H | | |

Tabelle 5: Verbindungen der Formel (Ie)

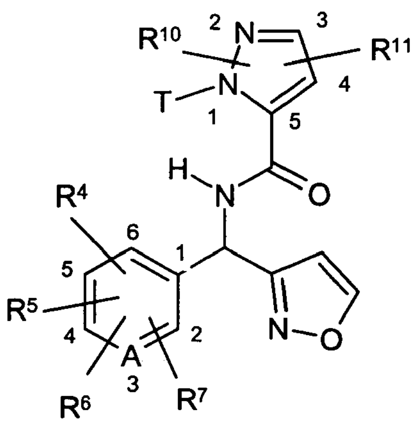

(Ie)

| Gemäß Beispiel 7 können außerdem die in Tabelle 5 zusammengefassten Verbindungen 285-287 der Formel (Ie) hergestellt werden. | | | | | | | | | |
|---|---|---|---|---|---|---|---|---|---|
| Beispiel Nr. | T | $R^{10}$ | $R^2$ | A | $R^4$ | $R^5$ | $R^6$ | Fp (˚C) | Log P |
| 285 | 1-$CH_3$ | 3-Phenyl | H | CH | 4-Cl | H | H | | |
| 286 | 1-$CH_3$ | 3-Phenyl | H | CH | 2-F | 4-$OCH_3$ | H | | |
| 287 | 1-$CH_3$ | 3-Phenyl | H | CH | 3-$OCH_3$ | H | H | | |

Tabelle 6: Verbindungen der Formel (If)

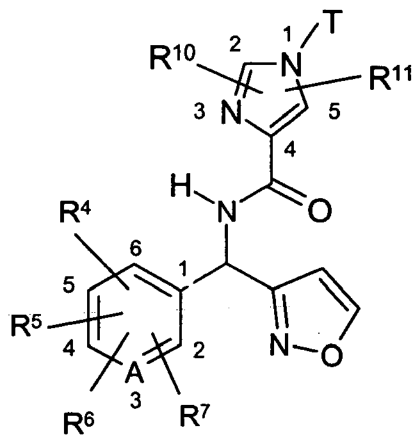

(If)

Gemäß Beispiel 8 können die in Tabelle 6 zusammengefassten Verbindungen 288-289 der Formel (If) hergestellt werden.

| Beispiel Nr. | T | $R^{10}$ | $R^{11}$ | A | $R^4$ | $R^5$ | $R^6$ | Fp (°C) | Log P |
|---|---|---|---|---|---|---|---|---|---|
| 288 | 1-Benzyl | 5-Cl | H | CH | 2-F | 4-$OCH_3$ | H | | |
| 289 | 1-Benzyl | 5-$NO_2$ | H | CH | 4-Cl | H | H | | 2.63 |

Tabelle 7: Verbindungen der Formel (Ig)

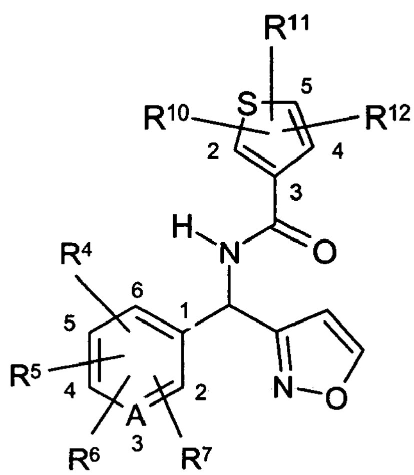

(Ig)

Gemäß Beispiel 9 können die in Tabelle 7 zusammengefassten Verbindungen 290-301 der Formel (Ig) hergestellt werden.

| Beispiel Nr. | $R^{10}$ | $R^{11}$ | $R^{12}$ | A | $R^4$ | $R^5$ | $R^6$ | Fp (°C) | Log P |
|---|---|---|---|---|---|---|---|---|---|
| 290 | 5-Br | 4-$OCH_3$ | H | CH | 4-Cl | H | H | | 3.64 |
| 291 | 5-Br | 4-$OCH_3$ | H | CH | 2-F | 4-$OCH_3$ | H | | |
| 292 | 2-Br | 4-$CH_3$ | 5-$CH_3$ | CH | 4-Cl | H | H | 135 | |
| 293 | 4-$CH_3$ | H | H | CH | 4-Cl | H | H | 138 | |
| 294 | 4-$CH_3$ | H | H | CH | 2-F | 4-$OCH_3$ | H | 133 | |

(fortgesetzt)

| Gemäß Beispiel 9 können die in Tabelle 7 zusammengefassten Verbindungen 290-301 der Formel (Ig) hergestellt werden. | | | | | | | | | |
|---|---|---|---|---|---|---|---|---|---|
| Beispiel Nr. | $R^{10}$ | $R^{11}$ | $R^{12}$ | A | $R^4$ | $R^5$ | $R^6$ | Fp (˚C) | Log P |
| 295 | 5-$CH_3$ | H | H | CH | 4-Cl | H | H | 145 | |
| 296 | 5-$CH_3$ | H | H | CH | 2-F | 4-$OCH_3$ | H | 123 | |
| 297 | 4-$OCH_3$ | H | H | CH | 4-Cl | H | H | | |
| 298 | 4-$OCH_3$ | H | H | CH | 2-F | 4-$OCH_3$ | H | | |
| 299 | 4-$OCH_3$ | 5-Cl | H | CH | 4-Cl | H | H | | |
| 300 | 4-$OCH_3$ | 5-Cl | H | CH | 2-F | 4-$OCH_3$ | H | | |
| 301 | 4-$OCH_3$ | 5-Cl | H | CH | 3-$OCH_3$ | H | H | | |

Tabelle 8: Verbindungen der Formel (Ih)

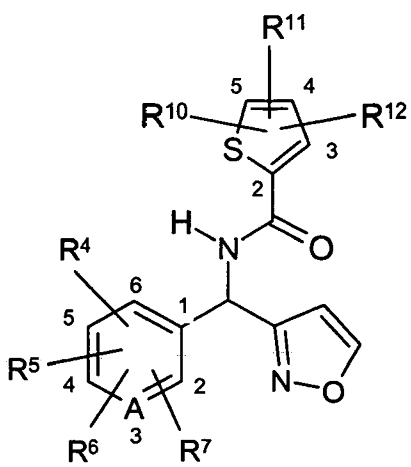

(Ih)

| Gemäß Beispiel 9 können auch die in Tabelle 8 zusammengefassten Verbindungen 302-304 der Formel (Ih) hergestellt werden. | | | | | | | | | |
|---|---|---|---|---|---|---|---|---|---|
| Beispiel Nr. | $R^{10}$ | $R^{11}$ | $R^{12}$ | A | $R^4$ | $R^5$ | $R^6$ | Fp (˚C) | Log P |
| 302 | 3,4-$OCH_2CH_2O$ | | H | CH | 4-Cl | H | H | | |
| 303 | 3,4-$OCH_2CH_2O$ | | H | CH | 2-F | 4-$OCH_3$ | H | | |
| 304 | 3,4-$OCH_2CH_2O$ | | H | CH | 3-$OCH_3$ | H | H | | |

Tabelle 9: Verbindungen der Formel (Ii)

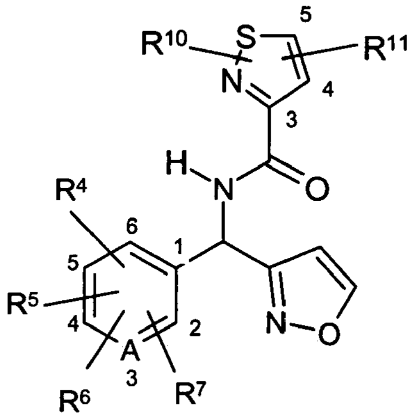

(Ii)

Gemäß Beispiel 10 können die in Tabelle 9 zusammengefassten Verbindungen 305-306 der Formel (Ii) hergestellt werden

| Beispiel Nr. | $R^{10}$ | $R^{11}$ | $R^{12}$ | A | $R^4$ | $R^5$ | $R^6$ | Fp(˚C) | Log P |
|---|---|---|---|---|---|---|---|---|---|
| 305 | 4-Cl | 5-Cl | H | CH | 4-Cl | H | H | | |
| 306 | 4-Cl | 5-Cl | H | CH | 3-$OCH_3$ | H | H | | 3.44 |

Tabelle 10: Verbindungen der Formel (Ij)

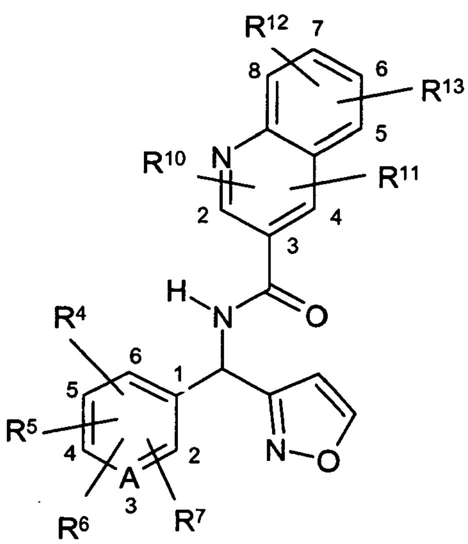

(Ij)

Gemäß Beispiel 11 können die in Tabelle 10 zusammengefassten Verbindungen 307-311 der Formel (Ij) erhalten werden.

| Beispiel Nr. | $R^{10}$ | $R^{11}$ | $R^{12}$ | A | $R^4$ | $R^5$ | $R^6$ | Fp(˚C) | Log P |
|---|---|---|---|---|---|---|---|---|---|
| 307 | H | H | H | CH | 4-Cl | H | H | | |
| 308 | H | H | H | CH | 3-$OCH_3$ | H | H | | |
| 309 | 2-Cl | 4-Cl | H | CH | 4-Cl | H | H | 215 | |
| 310 | 2-Cl | 4-Cl | H | CH | 2-F | 4-$OCH_3$ | H | | |

(fortgesetzt)

| Gemäß Beispiel 11 können die in Tabelle 10 zusammengefassten Verbindungen 307-311 der Formel (Ij) erhalten werden. | | | | | | | | | |
|---|---|---|---|---|---|---|---|---|---|
| Beispiel Nr. | $R^{10}$ | $R^{11}$ | $R^{12}$ | A | $R^4$ | $R^5$ | $R^6$ | Fp(°C) | Log P |
| 311 | 2-Cl | 4-Cl | H | CH | 3-$OCH_3$ | H | H | | |

Beispiel 312: 5-Chlor-N-[(2-fluor-4-methoxyphenyl)(isoxazol-3-yl)methyl]-1-methyl-1 H-pyrazol-4-carbothioamid

0,110 g (0, 302 mmol) 5-Chlor-N-[(2-fluor-4-methoxyphenyl)(isoxazol-3-yl)methyl]-1-methyl-1 H-pyrazol-4-carboxamid werden in 5 ml Toluol gelöst, mit 0,244 g (0,603 mmol) Lawessons Reagenz versetzt und 6 Stunden am Rückfluß gekocht. Die Mischung wird nach dem Abkühlen auf Raumtemperatur zweimal mit Wasser gewaschen. Die organische Phase wird über $MgSO_4$ getrocknet und eingengt. Das Rohprodukt wird durch präparative HPLC an Purospher RP18-e (Merck) gereinigt. Es werden 0,020 g (17,4 % der Theorie) eines wachsartigen Feststoffs erhalten.
Log P: 1,76
1 H-NMR ($CDCl_3$, 400 MHz) δ 9,10 (d, 1 H) 8,38 (d, 1 H) 8,16 (s, 1 H) 7,37 (t, 1 H) 7,15 (d, 1 H) 6,67 (m, 2H) 6,26 (d, 1 H) 3,88 (s, 3H) 3,79 (s, 3H)
MS(CI) 381 $(M+H)^+$

Beispiel 313: 5-Brom-N-[(4-chlorphenyl)(isoxazol-3-yl)methyl]-4-methoxythiophen-3-carbothioamid

0,070 g (0, 164 mmol) 5-Brom-N-[(4-chlorphenyl)(isoxazol-3-yl)methyl]-4-methoxythiophen-3-carboxamid werden in 5 ml Toluol gelöst, mit 0,132 g (0,327 mmol) Lawessons Reagenz versetzt und 4 Tage am Rückfluß gekocht. Die Mischung wird nach dem Abkühlen auf Raumtemperatur zweimal mit Wasser gewaschen. Die organische Phase wird über $MgSO_4$ getrocknet und eingengt. Das Rohprodukt wird durch präparative HPLC an Purospher RP18-e (Merck) gereinigt. Es werden 0,030 g (40,81 % der Theorie) eines wachsartigen Feststoffs mit einer Reinheit von 99% erhalten.
Log P: 4,51
1 H-NMR ($CDCl_3$, 400 MHz) δ 10,60 (s, br, 1 H) 8,40 (d, 1 H) 8,33 (s, 1 H) 7,46 (dd, 4H) 7,08 (d, 1 H) 6,20 (d, 1 H) 4,05 (s, 3H)
MS(CI) 443 $(M+H)^+$

Beispiel 314: (+)-2-Chlor-N-[(4-chlorphenyl)(isoxazol-3-yl)methyl]-4-isopropoxy-6-methylnicotinamid

0,119 g racemisches 2-Chlor-N-[(4-chlorphenyl)(isoxazol-3-yl)methyl]-4-isopropoxy-6-methylnicotinamid (Beispiel 152) wurden mittels präparativer HPLC an einer chiralen Phase (Chiralcel OD 20 μm der Firma Chiral Technologies Europe, Illkirch, Frankreich; Säulendimension 250 x 50 mm) getrennt.
Nach Abdestillieren des Eluenten verblieben 0,022 g (+)-2-Chlor-N-[(4-chlorphenyl)(isoxazol-3-yl)methyl]-4-isopropoxy-6-methylnicotinamid mit einem spezifischen Drehwert von $[\alpha]^{23}{}_{539}$= 31,2°, was einem Enantiomerenüberschuß von 97 % entspricht (bestimmt durch analytische HPLC an Chiralpack OD 5 μm der Firma Chiral Technologies Europe, Illkirch, Frankreich; Säulendimension 250 x 4,6 mm).
1 H-NMR ($CDCl_3$, 400 MHz) δ 8,40 (d,1 H) 7,36 (m, 4H) 6,91 (d, 1 H) 6,61 (s, 1 H) 6,47 (d, 1 H) 6,29 (d, 1H) 4,60 (m, 1 H) 2,49 (s, 3H) 1,28 (d, 6H)
MS(CI) 420 $(M+H)^+$

Beispiel 315: (-)-2-Chlor-N-[(4-chlorphenyl)(isoxazol-3-yl)methyl]-4-isopropoxy-6-methylnicotinamid

0,119 g racemisches 2-Chlor-N-[(4-chlorphenyl)(isoxazol-3-yl)methyl]-4-isopropoxy-6-methylnicotinamid (Beispiel 152) wurden mittels präparativer HPLC an einer chiralen Phase (Chiralcel OD 20 μm der Firma Chiral Technologies Europe, Illkirch, Frankreich; Säulendimension 250 x 50 mm) getrennt.
Nach Abdestillieren des Eluenten verblieben 0,011 g (-)-2-Chlor-N-[(4-chlorphenyl)(isoxazol-3-yl)methyl]-4-isopropoxy-6-methylnicotinamid mit einem Enantiomerenüberschuß von 83 % (bestimmt durch analytische HPLC an Chiralpack OD 5 μm der Firma Chiral Technologies Europe, Illkirch, Frankreich; Säulendimension 250 x 4,6 mm).

Beispiel 316: (+)-2-Chlor-N-[(3,4-difluorphenyl)(isoxazol-3-yl)methyl]-4-isopropoxy-6-methylnicotinamid

0,360 g racemisches 2-Chlor-N-[(3,4-difluorphenyl)(isoxazol-3-yl)methyl]-4-isopropoxy-6-methylnicotinamid (Beispiel 174) wurden mittels präparativer HPLC an einer chiralen Phase (Chiralcel IC 20 μm der Firma Chiral Technologies Europe, Illkirch, Frankreich; Säulendimension 250 x 50 mm) getrennt.
Nach Abdestillieren des Eluenten verblieben 0,071 g (+)-2-Chlor-N-[(4-chlorphenyl)(isoxazol-3-yl)methyl]-4-isopropoxy-6-methylnicotinamid mit einem spezifischen Drehwert von $[\alpha]^{23}{}_{589}$= 33,3°, was einem Enantiomerenüberschuß von 100% entspricht (bestimmt durch analytische HPLC an Chiralpack IC 5 μm der Firma Chiral Technologies Europe, Illkirch, Frankreich; Säulendimension 250 x 4,6 mm).
1 H-NMR ($CDCl_3$, 400 MHz) δ 8,42 (d,1 H) 7,28 (m, 1 H) 7,17 (m, 2H) 6,95 (d, 1 H) 6,62 (s, 1 H) 6,45 (d, 1 H) 6,30 (d,1 H) 4,63 (m, 1 H) 2,50 (s, 3H) 1,30 (dd, 6H)
MS(CI) 422 $(M+H)^+$

Beispiel 317: (-)-2-Chlor-N-[(3,4-difluorphenyl)(isoxazol-3-yl)methyl]-4-isopropoxy-6-methylnicotinamid

0,040 g racemisches 2-Chlor-N-[(3,4-difluorphenyl)(isoxazol-3-yl)methyl]-4-isopropoxy-6-methylnicotinamid (Beispiel 174) wurden mittels präparativer HPLC an einer chiralen Phase (Chiralcel IC 20 μm der Firma Chiral Technologies Europe, Illkirch, Frankreich; Säulendimension 250 x 50 mm) getrennt.
Nach Abdestillieren des Eluenten verblieben 0,061 g (-)-2-Chlor-N-[(4-chlorphenyl)(isoxazol-3-yl)methyl]-4-isopropoxy-6-methylnicotinamid mit einem spezifischen Drehwert von $[\alpha]^{23}{}_{589}$= -31,7°, was einem Enantiomerenüberschuß von 97% entspricht (bestimmt durch analytische HPLC an Chiralpack IC 5 μm der Firma Chiral Technologies Europe, Illkirch, Frankreich; Säulendimension 250 x 4,6 mm).

Beispiel 318: (-)-5-Chlor-N-[(2-fluor-4-methoxyphenyl)(isoxazol-3-yl)methyl]-1-methyl-1 H-pyrazol-4-carboxamid

0,040 g racemisches 5-Chlor-N-[(2-fluor-4-methoxyphenyl)(isoxazol-3-yl)methyl]-1-methyl-1 H-pyrazol-4-carboxamid (Beispiel 260) wurden mittels präparativer HPLC an einer chiralen Phase (Chiralcel OD 20 μm der Firma Chiral Technologies Europe, Illkirch, Frankreich; Säulendimension 250 x 50 mm) getrennt.
Nach Abdestillieren des Eluenten verblieben 0,010 g (-)-5-Chlor-N-[(2-fluor-4-methoxyphenyl)(isoxazol-3-yl)methyl]-1-methyl-1 H-pyrazol-4-carboxamid mit einem spezifischen Drehwert von $[\alpha]^{23}{}_{589}$= - 40°, was einem Enantiomerenüberschuß von 97% entspricht (bestimmt durch analytische HPLC an Chiralpack IC 5 μm der Firma Chiral Technologies Europe, Illkirch, Frankreich; Säulendimension 250 x 4,6 mm).
1 H-NMR ($CDCl_3$, 400 MHz) δ 8,35 (d, 1 H) 7,95 (s, 1 H) 7,46 (d, 1 H) 7,31 (t, 1 H) 6,66 (m, 2H) 6,56 (d, 1 H) 6,25 (d,1 H) 3,88 (s, 3H) 3,78 (s, 3H)
MS(CI) 365 $(M+H)^+$

Beispiel 319: (+)-5-Chlor-N-[(2-fluor-4-methoxyphenyl)(isoxazol-3-yl)methyl]-1-methyl-1H-pyrazol-4-carboxamid

0,040 g racemisches 5-Chlor-N-[(2-fluor-4-methoxyphenyl)(isoxazol-3-yl)methyl]-1-methyl-1 H-pyrazol-4-carboxamid (Beispiel 260) wurden mittels präparativer HPLC an einer chiralen Phase (Chiralcel OD 20 μm der Firma Chiral Technologies Europe, Illkirch, Frankreich; Säulendimension 250 x 50 mm) getrennt.
Nach Abdestillieren des Eluenten verblieben 0,009 g (+)-5-Chlor-N-[(2-fluor-4-methoxyphenyl)(isoxazol-3-yl)methyl]-1-methyl-1 H-pyrazol-4-carboxamid mit einem Enantiomerenüberschuß von 81 % (bestimmt durch analytische HPLC an Chiralpack IC 5 μm der Firma Chiral Technologies Europe, Illkirch, Frankreich; Säulendimension 250 x 4,6 mm).

Beispiel 320: (-)-4,5-Dichlor-N-[(3-methoxyphenyl)(isoxazot-3-yl)methyl]isothiazol-3-carboxamid

0,140 g racemisches 4,5-Dichlor-N-[(3-methoxyphenyl)(isoxazol-3-yl)methyl]isothiazol-3-carboxamid (Beispiel 306) wurden mittels präparativer HPLC an einer chiralen Phase (Chiralcel IC 20 μm der Firma Chiral Technologies Europe, Illkirch, Frankreich; Säulendimension 250 x 50 mm) getrennt.
Nach Abdestillieren des Eluenten verblieben 0,049 g (-)-4,5-Dichlor-N-[(3-methoxyphenyl)(isoxazol-3-yl)methyl]isothiazol-3-carboxamid mit einem spezifischen Drehwert von $[\alpha]^{23}{}_{589}$= -88,7°, was einem Enantiomerenüberschuß von 100% entspricht (bestimmt durch analytische HPLC an Chiralpack IC 5 μm der Firma Chiral Technologies Europe, Illkirch, Frankreich; Säulendimension 250 x 4,6 mm).
1 H-NMR ($CDCl_3$, 400 MHz) δ 8,38 (d,1 H) 8,22 (d, 1 H) 7,30 (t, 1 H) 7,00 (d, 1 H) 6,93 (m, 1 H) 6,85 (dd, 1 H) 6,40 (d, 1 H) 6,22 (d,1 H) 3.79 (s, 3H)
MS(CI) 384 $(M+H)^+$

Beispiel 321: (+)-4,5-Dichlor-N-[(3-methoxyphenyl)(isoxazol-3-yl)methyl]isothiazol-3-carboxamid

0,140 g racemisches 4,5-Dichlor-N-[(3-methoxyphenyl)(isoxazol-3-yl)methyl]isothiazol-3-carboxamid (Beispiel 306) wurden mittels präparativer HPLC an einer chiralen Phase (Chiraicel IC 20 µm der Firma Chiral Technologies Europe, Illkirch, Frankreich; Säulendimension 250 x 50 mm) getrennt.
Nach Abdestillieren des Eluenten verblieben 0,032 g (+)-4,5-Dichlor-N-[(3-methoxyphenyl)(isoxazol-3-yl)methyl]isothiazol-3-carboxamid mit einem spezifischen Drehwert von $[\alpha]^{23}{}_{589}$= 80°, was einem Enantiomerenüberschuß von 91% entspricht (bestimmt durch analytische HPLC an Chiralpack IC 5 µm der Firma Chiral Technologies Europe, Illkirch, Frankreich; Säulendimension 250 x 4,6 mm).
Synthese der Ausgangsmaterialien, Amine der allgemeinen Formal (IVa):

Beispiel 322: 1-Isoxazol-3-yl-1-(3-methoxyphenyl)methanamin

Stufe A: Isoxazol-3-yl(3-methoxyphenyl)methanon

30,00 g (0,20 mol) 3-Methoxyacetophenon werden in 500 ml Diethylether gelöst und mit 200 ml einer 4M Lösung von Chlorwasserstoff in 1,4-Dioxan versetzt. Die Mischung wird mit einem Eisbad gekühlt und anschließend werden 62,00 g (0,60 mol) n-Butylnitrit so zugetropft, dass die Temperatur nicht über 10°C steigt. Nach beendeter Zugabe wird auf Raumtemperatur erwärmt und 18 Stunden bei Raumtemperatur gerührt. Die etherische Reaktionsmischung wird mit gesättigter Kochsalzlösung gewaschen. Die organische Phase wird abgetrennt, über $MgSO_4$ getrocknet und eingeengt. Man erhält 55,30 g eines gelblichen Feststoffs, der ohne weitere Reinigung in 800 ml 2-Propanol gelöst wird.
In die so erhaltene Lösung wird 30 min Acetylen eingeleitet, dann werden 21,75 g (0,26 mol) Natiumhydrogencarbonat zugefügt und weitere 60 min Acetylen eingeleitet. Nach Zugabe einer zweiten Portion von 21,75 g (0,26 mol) Natriumhydrogencarbonat wird weitere 8 Stunden Acetylen eingeleitet. Die Reaktionsmischung wird 18 Stunden bei Raumtemperatur gerührt und dann in 300 ml Diethylether gegossen. Die so erhaltene Mischung wird mit Wasser gewaschen. Die organische Phase wird abgetrennt, über $MgSO_4$ getrocknet und eingeengt. Es werden 44,00 g eines orange-roten Öls erhalten, dass durch präparative HPLC an einer RP-18 Phase (YMC-Gel ODS-A, 10µm der Firma YMC-Europe, Dinslaken; Säulendimension 400 x 100 mm) gereinigt wird. Man erhält auf diese Weise 25,50 g (48,50 % der Theorie) Isoxazol-3-yl(3-methoxyphenyl)methanon.
1 H-NMR ($CDCl_3$, 400 MHz) δ 8,55 (d, 1 H) 7,95 (dd, 1 H) 7,80 (m, 1 H) 7,43 (t, 1 H) 7,20 (dd, 1 H) 6,90 (d, 1 H) 3,90 (s, 3H)
MS(CI) 204 $(M+H)^+$

Stufe B: Isoxazol-3-yl(3-methoxyphenyl)methanon Oxim

25,50 g (0,125 mol) Isoxazol-3-yl(3-methoxyphenyl)methanon aus Stufe A werden in 300 ml Ethanol gelöst und mit einer Lösung von 52,32 g (0,753 mol) Hydroxylaminhydrochlorid in 50 ml Wasser versetzt. Die Mischung wird 4 Stunden am Rückfluß gekocht, dann wird das Ethanol abdestilliert. Der Rückstand wird in Wasser aufgenommen und mit Diethylether extrahiert. Die organische Phase wird abgetrennt, über $MgSO_4$ getrocknet und eingeengt. Man erhält 27,00 g (98,6 % der Theorie) eines gelben Öls welches ein Gemisch der syn- und anti-Isomeren darstellt (doppelter Signalsatz im NMR).

Isomer A:

1 H-NMR ($CDCl_3$, 400 MHz) δ 8,55 (d, 1 H) 7,30 (t, 1 H) 7,18 (m, 1 H) 7,15 (m, 1 H) 7,00 (t, 1 H) 6,81 (d, 1 H) 3,82 (s, 3H)

Isomer B:

1 H-NMR ($CDCl_{3,}$ 400 MHz) δ 8,41 (d, 1 H) 7,40 (t, 1 H) 7,10 (m, 1 H) 7,08 (m, 1 H) 6,99 (t, 1 H) 6,70 (d, 1 H) 3,82 (s, 3H)
MS(CI) 219 $(M+H)^+$

Stufe C: N-[(1Z)-Isoxazol-3-yl(3-methoxyphenyl)methylen]-P,P-diphenylphosphinsäureamid

27,00 (0,124 mol) Isoxazol-3-yl(3-methoxyphenyl)methanon Oxim aus Stufe B werden in einer Mischung aus 300 ml Dichlormethan und 300 ml n-Heptan gelöst und mit 66,36 g (0,656 mol) Triethylamin versetzt. Bei 0°C werden

langsam 30,03 g (0,136 mol) Chlordiphenylphosphin zugetropft. Die Mischung wird 1 Stunde bei 0˚C und anschließend 18 Stunden bei Raumtemperatur gerührt. Das Lösungsmittel wird vollständig abdestilliert und der verbleibende Rückstand in einem Gemisch aus Ethylacetat und Wasser aufgenommen. Die organische Phase wird abgetrennt, über $MgSO_4$ getrocknet und eingeengt. Man erhält 55,60 g eines wachsartigen Feststoffs mit einer Reinheit von 88,7% (HPLC, 99,0 % der Theorie).

Stufe D: N-[Isoxazol-3-yl(3-methoxyphenyl)methyl]-P,P-diphenylphosphinsäureamid

55,60 g des rohen N-[(1Z)-Isoxazol-3-yl(3-methoxyphenyl)methylen]-P,P-diphenylphosphinsäureamids aus Stufe C (Gehalt: 88,7% entspricht 0,123 mol) werden in 200 ml Tetrahydrofuran gelöst. Die so erhaltene Lösung wird bei Raumtemperatur portionsweise mit 5,75 g (0,153 mol) Natriumborhydrid versetzt und anschließend 3 Stunden bei 40 ˚C und 2 Stunden bei Raumtemperatur gerührt. Dann werden vorsichtig 200 ml einer gesättigten Ammoniumchloridlösung hinzugefügt. Die Rektionsmischung wird komplett eingeengt und der Rückstand in einem Gemisch aus Ethylacetat und Wasser aufgenommen. Die organische Phase wird abgetrennt, über $MgSO_4$ getrocknet und eingeengt. Man erhält 50,00 g eines wachsartigen Feststoffs.

Stufe E: 1-Isoxazol-3-yl-1-(3-methoxyphenyl)methanamin

50,00 g des rohen -[Isoxazol-3-yl(3-methoxyphenyl)methyl]-P,P-diphenylphosphinsäureamids aus Stufe D werden in 300 ml halbkonzentrierter Salzsäure aufgenommen und 10 Stunden am Rückfluß gekocht. Nach Abkühlen auf Raumtemperatur wird die Mischung filtriert und anschließend mit Ethylacetat extrahiert. Der organische Extrakt wird verworfen. Die wässrige Lösung wird mit Natronlauge vorsichtig basisch gestellt und anschließend mit Dichlormethan extrahiert. Die organische Phase wird über $MgSO_4$ getrocknet und eingeengt. Man erhält 15,50 g (61,7 % der Theorie bezogen auf Stufe C) eines hellgelben Öls.

1 H-NMR ($CDCl_3$, 400 MHz) δ 8,30 (d, 1 H) 7,26 (m, 1 H) 7,00 (m, 2H) 6,82 (m, 1 H) 6,20 (d, 1 H) 5.85 (s, 1 H) 3,80 (s, 3H) MS(CI) 205 $(M+H)^+$

Die Massenspektroskopie wurde mit Hilfe der Ionisationsmethode durchgeführt, wobei die Angabe "(CI)" chemische Ionisation bedeutet, d.h. nachgewiesen wird der Molpeak der protonierten Verbindungen $(m + H)^+$.

Auf analoge Weise können die in Tabelle 11 zusammengefassten Amine 312-341 der Formel (IVa) erhalten werden.

Tabelle 11: Verbindungen der Formel (IVa):

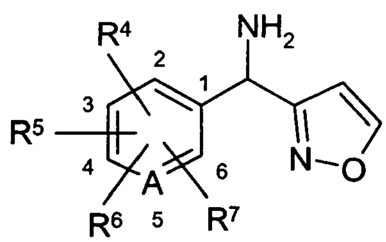

(IVa)

| Beispiel Nr. | A | $R^4$ | $R^5$ | $R^6$ | 1 H-NMR ($CDCl_3$, 400 MHz) δ | MS(CI) $(M+H)^+$ |
|---|---|---|---|---|---|---|
| 312 | CH | 4-Cl | H | H | 8,31 (d, 1H) 7,37 (d, 2H) 7,31 (d, 2H) 6,20 (d, 1 H) 5,46 (s, 1 H) | 209 |
| 313 | CH | 3-$CH_3$ | H | H | 8,30 (d, 1H) 7,23 (m, 3H) 7,10 (m, 1H) 6,20 (d, 1 H) 5,31 (s, 1 H) 2.35 (s, 3H) | 189 |

(fortgesetzt)

| Beispiel Nr. | A | $R^4$ | $R^5$ | $R^6$ | 1 H-NMR ($CDCl_3$, 400 MHz) δ | MS(CI) $(M+H)^+$ |
|---|---|---|---|---|---|---|
| 314 | CH | 2-Cl | 4-Cl | H | 8,33 (d, 1H) 7,48 (d, 1 H) 7,40 (d, 1H) 7,28 (dd, 1H) 6,20 (d, 1 H) 5,72 (s, 1 H) | |
| 315 | CH | 3-Cl | H | H | 8,31 (d, 1 H) 7,46 (m, 1 H) 7,29 (m, 3H) 6,20 (d, 1 H) 5,45 (s, 1 H) | 209 |
| 316 | CH | 4-CN | H | H | 8,34 (d, 1H) 7,68 (d, 2H) 7,58 (d, 2H) 6,20 (d, 1 H) 5,45 (s, 1 H) | 200 |
| 317 | CH | 2-Cl | H | H | 8,32 (d, 1 H) 7,51 (dd, 1 H) 7,49 (dd, 1 H) 7,28 (m, 2H) 6,20 (d, 1 H) 5,77 (s, 1 H) | 209 |
| 318 | CH | 2-Cl | 3-Cl | 4-($H_3CCH_2$) $SO_2$ | 8.38 (d, 1 H) 8,10 (d, 1 H) 7,77 (d, 1 H) 6,25 (d, 1H) 5,84 (s, 1 H) 3,44 (q, 2H) 1,30 (t, 3H) | 335 |
| 319 | CH | 2-$CF_3$ | H | H | 8,31 (d, 1 H) 7,68 (d, 1H) 7,62 (d, 1 H) 7.55 (t, 1H) 7.40 (t, 1H) 6,11 (d, 1 H) 5,72 (s, 1 H) | 243 |
| 320 | CH | 4-$OCF_3$ | H | H | 8,33 (d, 1H) 7,49 (d, 2H) 7,22 (d, 2H) 6,21 (d, 1 H) 5,42 (s, 1H) | 259 |
| 321 | CH | 3-Br | H | H | 8,31 (d, 1 H) 7,61 (m, 1 H) 7,42 (m, 1H) 7.35 (d, 1H) 7.22 (t, 1 H) 6,20 (d, 1 H) 5,35 (s, 1 H) | 253 |
| 322 | CH | 3-CN | H | H | | 200 |
| 323 | CH | 2-Br | 4-F | H | 8,34 (d, 1 H) 7,48 (dd, 1 H) 7,32 (dd, 1H) 7,05 (m, 1H) 6,20 (d, 1 H) 5,72 (s, 1 H) | 271 |
| 324 | CH | 3-$OCH_3$ | 4-$OCH_3$ | H | 8,30 (d, 1H) 7,96 (m, 2H) 7,90 (m, 1H) 6,19 (d, 1 H) 5,33 (s, 1 H) 3,87 (s, 6H) | |
| 325 | CH | 3-$CF_3$ | H | H | | 243 |

(fortgesetzt)

| Beispiel Nr. | A | $R^4$ | $R^5$ | $R^6$ | 1 H-NMR ($CDCl_3$, 400 MHz) δ | MS(CI) $(M+H)^+$ |
|---|---|---|---|---|---|---|
| 326 | CH | 4-$OCH_3$ | H | H | 8,29 (d, 1 H) 7,33 (dd, 2H) 6.89 (dd, 2H) 6,18 (d, 1 H) 5,32 (s, 1 H) 3,80 (s, 3H) | |
| 326 | CH | 2-F | 4-$CF_3$ | H | 8,35 (d, 1H) 7,63 (t, 1 H) 7,44 (d, 1H) 7,35 (d, 1H) 6,28 (d, 1H) 5,64 (s, 1 H) | 261 |
| 327 | | CH 4-$OCHF_2$ | H | H | 8,30 (d, 1H) 7,21 (d, 2H) 6,80 (d, 2H) 6,18 (d, 1 H) 5,29 (s, 1H) | 259 |
| 328 | CH | 4-$CF_3$ | H | H | 8,32 (d, 1H) 7,62 (d, 2H) 7,56 (d, 2H) 6,20 (d, 1 H) 5,45 (s, 1 H) | 243 |
| 329 | CH | 2-F | H | H | 8,33 (d, 1H) 7,43 (m, 1H) 7.30 (m, 1 H) 7.15 (m 1 H) 7.07 (m, 1 H) 6.25 (d, 1 H) 5,60 (s, 1 H) | |
| 330 | CH | 3-F | H | H | 8,31 (d, 1 H) 7,30 (m, 1 H) 7,19 (m, 2H) 6,99 (m, 1 H) 6,21 (d, 1 H) 5,38 (s, 1 H) | 193 |
| 331 | CH | 2-$CH_3$ | H | H | 8,30 (d, 1H) 7,20 (m, 4H) 6,13 (d, 1H) 5,57 (s, 1 H) 2,36 (s, 3H) | 189 |
| 332 | CH | 3-F | 4-F | H | 8,32 (d, 1 H) 7,30 (m, 1 H) 7,15 (m, 2H) 6,20 (d, 1 H) 5,85 (s, 1 H) | 211 |
| 333 | CH | 2-Br | H | H | 8,33 (d, 1 H) 7,59 (d, 1 H, 7,48 (d, 1H) 7,32 (t, 1H) 7,17 (t, 1H) 6,20 (d, 1 H) 5,76 (s, 1 H) | 253 |
| 334 | CH | 4-Br | H | H | | 253 |
| 335 | CH | 4-F | H | H | 8,33 (d, 1 H) 7,24 (m, 1 H) 7,20 (m, 1 H) 7.06 (t, 2H) 6,27 (d, 1 H) 6.02 (s, 1 H) | 193 |

(fortgesetzt)

| Beispiel Nr. | A | $R^4$ | $R^5$ | $R^6$ | 1 H-NMR ($CDCl_3$, 400 MHz) δ | MS(CI) $(M+H)^+$ |
|---|---|---|---|---|---|---|
| 336 | CH | 4-$CH_3$ | H | H | 8,30 (d, 1 H) 7,30 (d, 2H) 7,25 (d, 2H) 6,18 (d, 1 H) 5,34 (s, 1 H) 2,34 (s, 3H) | 189 |
| 337 | CH | 4-$(H_3C)SO_2$ | H | H | 8,35 (d, 1 H) 7,96 (m, 2H) 7,68 (m, 2H) 6,23 (d, 1 H) 5,49 (s, 1 H) 3.05 (s, 3H) | 253 |
| 338 | CH | 3-F | 4-$CF_3$ | H | | 261 |
| 339 | CH | 2-F | 4-$OCH_3$ | H | 8,32 (d, 1 H) 7,30 (t, 1 H) 6,69 (dd, 1 H) 6,63 (dd, 1 H) 6,24 (d, 1 H) 5,52 (s, 1 H) 3,80 (s, 3H) | 223 |
| 340 | N | 4-Cl | H | H | 8,48 (m, 1 H) 8,35 (d, 1 H) 7,74 (dd, 1H) 7,31 (d, 1H) 6,23 (d, 1 H) 5,43 (s, 1 H) | 210 |
| 341 | N | 3-Cl | 4-Cl | H | | 245 |

B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.

b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.

e) Ein in Wasser dispergierbares Granulat wird erhalten indem man
75 Gew.-Teile einer Verbindung der Formel (I) und/oder deren Salze,
10 Gew.-Teile ligninsulfonsaures Calcium,
5 Gew.-Teile Natriumlaurylsulfat,
3 Gew.-Teile Polyvinylalkohol und
7 Gew.-Teile Kaolin
mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man
25 Gew.-Teile einer Verbindung der Formel (I) und/oder deren Salze,
5 Gew.-Teile 2,2'-dinaphthylmethan-6,6'-disulfonsaures Natrium
2 Gew.-Teile oleoylmethyltaurinsaures Natrium,
1 Gew.-Teil Polyvinylalkohol,
17 Gew.-Teile Calciumcarbonat und
50 Gew.-Teile Wasser
auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

C. Biologische Beispiele

1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen).

Tabelle 1

| Testorganismus | | | | | | | | | | | | | | |
|---|---|---|---|---|---|---|---|---|---|---|---|---|---|---|
| Beispiel Nr. | Dosierung | | ALOMY | AVEFA | ECHCG | LOLMU | SETVI | ABUTH | AMARE | MATIN | POLCO | STEME | VERPE | VIOTR |
| 2 | 1280 | g/ha | | | 90 | | 100 | 100 | 100 | 90 | 100 | 100 | 100 | 100 |
| 3 | 1280 | g/ha | 80 | | 80 | 90 | 90 | 100 | 100 | 100 | 100 | 100 | 100 | 100 |
| 7 | 1280 | g/ha | | | | | 90 | | 100 | 100 | | 100 | 100 | 90 |
| 19 | 1280 | g/ha | | | 90 | | 100 | | 100 | 90 | | 100 | 100 | 100 |
| 20 | 1280 | g/ha | | | | | 100 | 90 | 100 | 90 | 100 | 100 | 90 | 100 |
| 21 | 1280 | g/ha | | | | | | | 90 | 90 | | 100 | | 90 |
| 29 | 1280 | g/ha | | | | | 90 | 90 | | | | | 100 | |
| 30 | 1280 | g/ha | | 90 | | | | | 100 | | | | | 100 |
| 31 | 1280 | g/ha | | | | | | | 100 | 90 | | 100 | 70 | 50 |
| 32 | 1280 | g/ha | | | | | | 90 | | 90 | | | 90 | |
| 34 | 1280 | g/ha | | | | | 100 | 100 | 100 | 100 | 90 | 90 | 100 | 100 |
| 35 | 1280 | g/ha | 90 | | 80 | 90 | 100 | 100 | 100 | 100 | 90 | 100 | 100 | 100 |
| 38 | 1280 | g/ha | 80 | | | | 80 | | 100 | 80 | | 100 | 100 | 90 |
| 46 | 1280 | g/ha | | | | | | | | | | 100 | 100 | 100 |
| 47 | 1280 | g/ha | | | | | 80 | 80 | 100 | 100 | 80 | 100 | | 100 |
| 48 | 1280 | g/ha | | | 90 | | 100 | 100 | 100 | 90 | 90 | 100 | | 100 |
| 49 | 1280 | g/ha | | | | | 90 | | | 90 | | | 80 | |
| 50 | 1280 | g/ha | | | | | 90 | | 100 | 100 | 90 | | | 90 |
| 51 | 1280 | g/ha | | | 80 | | 90 | 90 | 100 | 100 | 90 | | | 90 |
| 52 | 1280 | g/ha | | | 100 | | 100 | 90 | 100 | 100 | 90 | 100 | | 100 |
| 53 | 1280 | g/ha | | | 90 | | 90 | 100 | 100 | | | 100 | | 100 |
| 54 | 1280 | g/ha | | | | | 90 | | 100 | 100 | | 100 | | 90 |
| 55 | 1280 | g/ha | | | | | | | 100 | 90 | | 100 | | 100 |
| 56 | 1280 | g/ha | | | | | 80 | | 100 | 100 | | 80 | 90 | 80 |
| 57 | 1280 | g/ha | | 80 | | | | 80 | 100 | 90 | | 100 | 100 | 90 |
| 59 | 1280 | g/ha | | | | | | 90 | 100 | 90 | | 100 | 100 | 100 |

| Testorganismus | | | | | | | | | | | | | | |
|---|---|---|---|---|---|---|---|---|---|---|---|---|---|---|
| Beispiel Nr. | Dosierung | | ALOMY | AVEFA | ECHCG | LOLMU | SETVI | ABUTH | AMARE | MATIN | POLCO | STEME | VERPE | VIOTR |
| 60 | 1280 | g/ha | | | | | 80 | | 100 | 100 | | 100 | | |
| 61 | 1280 | g/ha | | | 90 | | 90 | 90 | 100 | 100 | 90 | 100 | | 100 |
| 64 | 1280 | g/ha | | | 80 | | 80 | 100 | 100 | 90 | | | 100 | 90 |
| 67 | 1280 | g/ha | | | | | | | | 90 | | 100 | 100 | |
| 70 | 1280 | g/ha | | | | | 80 | | 80 | | | 100 | 100 | 90 |
| 72 | 1280 | g/ha | | | | | | | 90 | 90 | | 100 | 90 | |
| 73 | 1280 | g/ha | | | | | | | 100 | | | 90 | 90 | |
| 77 | 1280 | g/ha | | | | | | | 80 | | | 100 | 100 | |
| 78 | 1280 | g/ha | | | | | | | 80 | | | 90 | | 90 |
| 79 | 1280 | g/ha | | | | | | 90 | 100 | | | 100 | 100 | 80 |
| 87 | 1280 | g/ha | | | | | | | 100 | 80 | | 100 | 100 | 100 |
| 88 | 1280 | g/ha | | | | | | | 80 | | | 90 | 90 | 90 |
| 89 | 1280 | g/ha | | | | | | | 90 | | | 100 | 100 | 90 |
| 97 | 1280 | g/ha | | | | | | | | 90 | | 100 | 80 | 100 |
| 98 | 1280 | g/ha | | | | | | | 80 | | | 100 | 90 | 80 |
| 99 | 1280 | g/ha | | | | | | | 90 | | | 90 | 100 | 100 |
| 100 | 1280 | g/ha | | | | | | | 100 | | | 90 | 100 | 100 |
| 102 | 1280 | g/ha | | | | | | 80 | | 80 | | | 90 | |
| 106 | 1280 | g/ha | | | | | | | 100 | | | 90 | 90 | 100 |
| 136 | 1280 | g/ha | | | | | | | 100 | | | 100 | 80 | |
| 142 | 1280 | g/ha | | | | | | | 100 | 90 | | 100 | 80 | 90 |
| 144 | 1280 | g/ha | | | | | | | 100 | | | 100 | 100 | |
| 145 | 1280 | g/ha | | | 90 | | 100 | 90 | 100 | 100 | | 100 | | 100 |
| 146 | 1280 | g/ha | 80 | | 90 | 80 | 90 | 90 | 100 | 90 | 90 | 90 | 100 | 100 |
| 149 | 1280 | g/ha | 90 | | 90 | 90 | 100 | 100 | 100 | 100 | 90 | 100 | 100 | 100 |
| 150 | 1280 | g/ha | | | | | 90 | 90 | 90 | 90 | | 100 | 90 | 90 |

| Testorganismus | | | | | | | | | | | | | | |
|---|---|---|---|---|---|---|---|---|---|---|---|---|---|---|
| Beispiel Nr. | Dosierung | | ALOMY | AVEFA | ECHCG | LOLMU | SETVI | ABUTH | AMARE | MATIN | POLCO | STEME | VERPE | VIOTR |
| 152 | 1280 | g/ha | 80 | | 90 | 90 | 100 | 100 | 100 | 100 | 100 | 90 | 100 | 100 |
| 160 | 1280 | g/ha | | | | | 90 | 100 | 100 | 100 | 100 | 100 | 100 | 100 |
| 161 | 1280 | g/ha | 90 | 80 | 80 | 90 | 100 | 100 | 100 | 100 | 100 | 100 | 100 | 100 |
| 162 | 1280 | g/ha | 90 | | 80 | 80 | 80 | 100 | 100 | 100 | 100 | 100 | 100 | 100 |
| 164 | 1280 | g/ha | | | | | | | 100 | | 100 | 90 | 100 | 100 |
| 174 | 1280 | g/ha | 60 | | | | | 100 | 100 | 80 | | 100 | 100 | |
| 201 | 1280 | g/ha | | | | | | | | | | 100 | 100 | 100 |
| 261 | 1280 | g/ha | | | | | 90 | | 100 | 100 | | 100 | 100 | |
| 262 | 1280 | g/ha | | | | | | | 100 | 90 | | 100 | 100 | 100 |
| 265 | 1280 | g/ha | | | | | | 90 | 100 | 100 | | 100 | 100 | 100 |

Wie die Ergebnisse zeigen, weisen erfindungsgemäße Verbindungen eine gute herbizide Vorauflaufwirksamkeit gegen ein breites Spektrum von Ungräsem und Unkräutern auf. Beispielsweise haben die Verbindungen Nr. 3, 35, 149, 152, 161, 162 und andere Verbindungen aus Tabelle 1 sehr gute herbizide Wirkung gegen Schadpflanzen wie Alopecurus myosuroides, Avena fatua, Echinochloa crus-galli, Lolium multiflorum, Setaria viridis, Abutilon theophrasti, Amaranthus retroflexus, Matricaria inodora, Polygonum convolvolus, Stellaria media, Veronica persica und Viola tricolor im Vorauflaufverfahren bei einer Aufwandmenge von 1.28 kg und weniger Aktivsubstanz pro Hektar.

Gleichzeitig lassen erfindungsgemäße Verbindungen zweikeimblättrige Kulturen wie Soja, Baumwolle, Raps, Zuckerrüben oder Kartoffeln im Nachauflaufverfahren selbst bei hohen Wirkstoffdosierungen ungeschädigt. Einige Substanzen schonen darüber hinaus auch Gramineen-Kulturen wie Gerste, Weizen, Roggen, Hirse, Mais oder Reis. Die erfindungsgemäßen Verbindungen zeigen teilweise eine hohe Selektivität und eignen sich deshalb im Vorauflaufverfahren zur Bekämpfung von unerwünschtem Pflanzenwuchs in landwirtschaftlichen Kulturen.

2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 l/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Tabelle 2

| Testorganismus | | | | | | | | | | | | |
|---|---|---|---|---|---|---|---|---|---|---|---|---|
| Beispiel Nr. | Dosierung | | ECHCG | SETVI | ABUTH | AMARE | MATIN | PHBPU | POLCO | STEME | VERPE | VIOTR |
| 2 | 1280 | g/ha | 90 | 90 | 100 | 100 | 90 | 80 | 90 | 90 | 90 | 100 |
| 3 | 1280 | g/ha | | | 100 | 100 | 100 | 80 | 80 | 100 | 100 | 80 |
| 7 | 1280 | g/ha | | | | 90 | 100 | 80 | | 100 | 100 | 100 |
| 9 | 1280 | g/ha | 90 | | 90 | 90 | 90 | 90 | | 100 | 100 | 90 |
| 14 | 1280 | g/ha | | | 80 | | | 90 | | 100 | 80 | 90 |
| 15 | 1280 | g/ha | 100 | | | | 100 | | | | 100 | |
| 16 | 1280 | g/ha | 100 | | 80 | | 100 | | | | 100 | |
| 19 | 1280 | g/ha | 80 | 90 | 100 | 100 | | 90 | | 100 | 90 | 100 |
| 20 | 1280 | g/ha | 100 | 100 | 100 | 100 | 90 | 100 | 100 | 100 | 100 | 100 |

| Testorganismus | | | | | | | | | | | | |
|---|---|---|---|---|---|---|---|---|---|---|---|---|
| Beispiel Nr. | Dosierung | | ECHCG | SETVI | ABUTH | AMARE | MATIN | PHBPU | POLCO | STEME | VERPE | VIOTR |
| 21 | 1280 | g/ha | | | 80 | 100 | 90 | 100 | 80 | 100 | 100 | 90 |
| 22 | 1280 | g/ha | | | 80 | 90 | 100 | 90 | | 100 | 100 | 90 |
| 26 | 1280 | g/ha | 80 | | 90 | 80 | 80 | | | | 90 | 80 |
| 29 | 1280 | g/ha | | | 100 | 90 | 90 | | | | 100 | |
| 30 | 1280 | g/ha | | | | 80 | | | | 100 | 90 | |
| 31 | 1280 | g/ha | | | 100 | 100 | 100 | | 80 | 100 | 100 | 80 |
| 34 | 1280 | g/ha | 90 | 80 | 100 | 100 | 100 | 90 | | 100 | 100 | 90 |
| 35 | 1280 | g/ha | 80 | | 100 | 100 | 100 | 80 | 100 | 100 | 100 | 100 |
| 38 | 1280 | g/ha | | | 90 | 100 | 90 | 80 | | 100 | 100 | 80 |
| 46 | 1280 | g/ha | | | | 100 | | | | 100 | 100 | 100 |
| 47 | 1280 | g/ha | | 80 | 100 | 90 | | 80 | 80 | 100 | 90 | 90 |
| 48 | 1280 | g/ha | 80 | 90 | 100 | 100 | 80 | 90 | 90 | 100 | 90 | 90 |
| 49 | 1280 | g/ha | | 80 | 90 | 80 | | | | | 80 | |
| 50 | 1280 | g/ha | 80 | 80 | 100 | 100 | 90 | 90 | 80 | | 80 | 90 |
| 51 | 1280 | g/ha | 80 | 80 | 100 | 100 | 90 | 90 | 80 | 80 | 90 | 80 |
| 52 | 1280 | g/ha | 100 | 100 | 100 | 100 | 100 | 90 | 90 | 100 | 100 | 100 |
| 53 | 1280 | g/ha | 100 | 100 | 100 | 100 | 100 | 90 | 100 | 100 | 100 | 100 |
| 54 | 1280 | g/ha | | 90 | 90 | 100 | 90 | 80 | | 100 | 90 | 90 |
| 55 | 1280 | g/ha | 80 | | 90 | 100 | | 80 | | 100 | 90 | 90 |
| 56 | 1280 | g/ha | | | 100 | 100 | 80 | | 100 | 80 | | |
| 57 | 1280 | g/ha | 50 | 70 | 80 | 100 | 90 | 80 | | 100 | 100 | 100 |
| 59 | 1280 | g/ha | | | | 100 | 80 | | | 100 | 100 | 80 |
| 60 | 1280 | g/ha | | 90 | 90 | 100 | 90 | 90 | | 100 | 80 | 90 |
| 61 | 1280 | g/ha | 100 | 80 | 100 | 100 | 90 | 90 | 80 | 100 | 90 | 90 |
| 64 | 1280 | g/ha | | | 90 | 100 | | | | 90 | 90 | |
| 67 | 1280 | g/ha | | | | 100 | | | | 100 | 100 | 90 |
| 70 | 1280 | g/ha | 80 | | 100 | 100 | 80 | 80 | | 80 | 100 | 100 |

| Testorganismus | | | | | | | | | | | | |
|---|---|---|---|---|---|---|---|---|---|---|---|---|
| Beispiel Nr. | Dosierung | | ECHCG | SETVI | ABUTH | AMARE | MATIN | PHBPU | POLCO | STEME | VERPE | VIOTR |
| 71 | 1280 | g/ha | | | | 80 | 100 | | | | 100 | 90 |
| 72 | 1280 | g/ha | | | | 80 | | | | 100 | 80 | |
| 73 | 1280 | g/ha | | | 80 | 80 | 80 | | | 100 | 100 | 60 |
| 77 | 1280 | g/ha | | | 80 | | | | | 100 | 100 | |
| 78 | 1280 | g/ha | | | 100 | 80 | | | | 90 | | |
| 79 | 1280 | g/ha | | | 90 | 100 | 80 | 80 | | 100 | 100 | 90 |
| 87 | 1280 | g/ha | 80 | 80 | 90 | 100 | 100 | 80 | 90 | 100 | 100 | 100 |
| 88 | 1280 | g/ha | | | 80 | 90 | 90 | 80 | | 100 | 100 | 90 |
| 89 | 1280 | g/ha | | | 100 | 100 | 80 | | 100 | 100 | 100 | |
| 92 | 1280 | g/ha | | | 90 | 100 | | | | 100 | 90 | 100 |
| 97 | 1280 | g/ha | | | 80 | 80 | | | | 100 | | 80 |
| 98 | 1280 | g/ha | | | | 90 | | | | 100 | 80 | 80 |
| 100 | 1280 | g/ha | | | 90 | 90 | | | | 90 | 90 | 80 |
| 102 | 1280 | g/ha | | | 100 | 100 | | | | | 90 | 80 |
| 103 | 1280 | g/ha | | | 90 | 100 | 90 | | | | 100 | |
| 136 | 1280 | g/ha | | | 80 | 100 | | | | 100 | 90 | |
| 142 | 1280 | g/ha | | | | 100 | | | | 90 | 80 | |
| 143 | 1280 | g/ha | | | | 90 | | | | 90 | 80 | |
| 144 | 1280 | g/ha | | | 80 | 100 | 80 | | | 100 | 80 | 90 |
| 145 | 1280 | g/ha | 90 | 100 | 100 | 100 | 90 | 90 | 80 | 100 | 100 | 100 |
| 146 | 1280 | g/ha | 90 | 90 | 100 | 100 | 80 | 80 | 80 | 80 | 100 | 100 |
| 149 | 1280 | g/ha | | 80 | 100 | 100 | 90 | 100 | 100 | 100 | 100 | 100 |
| 150 | 1280 | g/ha | | | 100 | 100 | 90 | 90 | | 90 | 100 | 80 |
| 152 | 1280 | g/ha | | 80 | 100 | 100 | 90 | 90 | | 90 | 100 | 100 |
| 160 | 1280 | g/ha | | 80 | 100 | 100 | 90 | 80 | 90 | 80 | 100 | 80 |
| 161 | 1280 | g/ha | | 80 | 100 | 100 | 100 | 90 | 90 | 100 | 100 | 100 |
| 162 | 1280 | g/ha | 80 | 90 | 100 | 100 | 80 | 80 | 90 | 100 | 100 | 80 |

| Testorganismus | | | | | | | | | | | | |
|---|---|---|---|---|---|---|---|---|---|---|---|---|
| Beispiel Nr. | Dosierung | | ECHCG | SETVI | ABUTH | AMARE | MATIN | PHBPU | POLCO | STEME | VERPE | VIOTR |
| 164 | 1280 | g/ha | | | 90 | 100 | | 80 | | | 80 | 80 |
| 174 | 1280 | g/ha | | | 100 | 100 | 90 | 80 | | 100 | 100 | 80 |
| 209 | 1280 | g/ha | | | 80 | | | | | 100 | | 80 |
| 212 | 1280 | g/ha | | | 80 | 80 | | | | 100 | 80 | |
| 261 | 1280 | g/ha | | | 80 | 90 | 100 | 80 | | 100 | 100 | 100 |
| 265 | 1280 | g/ha | | | 100 | 100 | 80 | | | 100 | 100 | 100 |
| 266 | 1280 | g/ha | | | | | | | | 100 | 90 | 80 |
| 295 | 1280 | g/ha | | | 80 | | 80 | | | 100 | | 90 |

Wie die Ergebnisse zeigen, weisen erfindungsgemäße Verbindungen eine gute herbizide Nachauflaufwirksamkeit gegen ein breites Spektrum von Ungräsern und Unkräutern auf. Beispielsweise haben die Verbindungen Nr. 2, 20, 52, 53, 61,145, 146,162 und andere Verbindungen aus Tabelle 2 sehr gute herbizide Wirkung gegen Schadpflanzen wie Echinochloa crus-galli, Setaria viridis, Abutilon theophrasti, Amaranthus retroflexus, Matricaria inodora, Pharbitis purpurea, Polygonum convolvolus, Stellaria media, Veronica persica und Viola tricolor im Nachauflaufverfahren bei einer Aufwandmenge von 1.28 kg und weniger Aktivsubstanz pro Hektar.

Gleichzeitig lassen erfindungsgemäße Verbindungen zweikeimblättrige Kulturen wie Soja, Baumwolle, Raps, Zuckerrüben oder Kartoffeln im Nachauflaufverfahren selbst bei hohen Wirkstoffdosierungen ungeschädigt.

Einige Substanzen schonen darüber hinaus auch Gramineen-Kulturen wie Gerste, Weizen, Roggen, Hirse, Mais oder Reis. Die erfindungsgemäßen Verbindungen zeigen teilweise eine hohe Selektivität und eignen sich deshalb im Nachauflaufverfahren zur Bekämpfung von unerwünschtem Pflanzenwuchs in landwirtschaftlichen Kulturen.

## Patentansprüche

1. Verbindungen der allgmeinen Formel (I) und deren *N*-Oxide oder Salze,

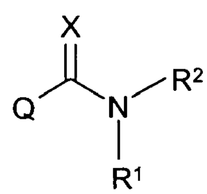

(I)

worin

Q ein aromatischer Heterocyclus, mit der Ausnahme von 4-Pyridyl,
X ein Sauerstoff- oder Schwefelatom,
$R^1$ Wasserstoff, ($C_1$-$C_4$)-Alkyl, ($C_3$-$C_4$)-Cycloalkyl oder

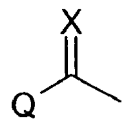

$R^2$ ein Fragment der allgemeinen Formel (II)

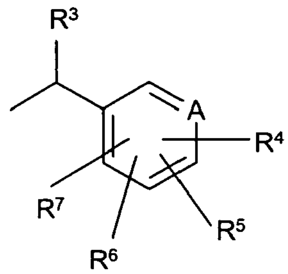

(II)

in dem A = CH oder N ist, und

$R^3$ ein fünfgliedriger aromatischer Ring, der ausgewählt ist aus der Gruppe bestehend aus 1,2-Oxazol, 1,3-Oxazol, 1,2-Thiazol, 1,3-Thiazol, Pyrazol, Imidazol, 1,2,4-Triazol und 1,2,4-Oxadiazol,

$R^4$, $R^5$, $R^6$ und $R^7$ unabhängig voneinander Wasserstoff, Halogen, Cyano, Nitro, Alkyl, Cycloalkyl, Alkenyl, Cycloakenyl, Alkinyl, Alkoxy, Cycloalkoxy, Alkenyloxy, Cycloalkenyloxy, Alkinyloxy, Alkylthio, Cycloalkylthio, Alkenylthio, Cycloalkenylthio, Alkinylthio, Alkylsulfinyl, Alkenylsulfinyl, Alkinylsulfinyl, Alkylsulfonyl, Alkenylsulfonyl, Alkinylsulfonyl, $NR^8R^9$, $R^8CONR^9$, $R^8R^9NSO_2$ oder gegebenenfalls substituiertes Aryl, sowie

$R^8$ und $R^9$ unabhängig voneinander Wasserstoff, Alkyl, Cycloalkyl, Alkenyl oder Alkinyl

bedeuten.

2. Verbindungen und deren *N*-Oxide oder Salze nach Anspruch 1, **dadurch gekennzeichnet, dass**

$R^3$ ein fünfgliedriger aromatischer Ring bedeutet, der ausgewählt ist aus der Gruppe bestehend aus 1,2-Oxazol, 1,3-Oxazol, 1,2-Thiazol, 1,3-Thiazol und Pyrazol.

3. Verbindungen und deren *N*-Oxide oder Salze nach einem der Ansprüche 1 und 2, **dadurch gekennzeichnet, dass**

$R^4$ $R^5$, $R^6$ und $R^7$ unabhängig voneinander Wasserstoff, Halogen, Cyano, Nitro, $(C_1\text{-}C_6)$-Alkyl, wobei der Alkylrest unsubstituiert ist oder durch einen oder mehrere Substituenten, ausgewählt aus der aus Halogen, Cyano, $(C_1\text{-}C_4)$- Alkoxy oder $(C_1\text{-}C_4)$-Alkylthio bestehenden Gruppe, substituiert ist, $(C_3\text{-}C_7)$- Cycloalkyl, wobei der Cycloalkylrest unsubstituiert ist oder durch einen oder mehrere Substituenten, ausgewählt aus der aus Halogen, Cyano, $(C_1\text{-}C_4)$- Alkyl, $(C_1\text{-}C_4)$-Alkoxy oder $(C_1\text{-}C_4)$-Alkylthio bestehenden Gruppe, substituiert ist, $(C_2\text{-}C_6)$-Alkenyl, wobei der Alkenylrest unsubstituiert ist oder durch einen oder mehrere Substituenten, ausgewählt aus der aus Halogen, Cyano, $(C_1\text{-}\ C_4)$-Alkoxy oder $(C_1\text{-}C_4)$-Alkylthio bestehenden Gruppe, substituiert ist, $(C_3\text{-}C_7)$-Cycloalkenyl, wobei der Cycloalkenylrest unsubstituiert ist oder durch einen oder mehrere Substituenten, ausgewählt aus der aus Halogen, Cyano, $(C_1\text{-}C_4)$-Alkyl, $(C_1\text{-}C_4)$-Alkoxy oder $(C_1\text{-}C_4)$-Alkylthio bestehenden Gruppe, substituiert ist, $(C_2\text{-}C_6)$-Alkinyl, wobei der Alkinylrest unsubstituiert ist oder durch einen oder mehrere Substituenten, ausgewählt aus der aus Halogen, Cyano, $(C_1\text{-}C_4)$-Alkoxy oder $(C_1\text{-}C_4)$-Alkylthio bestehenden Gruppe, substituiert ist, $(C_1\text{-}C_6)$-Alkoxy, wobei der Alkoxyrest unsubstituiert ist oder durch einen oder mehrere Substituenten, ausgewählt aus der aus Halogen, Cyano, $(C_1\text{-}C_4)$-Alkoxy oder $(C_1\text{-}C_4)$-Alkylthio bestehenden Gruppe, substituiert ist, $(C_3\text{-}C_7)$-Cycloalkoxy, wobei der Cycloalkoxyrest unsubstituiert ist oder durch einen oder mehrere Substituenten, ausgewählt aus der aus Halogen, Cyano, $(C_1\text{-}C_4)$-Alkyl, $(C_1\text{-}C_4)$-Alkoxy oder $(C_1\text{-}C_4)$-Alkylthio bestehenden Gruppe, substituiert ist, $(C_2\text{-}C_6)$-Alkenyloxy, wobei der Alkenyloxyrest unsubstituiert ist oder durch einen oder mehrere Substituenten, ausgewählt aus der aus Halogen, Cyano, $(C_1\text{-}C_4)$-Alkoxy oder $(C_1\text{-}C_4)$- Alkylthio bestehenden Gruppe, substituiert ist, $(C_3\text{-}C_7)$-Cycloalkenyloxy, wobei der Cycloalkenyloxyrest unsubstituiert ist oder durch einen oder mehrere Substituenten, ausgewählt aus der aus Halogen, Cyano, $(C_1\text{-}C_4)$-Alkyl, $(C_1\text{-}\ C_4)$-Alkoxy oder $(C_1\text{-}C_4)$-Alkylthio bestehenden Gruppe, substituiert ist, $(C_2\text{-}\ C_6)$-Alkinyloxy, wobei der Alkinyloxyrest unsubstituiert ist oder durch einen oder mehrere Substituenten, ausgewählt aus der

aus Halogen, Cyano, ($C_1$- $C_4$)-Alkoxy oder ($C_1$-$C_4$)-Alkylthio bestehenden Gruppe, substituiert ist, ($C_1$- $C_6$)-Alkylthio, wobei der Alkylthiorest unsubstituiert ist oder durch einen oder mehrere Substituenten, ausgewählt aus der aus Halogen, Cyano, ($C_1$-$C_4$)- Alkoxy oder ($C_1$-$C_4$)-Alkylthio bestehenden Gruppe, substituiert ist, ($C_3$-$C_7$)-Cycloalkylthio, wobei der Cycloalkylthiorest unsubstituiert ist oder durch einen oder mehrere Substituenten, ausgewählt aus der aus Halogen, Cyano, ($C_1$- $C_4$)-Alkyl, ($C_1$-$C_4$)-Alkoxy oder ($C_1$-$C_4$)-Alkylthio bestehenden Gruppe, substituiert ist, ($C_2$-$C_6$)-Alkenylthio, wobei der Alkenylthiorest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthlo bestehenden Gruppe, substituiert ist, ($C_3$-$C_7$)-Cycloalkenylthio, wobei der Cycloalkenylthiorest unsubstituiert ist oder durch einen oder mehrere Substituenten, ausgewählt aus der aus Halogen, Cyano, ($C_1$-$C_4$)-Alkyl, ($C_1$-$C_4$)-Alkoxy oder ($C_1$-$C_4$)- Alkylthio bestehenden Gruppe, substituiert ist, ($C_2$-$C_6$)-Alkinylthlo, wobei der Alkinylthiorest unsubstituiert ist oder durch einen oder mehrere Substituenten, ausgewählt aus der aus Halogen, Cyano, ($C_1$-$C_4$)-Alkoxy oder ($C_1$-$C_4$)- Alkylthio bestehenden Gruppe, substituiert ist, ($C_1$-$C_6$)-Alkylsulfinyl, wobei der Alkylsulfinylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthlo bestehenden Gruppe, substituiert ist, ($C_2$-$C_6$)-Alkenylsulfinyl, wobei der Alkenylsulfinylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_2$-$C_6$)-Alkinylsulfinyl, wobei der Alkinylsulfinylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_1$-$C_6$)-Alkylsulfonyl wobei der Alkylsulfonylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_2$-$C_6$)-Alkenylsulfonyl wobei der Alkenylsulfonylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_2$-$C_6$)-Alkinylsulfonyl wobei der Alkinylsulfonylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, $NR^8R^9$, $R^8CONR^9$, $R^8R^9NSO_2$ oder gegebenenfalls substituiertes Aryl

bedeuten.

**4.** Verbindungen und deren *N*-Oxide oder Salze nach einem der Ansprüche 1 bis 3, **dadurch gekennzeichnet, dass**

$R^4$, $R^5$,$R^6$ und $R^7$ unabhängig voneinander Wasserstoff, Halogen, Cyano, Nitro, ($C_1$- $C_5$)-Alkyl, Dihalogenalkyl, Trihalogenalkyl, ($C_1$-$C_3$)-Alkoxy-($C_1$-$C_5$)-alkyl, ($C_1$- $C_5$)-Alkoxy, Dihalogenalkoxy,Trihalogenalkoxy, ($C_1$-$C_3$)-Alkoxy-($C_1$-$C_5$)-alkoxy, ($C_1$-$C_3$)-Alkenyloxy, ($C_1$-$C_3$)-Alkinyloxy, ($C_1$-$C_5$)-Alkylthio, Dihalogenalkylthio,Trihalogenalkylthio, ($C_1$-$C_5$)-Alkylsulfinyl, ($C_1$-$C_5$)- Alkylsulfonyl oder ($C_1$-$C_5$)-Dialkylamino

bedeuten.

**5.** Verbindungen und deren *N*-Oxide oder Salze nach einem der Ansprüche 1 bis 4, **dadurch gekennzeichnet, dass**

$R^3$ 1,2-Oxazol, und
$R^4$, $R^5$, $R^6$ und $R^7$ unabhängig voneinander Wasserstoff, Fluor, Chlor, Brom, Cyano, Nitro, ($C_1$-$C_4$)-Alkyl, Difluormethyl und Trifluormethyl, ($C_1$-$C_4$)-Alkoxy, Difluormethoxy und Trifluormethoxy, ($C_1$-$C_4$)-Alkylthio, Difluormethylthio, Trifluormethylthio, ($C_1$-$C_2$)-Sulfinyl, ($C_1$-$C_2$)-Sulfonyl und ($C_1$-$C_2$)-Dialkylamino

bedeuten.

**6.** Verbindungen und deren *N*-Oxide oder Salze nach einem der Ansprüche 1 bis 5, **dadurch gekennzeichnet, dass**

$R^8$ und $R^9$ unabhängig voneinander Wasserstoff, ($C_1$-$C_6$)-Alkyl, wobei der Alkylrest unsubstituiert ist oder durch einen oder mehrere Substituenten, ausgewählt aus der aus Halogen, Cyano, ($C_1$-$C_4$)-Alkoxy oder ($C_1$-$C_4$)-Alkylthio bestehenden Gruppe, substituiert ist, ($C_3$-$C_7$)-Cycloalkyl, wobei der Cycloalkylrest unsubstituiert ist oder durch einen oder mehrere Substituenten, ausgewählt aus der aus Halogen, Cyano, ($C_1$-$C_4$)-Alkyl, ($C_1$-$C_4$)-Alkoxy oder ($C_1$-$C_4$)-Alkylthlo bestehenden Gruppe, substituiert ist, ($C_2$-$C_6$)-Alkenyl, wobei der Alkenylrest unsubstituiert ist oder durch einen oder mehrere Substituenten, ausgewählt aus der aus Halogen, Cyano, ($C_1$-$C_4$)-Alkoxy oder ($C_1$-$C_4$)- Alkylthio bestehenden Gruppe, substituiert ist, ($C_2$-$C_6$)-Alkinyl, wobei der Alkinylrest unsubstituiert ist oder durch einen oder mehrere Substituenten, ausgewählt aus der aus Halogen, Cyano, ($C_1$-$C_4$)-Alkoxy oder ($C_1$-$C_4$)- Alkylthio bestehenden Gruppe, substituiert ist,

bedeuten.

7. Verbindungen und deren *N*-Oxide oder Salze nach einem der Ansprüche 1 bis 6, **dadurch gekennzeichnet, dass** Q ein heterocyclischer, aromatischer Rest ist, der ausgewählt ist aus der aus Q1 bis Q12 bestehenden Gruppe

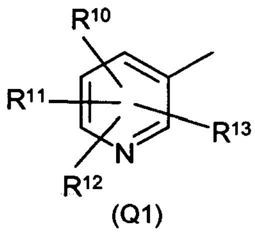
(Q1)

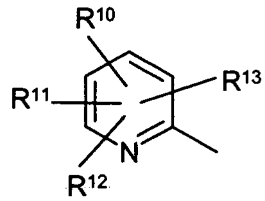
(Q2)

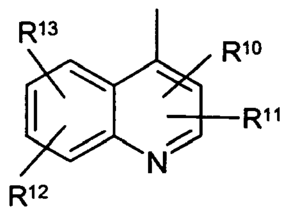
(Q3)

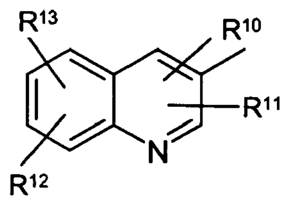
(Q4)

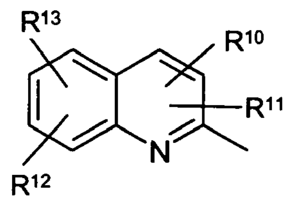
(Q5)

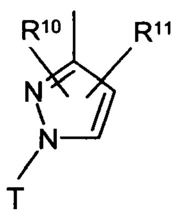
(Q6)

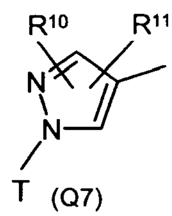
(Q7)

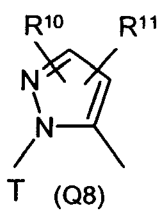
(Q8)

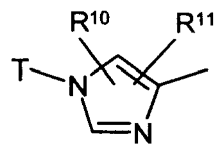
(Q9)

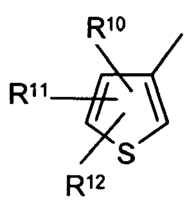
(Q10)

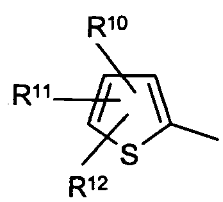
(Q11)

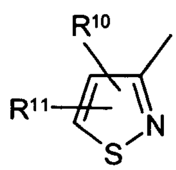
(Q12)

und unsubstituiert oder durch einen oder mehrere Substituenten, ausgewählt aus der aus $R^{10}$, $R^{11}$,$R^{12}$oder $R^{13}$ bestehenden Gruppe, substituiert ist, wobei

$R^{10}$, $R^{11}$,$R^{12}$ oder $R^{13}$ unabhängig voneinander die in einem der Ansprüche 1, 3, 4 oder 5 angegebene Bedeutung von $R^4$, $R^5$, $R^6$ und $R^7$ haben, und
T ($C_1$-$C_6$)-Alkyl, Benzyl oder Phenyl

bedeutet.

8. Verbindung der allgemeinen Formel (IVa) und deren N-Oxide oder Salze

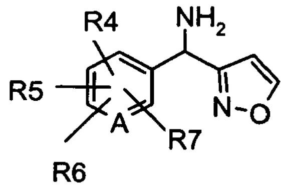

(IVa)

worin

A = CH oder N ist, und
$R^4$, $R^5$, $R^6$ und $R^7$ die in einem der Ansprüche 1, 3, 4 oder 5 angegebene Bedeutung haben.

**9.** Verfahren zur Herstellung der Verbindungen der Formel (I), deren *N*-Oxide oder deren Salze, nach einem der Ansprüche 1 bis 7, worin
Q, X, $R^1$, $R^2$, $R^3$, $R^4$, $R^5$, $R^6$ und $R^7$ die in einem der Ansprüche 1, 2, 3, 4 oder 5 angegebene Bedeutung haben, und worin
$R^8$, $R^9$, $R^{10}$, $R^{11}$, $R^{12}$, $R^{13}$ und T die in einem der Ansprüche 6, 7 oder 8 angegebene Bedeutung haben, **dadurch gekennzeichnet, dass**
eine Verbindung der Formel (III)

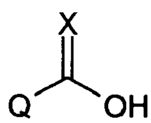

(III)

,

worin
X die in Anspruch 1 angegebene Bedeutung hat, mit einem Amin der Formel (IV)

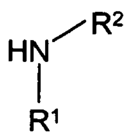

(IV)

,

worin

$R^1$ Wasserstoff, $(C_1\text{-}C_4)$-Alkyl, $(C_3\text{-}C_4)$-Cycloalkyl oder

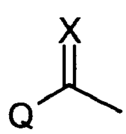

bedeutet, und

$R^2$ ein Fragment der allgemeinen Formel (II)

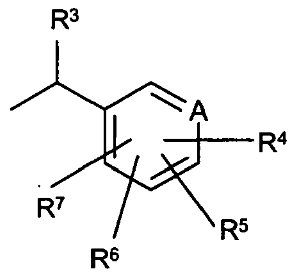

(II)

bedeutet, in dem A = CH oder N ist, und
$R^3$, $R^4$, $R^5$, $R^6$ und $R^7$ die in einem der Ansprüche 1, 2, 3, 4 oder 5 angegebene Bedeutung haben,
zu einer Verbindung der Formel (I)

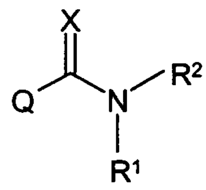

(I)

umgesetzt wird.

**10.** Verfahren zur Herstellung von Verbindungen der allgemeinen Formel (IVa) und deren N-Oxide oder Salze nach Anspruch 8, **dadurch gekennzeichnet, dass**

a) eine Verbindung der Formel (VI)

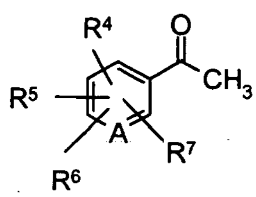

(VI)

,

worin

A = CH oder N ist, und
$R^4$, $R^5$, $R^6$ und $R^7$ die in einem der Ansprüche 1, 2, 3, 4 oder 5 angegebene Bedeutung haben,

in Gegenwart einer Säure mit einem Alkylnitrit zu einer Verbindung der Formel (VII)

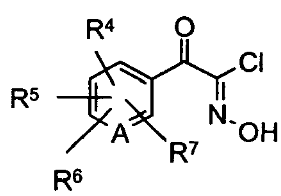

(VII)

umgesetzt werden und anschließend in Gegenwart einer Base mit Acetylen zu einer Verbindung der Formel (VIII)

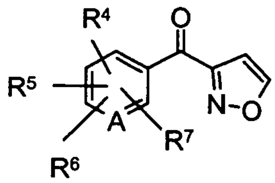

(VIII)

umgesetzt wird, und

b) eine Verbindung der Formel (VIII) in Gegenwart von Hydroxylamin oder einem seiner Salze zu einer Verbindung der Formel (IX)

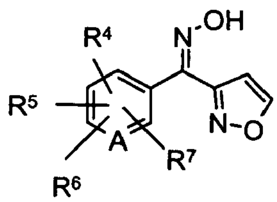

(IX)

umgesetzt wird, und anschließend

c) eine Verbindung der Formel (IX) in Gegenwart eines Diarylchlorphosphans und einer organischen Base zu einer Verbindung der Formel (X)

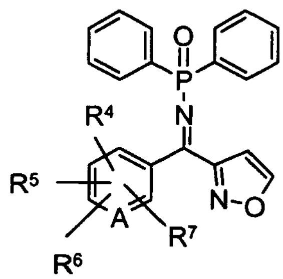

(X)

umgesetzt wird, und abschließend

d) eine Verbindung der Formel (X) in Gegenwart von Natriumborhydrid chemoselektiv, d.h. ohne Öffnung des Isoxazolrings, reduziert wird und nachfolgend durch Kochen in saurer Lösung mit anschließender Neutralisation zu einer Verbindung der Formel (IV a)

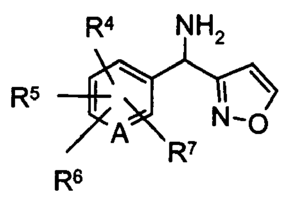

(IV a)

umgesetzt wird.

**11.** Verfahren zur Herstellung von Verbindungen der allgemeinen Formel (IVa) und deren N-Oxide oder Salze nach Anspruch 8, **dadurch gekennzeichnet, dass**

a) eine Verbindung der Formel (VIII) in Gegenwart von Hydroxylamin oder einem seiner Salze zu einer Verbindung der Formel (IX)

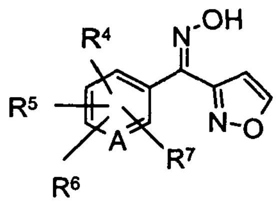

(IX)

,

worin

A = CH oder N ist, und
$R^4$, $R^5$, $R^6$ und $R^7$ die in einem der Ansprüche 1, 2, 3, 4 oder 5 angegebene Bedeutung haben,

umgesetzt wird, und anschließend
b) eine Verbindung der Formel (IX) in Gegenwart eines Diarylchlorphosphans und einer organischen Base zu einer Verbindung der Formel (X)

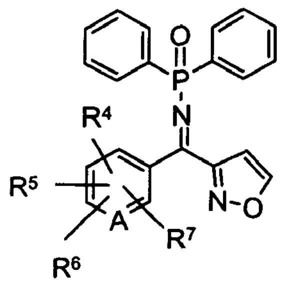

(X)

umgesetzt wird, und abschließend
c) eine Verbindung der Formel (X) in Gegenwart von Natriumborhydrid chemoselektiv, d.h. ohne Öffnung des Isoxazolrings reduziert, wird und nachfolgend durch Kochen in saurer Lösung mit anschließender Neutralisation zu einer Verbindung der Formel (IV a)

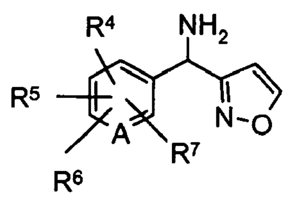

(IV a)

umgesetzt wird.

**12.** Verfahren zur Herstellung von Verbindungen der allgemeinen Formel (IVa) und deren N-Oxide oder Salze nach Anspruch 8, **dadurch gekennzeichnet, dass**

a) eine Verbindung der Formel (IX) in Gegenwart eines Diarylchlorphosphans und zu einer Verbindung der Formel (X)

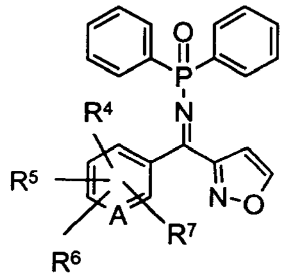

(X)

,

worin

A = CH oder N ist, und
$R^4$, $R^5$, $R^6$ und $R^7$ die in einem der Ansprüche 1, 2, 3, 4 oder 5 angegebene Bedeutung haben,

umgesetzt wird, und anschließend
b) eine Verbindung der Formel (X) in Gegenwart von Natriumborhydrid chemoselektiv, d.h. ohne Öffnung des Isoxazolrings, reduziert wird und nachfolgend durch Kochen in saurer Lösung mit anschließender Neutralisation zu einer Verbindung der Formel (IV a)

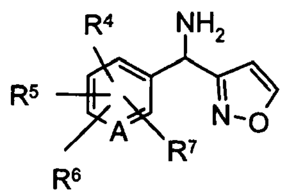

(IV a)

umgesetzt wird.

**13.** Verfahren zur Herstellung von Verbindungen der allgemeinen Formel (IVa) und deren N-Oxide oder Salze nach Anspruch 8, **dadurch gekennzeichnet, dass** eine Verbindung der Formel (X)

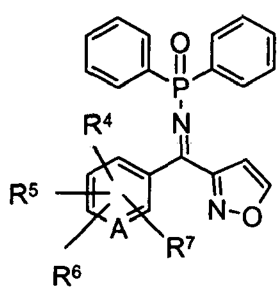

(X)

,

worin

A = CH oder N ist, und
$R^4$, $R^5$, $R^6$ und $R^7$ die in einem der Ansprüche 1, 2, 3, 4 oder 5 angegebene Bedeutung haben,

in Gegenwart von Natriumborhydrid chemoselektiv, d.h. ohne Öffnung des Isoxazolrings, reduziert wird und nachfolgend durch Kochen in saurer Lösung mit anschließender Neutralisation zu einer Verbindung der Formel (IV a)

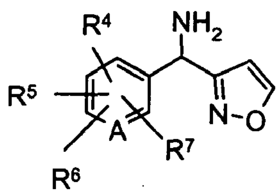

(IV a)

umgesetzt wird.

14. Herbizides oder pflanzenwachstumsregulierendes Mittel, **dadurch gekennzeichnet, dass** es eine wirksame Menge einer oder mehrerer der Verbindungen der Formel (I) oder eines ihrer *N*-Oxide oder eines ihrer Salze nach einem der Ansprüche 1 bis 7 und mindestens eines der im Pflanzenschutz üblichen Formulierungshilfsmittels enthält.

15. Verfahren zur Bekämpfung von Schadpflanzen und zur Wachstumsregulierung von Pflanzen, **dadurch gekennzeichnet, dass** man eine wirksame Menge einer oder mehrerer der Verbindungen der Formel (I) oder eines ihrer *N*-Oxide oder eines ihrer Salze nach einem der Ansprüche 1 bis 7 auf Schadpflanzen bzw. Pflanzen, der Pflanzensamen oder die Fläche, auf der die Pflanzen wachsen, appliziert.

Europäisches Patentamt
European Patent Office
Office européen des brevets

# EUROPÄISCHER RECHERCHENBERICHT

Nummer der Anmeldung
EP 08 01 3316

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2007/086799 A (ASTRAZENECA AB [SE]; ALBERT JEFFREY [US]; ALHAMBRA CRISTOBAL [US]; KAN) 2. August 2007 (2007-08-02)<br>* Ansprüche 1,11,12; Beispiele 6,8,9,13,15,23,31 *<br>* Seite 15, Zeile 13 - Zeile 27 *<br>* Seite 27, Zeile 9 - Zeile 11 *<br>----- | 1-5,9 | INV.<br>C07D413/12<br>C07D413/14<br>C07D413/06<br>C07D261/08<br>A01N43/80 |
| X | DATABASE HCAPLUS [Online]<br>ACS;<br>XP002510925<br>gefunden im STN<br>Database accession no. 141:140318 (DN)<br>* Zusammenfassung *<br>* RN725271-90-9 *<br>-& JP 2004 203871 A (YAMANOUCHI PHARMACEUTICAL CO.)<br>22. Juli 2004 (2004-07-22)<br>* Absatz [0026]; Beispiel 59; Tabelle 2 *<br>----- | 1-5,9 | |
| X | OHTA S ET AL: "REACTION OF 1-(1-ALKOXYALKYL)-1H-1,2,4-TRIAZOLE WITH AMIDES"<br>HETEROCYCLES, ELSEVIER SCIENCE PUBLISHERS B.V. AMSTERDAM, NL,<br>Bd. 31, Nr. 11,<br>1. Januar 1990 (1990-01-01), Seiten 2029-2036, XP001096277<br>ISSN: 0385-5414<br>* Tabelle II; Verbindung 6D *<br>* scheme 1 *<br>----- | 1,3,4,7 | RECHERCHIERTE SACHGEBIETE (IPC)<br>C07D<br>A01N |
| D,A | WO 2005/070889 A (DU PONT [US]; HANAGAN MARY ANN [US]; SELBY THOMAS PAUL [US]; SHARPE PA) 4. August 2005 (2005-08-04)<br>* Ansprüche 1,13-15; Tabelle 1B *<br>-----<br>-/-- | 1-15 | |

Der vorliegende Recherchenbericht wurde für alle Patentansprüche erstellt

3

| Recherchenort | Abschlußdatum der Recherche | Prüfer |
|---|---|---|
| Berlin | 22. Januar 2009 | Krische, Detlef |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 08 01 3316

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | KARANOV E ET AL: "PLANT GROWTH REGULATING ACTIVITY OF SOME NOVEL 3-(1-ACYLAMINOBENZYL)-2H-1-BENZOPYRAN-2-ONES" COMPTES RENDUS DE L'ACADEMIE BULGARE DES SCIENCES, SOFIA, GB, Bd. 50, Nr. 4, 1. Januar 1997 (1997-01-01), Seiten 93-96, XP008100342 ISSN: 0861-1459 * Seite 93; Verbindung 6 * ----- | 1-15 | |
| D,A | KAI H ET AL: "A Convenient Synthesis of 3-benzoylisoxazoles by 1,3-Dipolar Cycloaddition" HETEROCYCLES, ELSEVIER SCIENCE PUBLISHERS B.V. AMSTERDAM, NL, Bd. 57, Nr. 12, 1. Januar 2002 (2002-01-01), Seiten 2299-2308, XP008100293 ISSN: 0385-5414 * Zusammenfassung; Tabelle 3; Verbindung 1 * ----- | 8,10 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| Berlin | 22. Januar 2009 | Krische, Detlef |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

3

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 08 01 3316

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-01-2009

| Im Recherchenbericht angeführtes Patentdokument | Datum der Veröffentlichung | Mitglied(er) der Patentfamilie | Datum der Veröffentlichung |
|---|---|---|---|
| WO 2007086799 A | 02-08-2007 | EP 1981882 A1 | 22-10-2008 |
| JP 2004203871 A | 22-07-2004 | KEINE | |
| WO 2005070889 A | 04-08-2005 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2005070889 A
- EP 0221044 A
- EP 0131624 A
- WO 9211376 A
- WO 9214827 A
- WO 9119806 A
- EP 0242236 A
- EP 242246 A
- WO 9200377 A
- EP 0257993 A
- US 5013659 A
- EP 0142924 A
- EP 0193259 A
- WO 9113972 A
- EP 309862 A
- EP 0464461 A
- WO 9107874 A
- EP 333131 A
- EP 269806 A
- EP 268554 A
- EP 174562 A
- EP 346620 A
- WO 9108202 A
- WO 9507897 A
- EP 86750 A
- EP 94349 A
- EP 191736 A
- EP 0492366 A
- WO 200234048 A
- EP 0582198 A
- WO 9745016 A
- WO 9916744 A
- EP 365484 A
- WO 2004084631 A
- WO 2005015994 A
- WO 2005016001 A
- WO 2005112630 A
- WO 9838856 A
- WO 9827049 A
- WO 1999000020 A
- WO 2007023719 A
- WO 2007023764 A
- WO 199813361 A
- JP 60087254 A


### In der Beschreibung aufgeführte Nicht-Patentliteratur


- **D. Tiebes.** Combinatorial Chemistry - Synthesis, Analysis, Screening. Verlag Wiley, 1999, 1-34
- *ChemFiles,* vol. 4 (1
- **Barry A. Bunin.** The Combinatorial Index. Verlag Academic Press, 1998
- Combinatorial Chemistry - Synthesis, Analysis, Screening. Verlag Wiley, 1999
- Microwaves in Organic and Medicinal Chemistry. Verlag Wiley, 2005
- *Heterocycles,* 2002, vol. 57 (12), 2299-2308
- *Carbohydrate Research,* 1995, vol. 288, 345-50
- **Winnacker-Küchler.** Chemische Technologie. C. Hanser Verlag, 1986, vol. 7
- **Wade van Valkenburg.** Pesticide Formulations. Marcel Dekker, 1973
- Spray Drying. **K. Martens.** Handbook. G. Goodwin Ltd, 1979
- **Watkins.** Handbook of Insecticide Dust Diluents and Carriers. Darland Books
- Introduction to Clay Colloid Chemistry. J. Wiley & Sons
- **C. Marsden.** Solvents Guide. Interscience, 1963
- **McCutcheon's.** Detergents and Emulsifiers Annual. MC Publ. Corp,
- **Sisley ; Wood.** Encyclopedia of Surface Active Agents. Chem. Publ. Co. Inc, 1964
- **Schönfeldt.** Grenzflächenaktive Äthylenoxidaddukte. Wiss. Verlagsgesell, 1976
- Spray-Drying Handbook. G. Goodwin Ltd, 1979
- Agglomeration. **J.E. Browning.** Chemical and Engineering. 1967, 147 ff
- Perry's Chemical Engineer's Handbook. McGraw-Hill, 1973, 8-57
- **G.C. Klingman.** Weed Control as a Science. John Wiley and Sons, Inc, 1961, 81-96
- **J.D. Freyer ; S.A. Evans.** Weed Control Handbook. Blackwell Scientific Publications, 1968, 101-103
- Gene Transfer to Plants, Springer Lab Manual. Springer Verlag, 1995

- **Christou.** Trends in Plant Science. 1996, vol. 1, 423-431
- **Sambrook et al.** Molecular Cloning, A Laboratory Manual. Cold Spring Harbor Laboratory Press, 1989
- **Winnacker.** Gene und Klone. VCH, 1996
- **Braun et al.** *EMBO J.,* 1992, vol. 11, 3219-3227
- **Wolter et al.** *Proc. Natl. Acad. Sci. USA,* 1988, vol. 85, 846-850
- **Sonnewald et al.** *Plant J.,* 1991, vol. 1, 95-106
- *Weed Research,* 1986, vol. 26, 441-445
- The Pesticide Manual. The British Crop Protection Council and the Royal Soc. of Chemistry, 2003